Europäisches Patentamt

European Patent Office

Office européen des brevets

(11) Veröffentlichungsnummer: **0 410 147 B1**

(12) **EUROPÄISCHE PATENTSCHRIFT**

(45) Veröffentlichungstag der Patentschrift: **30.11.94**

(21) Anmeldenummer: **90112064.2**

(22) Anmeldetag: **25.06.90**

Teilanmeldung 93120518.1 eingereicht am 25/06/90.

(51) Int. Cl.⁵: **C12N 15/41**, C12N 15/57, C07K 7/00, A61K 37/64

(54) **Modifizierte VP1/P2A Regionen des rhinoviralen Systems.**

(30) Priorität: **25.06.89 DE 3920860**
**08.06.90 DE 4018376**

(43) Veröffentlichungstag der Anmeldung:
**30.01.91 Patentblatt 91/05**

(45) Bekanntmachung des Hinweises auf die Patenterteilung:
**30.11.94 Patentblatt 94/48**

(84) Benannte Vertragsstaaten:
**AT BE CH DE DK ES FR GB GR IT LI LU NL SE**

(56) Entgegenhaltungen:
EP-A- 0 263 903
EP-A- 0 321 973

VIROLOGY, Band 169, 1989, Seiten 68-77, Academic Press, Inc.; W. SOMMERGRUBER et al.: "Polypeptide 2A of human rhinovirus type 2: Identification as a protease and characterization by mutational analysis"

IDEM

(73) Patentinhaber: **BOEHRINGER INGELHEIM IN-TERNATIONAL GmbH**
**Postfach 200**
**D-55216 Ingelheim (DE)**

(72) Erfinder: **Sommergruber, Wolfgang, Dr. Biochem.**
**Stoesslgasse 6/5**
**A-1130 Wien (AT)**
Erfinder: **Volkmann, Peter, Dr. Biol.**
**Binagasse 19**
**A-1238 Wien (AT)**
Erfinder: **Fessl, Friederike, Dipl.-Ing.**
**Aspettenstrasse 30/2/10**
**A-2480 Perchtoldsdorf (AT)**
Erfinder: **Maurer-Fogy, Ingrid, Dr.**
**Lindauergasse 35e**
**A-1238 Wien (AT)**
Erfinder: **Küchler, Ernst, Dr.**
**Görgengasse 23/6/26**
**A-1190 Wien (AT)**
Erfinder: **Blaas, Dieter, Dipl.-Ing. Dr.**
**Liechtensteinstrasse 119**
**A-1090 Wien (AT)**
Erfinder: **Skern, Timothy, Dr. Biochem.**
**Lerchenfelder Gürtel 45/15**
**A-1160 Wien (AT)**

JOURNAL OF VIROLOGY, Band 61, Nr. 12, Dezember 1987, Seiten 3680-3687, American Society for Microbiology; G.D. PARKS et al.: "Site-specific mutations at a picornavirus VP3/VP1 cleavage site disrupt in vitro processing and assembly of capsid precursors"

Erfinder: **Zorn, Manfred**
**Lawrence Berkley Lab. MS 50B-3238 Cyclotron Road 1**
**Berkley California 94720 (US)**
Erfinder: **Auer, Herbert**
**Währinger Gürtel 82/23**
**A-1090 Wien (AT)**

**Beschreibung**

Gegenstand der vorliegenden Erfindung sind Systeme, die es ermöglichen, Mutationen in der VP1/2A und in der gesamtkodierenden Region der Protease 2A einzufügen, Testsysteme, mit denen die "cis"- und "trans"-Aktivität untersucht werden können, Oligonukleotide, die die Mutationen kodieren, sowie die hieraus abgeleiteten Oligopeptide und deren Verwendung. Weiterhin Oligopeptide, die von der Protease 2A in trans mit unterschiedlicher Effizienz gespalten werden, sowie deren Verwendung.

Rhinoviren sind ss(+)RNA - Viren und repräsentieren eine Gattung innerhalb der Picornaviridae (Cooper, P. D et al., 1978, Intervirology 10, 165 - 180; MacNaughton, M. R., 1982, Current Top. Microbiol. Immunol. 97, 1 - 26). Sie sind weit verbreitet, befallen den oberen respiratorischen Trakt des Menschen und verursachen akute Infektionen, die zu Schnupfen, Husten, Heiserkeit etc. führen und allgemein als Erkältungen bezeichnet werden (Stott, E. J. und Killington, R. A., 1972, Ann. Rev. Microbiol. 26, 503 - 524). Infektionen durch Rhinoviren zählen zu den häufigsten Erkrankungen des Menschen. Die Krankheit verläuft zwar meist harmlos, dennoch kommt es -bedingt durch eine vorübergehende Schwächung des Organismus- zu Sekundärinfektionen durch andere Viren oder Bakterien, die dann unter Umständen schwere Erkrankungen zur Folge haben. Von den insgesamt ca. 115 verschiedenen, bekannten Serotypen von humanen Rhinoviren sind bis jetzt 4 Serotypen (HRV 1B, 2, 14 und 89) kloniert und komplett sequenziert worden: Deutsche Patentanmeldung P 35 05 148.5; Skern; T. et al., 1985, Nucleic Acids Res. 13, 2111-2126; Düchler, M. et al., 1987, Proc. Natl. Acad. Sci. USA 84, 2605 - 2609; Stanway, G. et al., 1984, Nucleic Acids Res. 12, 7859 - 7877; Callahan, P. L. et al., 1985, Proc. Natl. Acad. Sci. USA 82, 732 - 736; Hughes, R. et al., 1988, J. Gen Virol. 69, 49 - 58).

Kaum ein anderes virales System ist bei seiner Regulation des Infektionsablaufes dermaßen von einer kontrolliert limitierten Proteolyse abhängig, wie das der Picornaviridae. Die genomische einzelsträngige (+)-RNA der Rhinoviren wird kurz nach der Infektion durch Abspaltung des an das 5'Ende gebundenen Oligopeptids VPg modifiziert und dient als mRNA für die Synthese eines Polyproteins, das den gesamten durchgehenden Leserahmen der Nukleinsäuresequenz umfaßt (Butterworth, B. E., 1973, Virology 56, 439 - 453; Mc Lean, C. und Rueckert, R. R., 1973, J. Virol. 11, 341 - 344; Mc Lean, C. et al., 1976, J. Virol. 19, 903 - 914). Die reifen viralen Proteine entstehen ausschließlich durch proteolytische Spaltung aus diesem Polyprotein, wobei die dabei wirksamen Proteasen selbst Bestandteil dieses Polyproteins sind. Der erste Schritt bei diesem Prozessieren ist die Abspaltung der Vorstufe der Hüllenproteine, die durch die Protease P2A vorgenommen wird. In der Reihenfolge der Gene liegt die Sequenz der Protease P2A unmittelbar hinter dem für die Hüllenproteine kodierenden Abschnitt. P2A ist somit aufgrund ihrer Lokalisation im Polyprotein die erste nachweisbare enzymatische Funktion des Virus. Sie spaltet sich selbst vom Precursor der Hüllenproteine ab und ist für die Trennung des Kapsidprecursors P1 vom Rest des Polyproteins verantwortlich ("cis"-Aktivität). Die Trennung der Hüllenproteinregion von dem für die Replikation verantwortlichen Abschnitt findet bereits während der Translation des Polyproteins statt. Bei Poliovirus kann diese primäre Spaltung an der P1-P2 Region intermolekular d.h. "in trans" von der reifen Protease 2A vorgenommen werden (Kräusslich, H.-G. and Wimmer, E., 1988, Ann. Rev. Biochem., 57, 701 - 754).

Dieser Schritt ist für den weiteren Ablauf der viralen Infektion essentiell (Kompartimentierung von Replikation und Virus-assembly). Bei Cardio- und Aphtoviren wird im Gegensatz zu den Polioviren diese Spaltung von der Protease 3C katalysiert (Kräusslich, H.-G. and Wimmer, E., 1988, loc. cit.). Vom Poliovirussystem weiß man, daß wahrscheinlich alle an dieser Reifungsspaltung beteiligten Enzyme viral kodiert sind (Toyoda, H. et al., 1986, Cell, 45, 761-770). Im Poliovirus findet man drei Typen von Spaltsignalen; die am meisten verwendeten Q-G Stellen, welche von der viralen Protease P3C erkannt werden, und die Y-G Stelle, welche von P2A als Erkennungssignal verwendet wird. Zunächst rückte die Protease P3C in den Mittelpunkt des Interesses bei der Aufklärung des proteolytischen Processings von Picornaviren. Schon sehr früh konnte eine der P3C äquivalente proteolytische Aktivität in EMC beschrieben werden (Pelham, H. R. B. , 1978, Eur. J. Biochem. 85, 457 - 461; Palmenberg A. C. et al., 1979, J. Virol. 32, 770 - 778). Im Laufe weiterer Untersuchungen stellte sich heraus, daß das Leaderpeptid (L) von Cardio- (z. B. EMCV) und Aphtoviren (z. B. FMDV), welches bei Rhino- und Enteroviren nicht vorhanden ist, am proteolytischen Processing von EMCV beteiligt ist (Palmenberg, A. C., 1987, J. Cell. Biochem. 33, 1191-1198). In weiterer Folge konnte durch die Isolierung von Polio P3C und mit Hilfe von immunologischen Methoden gezeigt werden, daß P3C sich selbst autokatalytisch aus dem Polyprotein herausschneidet, um dann in "trans" alle potentiellen Q-G Spaltstellen anzugreifen.

Der Einsatz von rekombinanten Systemen, die unter anderem die P3C-Region repräsentierten, ermöglichte die Expression von P3C einiger Entero- und Rhinoviren (Werner, G. et al., 1986, J. Virol. 57, 1084 - 1093) und die genaue Charakterisierung der P3C von Polio (Hanecak, R. et al., 1984, Cell 37, 1037 - 1073; Korant, B. D. und Towatari, T., 1986, Biomed. Biochim.

Acta 45, 1529 - 1535) sowie der äquivalenten proteolytischen Funktion in FMDV (Klump, W. et al., 1984, Proc. Natl. Acad. Sci. USA 81, 3351 - 3355; Burroughs, J. N. et al., 1984, J. Virol. 50, 878 - 883). Durch "in vitro" Mutagenesestudien konnte nachgewiesen werden, daß der Austausch der hoch konservierten Aminosäuren Cystein (Positionsnummer 47) und Histidin (Positionsnummer 161) in P3C von Poliovirus zu einem inaktiven Enzym führt, während die Mutation des nicht konservierten Cysteins (Positionsnummer 153) keinen nennenswerten Einfluß auf die proteolytische Aktivität von Polio-P3C hat. Diese durch Oligonukleotide bewirkte Mutagenese von rekombinanten P3C und die zusätzlich durchgeführten Inhibitorstudien lassen den Schluß zu, daß Polio-P3C zu der Klasse der Cysteinproteasen gehört (Ivanoff, L. A. et al., 1986, Proc. Natl. Acad. Sci. USA 83, 5392 - 5396). Ebenfalls durch "in vitro" Mutagenese von Polio-P3C (und zwar durch Austausch des konservierten Valin gegen Alanin in Position 54 der Protease) konnte gezeigt werden, daß diese Mutation in einer "full size" cDNA von Polio nach Transfektion in COS 1 Zellen zu einem Polymerase-defizienten Virus führt (Dewalt, P. G. und Semler, B. L., 1987, J. Virol. 61, 2162 - 2170).

Antikörper, die gegen Polio-P3C entwickelt wurden, unterbanden zwar eindeutig sämtliche an Q-G durchgeführten Spaltungen, nicht aber die Spaltung zwischen Y-G (Hanecak, R. et al., 1982, Proc. Natl. Acad. Sci. USA 79, 3973 - 3977). Diese Beobachtung führte zum Schluß, daß das proteolytische Processing an Y - G Stellen einer eigenen Protease bedarf. Der Sitz dieser zweiten proteolytischen Aktivität konnte in Poliovirus eindeutig P2A zugeordnet werden. Interessant war der Befund, daß P2A eine alternative Spaltung in der Protease-Polymeraseregion (3CD) durchführt, welche ebenfalls an einer Y - G Stelle stattfindet und inaktive Enzyme 3C und 3D liefert. Diese Spaltung dient möglicherweise zur quantitativen Regulation der Enzyme 3C und 3D (Lee, C.K. und Wimmer, E., 1988, Virol., 166, 1435 - 1441).

Da es während der Infektion mit Poliovirus in HeLa-Zellen sehr rasch zu einem Abschalten der Wirtsproteinsynthese kommt, die Translation der Poliovirus-RNA jedoch unbehindert ablaufen kann, nahm man an, daß ein oder mehrere Regulationsfaktoren der Translation während der Infektion verändert werden. Tatsächlich zeigen Befunde, daß der eukaryontische Initiationsfaktor 4F durch proteolytische Spaltung der p220 Komponente während der Poliovirusinfektion in HeLa-Zellen verändert wird (Etchison, D. et al., 1984, J. Virol. 51, 832 - 837; Etchison, D. et al., 1982, J. Biol. Chem. 257, 14806 - 14810). In der Folge konnte gezeigt werden, daß P2A indirekt für diese Modifikation von p220 in infizierten Zellen verantwortlich ist (Kräusslich, H. G. et al., 1987, J. Virol. 61, 2711 - 2718; Lloyd, R.E. et al. 1986, Virol., 150, 299 - 303; Lloyd, R. E. et al., 1987, J. Virol., 61, 2450 - 2488). Erst kürzlich konnte gezeigt werden, daß in mit Rhinovirus infizierten Zellen ebenfalls der Abbau von p220 erfolgt (Kräusslich, H.-G. unpublizierte Daten). Die Frage nach der "trans"-Aktivität der Protease P2A konnte im Poliovirussystem positiv beantwortet werden, indem durch Insertionen und Deletionen in P2A eine unprozessierte P1-P2 Region in E. coli exprimiert wurde, die von P2A aus mit Poliovirus infizierten Zellen (Nicklin, M. J. H., et al., Proc. Natl. Acad. Sci. USA, 1987, 84, 4002 - 4006), P2A, das "in vitro" translatiert (Kräusslich, H.-G. et al. 1987, J. Virol., 61, 2711 - 2718) oder in E. coli exprimiert wurde, oder mit einem gereinigten 2A Protein von Poliovirus (König, and Rosenwirth, B., 1988, J. Virol., 62, 1243 - 1250) gespalten werden konnte. Durch Großmaßstab Produktion von rekombinanter Polio 3C in E. coli und Reinigung dieser Protease, konnte die spezifische Spaltungseffizienz bei Verwendung von nativen und synthetischen Substraten "in vitro" gezeigt werden (Nicklin, M.J.H. et al., 1988, J. Virol., 62, 4586 - 4593).

Neben der "cis"-Aktivität wurde im Rhinovirussystem auch eine "trans"-Aktivität der Protease 2A durch spezifische Spaltung eines Peptidsubstrates, welches die native Spaltregion zwischen VP1 und 2A repräsentiert, nachgewiesen (Sommergruber, W., et al., 1989, Virol, 169, 68 - 77;). Weiter konnte durch Klonieren und Espression der Protease 3C von HRV14 in einem E. coli Maxizellsystem gezeigt werden, daß Rhino 3C in rekombinanten Systemen proteolytisch aktiv ist, eine Vorstufe von 3C "in trans" für die Freisetzung von 3C verantwortlich ist und daß $ZnCl_2$ spezifisch das Processing von 3C-Precursorformen inhibiert (Cheah, K. C., et al., 1988, Gene, 69, 265-274). Ferner konnte in E. coli Extrakten, die die Protease 3C von HRV14 enthielten, die spezifische Spaltung eines Peptidsubstrates nachgewiesen werden (Libby, R.T. et al., 1988, 27, 6262 - 6268).

Die Protease 3C ist wie 2A in der Lage sich autokatalytisch aus dem Polyprotein abzuspalten (Hanecak, R., et al., 1984, loc. cit.). Dabei wird die aminoterminale Spaltstelle deutlich gegenüber dem Carboxyende bevorzugt. Weiters kann eine unterschiedliche Affinität zu anderen Spaltstellen beobachtet werden. Zur Spaltung des Kapsidproteinvorläufers P1 in 1ABC und 1D reicht 3C, die weitere Spaltung in 1AB und 1C erfordert den Komplex 3CD, die Protease mit der carboxyterminalen Replikase. In Poliovirus wird die Substratspezifität der Protease 3C möglicherweise durch die Polymerase 3D über strukturelle Veränderungen moduliert (Jore, J., et al., 1988, J. Gen. Virol., 69, 1627 - 1636; Ypma-Wong, M. F., et al., 1988, Virol., 166, 265 - 270).

Ein Vergleich der Aminosäuresequenzen der einzelnen Proteine zeigt, daß die viralen Enzyme in besonderem Maße konserviert sind. So beträgt etwa die Homologie zwischen der Protease P2A von HRV89 und HRV2 85%; bei der Protease P3C sind 75% der Aminosäuren identisch (Düchler, M. et al., 1987, loc. cit.). Diese Werte liegen wesentlich über den durchschnittlich im Gesamtprotein beobachteten Prozentsätzen. Man kann daher davon ausgehen, daß gerade die viralen Enzyme in der Evolution besonders gut konserviert sind und in ihren Eigenschaften bei verschiedenen Rhinoviren sehr ähnlich sind.

Sehr interessant sind auch die Befunde, daß zwei den picornaviralen Proteasen P3C und P2A ähnliche Proteine in dem pflanzenviralen System der Comoviridae (Cowpea Mosaic Virus) gefunden wurden (Garcia, J. A. et al., 1987, Virology, 159, 67 - 75; Verver, J. et al., 1987, EMBO, 6, 549 - 554). Diese beiden viralen Proteine sind am proteolytischen Processing der beiden durch zwei getrennt verpackte ss (+)RNA-Moleküle (B und M RNA) kodierten Polyproteine beteiligt, wobei eine große Ähnlichkeit der beiden Cowpea mosaic-Proteasen in Sequenz und Spaltspezifität zu den Picornaviren festzustellen ist. Diese bemerkenswerte Homologie von nicht strukturellen Proteinen zwischen Picorna- und Comoviren weist nicht nur auf eine genetische Verwandtschaft zwischen diesen beiden Virusfamilien hin, sondern zeigt auch auf, wie essentiell das virale proteolytische Processing für diese beiden Virusfamilien ist.

Die dritte Art der viralen Reifungsspaltung, nämlich die von VP0 (Vorläuferprotein von VP2 und VP4) konnte bei Mengo und Rhinovirus mit Hilfe von Röntgenstrukturdaten beschrieben werden. Dieses letzte proteolytische Ereignis bei der viralen Maturation scheint auf einem ungewöhnlichen autokatalytischen Serinprotease Typ zu beruhen, bei welchem basische Gruppen der viralen RNA an der Ausbildung des katalytischen Zentrums beteiligt sind, wobei diese basischen Gruppen als Protonenakzeptor fungieren (Arnold, E. et al., 1987, Proc. Natl. Acad. Sci. USA, 84, 21 - 25). Im Falle des FMDV (Acharya, R., et al., 1989, Nature, 337, 709 - 716) gibt die Kristallstruktur allerdings keinen Hinweis auf einen solchen Mechanismus bei Aphtoviren.

Die Spaltstellen der viralen Proteasen wurden im Poliovirussystem durch N-terminales Sequenzieren der meisten Poliovirusproteine ermittelt (Pallansch, M. A. et al., 1984, J. Virol., 49, 873 - 880). Die Lage der Schnittstellen von HRV2 zwischen VP4/VP2, VP2/VP3 sowie VP3/VP1 konnte durch N-terminales Sequenzieren von VP2, VP3 und VP1 bestimmt werden. Das Spaltsignal zwischen VP1 und P2A wurde einerseits durch C-terminales Sequenzieren von VP1 (Kowalski, H. et al., 1987, J. Gen. Virol. 86, 3197 - 3200) andererseits durch N-terminale Sequenzanalyse von P2A bestimmt (Sommergruber, W., et al. 1989, loc. cit.). Außerdem konnten durch das Klonieren und Sequenzieren von HRV2 (Skern, T. et al., 1985 Nucleic Acids Res., 13, 2111-2126) an Hand von Sequenzvergleichen mit Poliovirus und HRV14 die meisten Spaltstellen abgeleitet werden. So konnten in HRV2 fünf verschiedene Spaltsignale gefunden werden: Q-S, Q-G, Q-N, A-G und E-S.

Cysteinproteasen sind in der Natur weit verbreitet (z. B. Papain, Cathepsin B, H und S) und ihre Charakterisierung und Inhibierung ist von großem wissenschaftlichen und therapeutischen Wert (zur Übersicht siehe Turk, V., 1986, Cysteine Proteinases and their Inhibitors, Walter de Gruyter; Barrett, A. J. und Salvesen, G., 1986, Proteinase Inhibitors, Elsevier).

Die generelle Bedeutung der Inhibierung von viral kodierten Proteasen wurde nicht zuletzt durch Arbeiten mit der Protease des humanen Immundefizienz Virus 1 (HIV I) wieder in den Blickpunkt von möglichen antiviralen Therapieansätzen gerückt. Durch Deletions- und Punktmutationen in der Proteaseregion dieser Art von Retroviren konnte die essentielle Rolle der Protease bei der Reifung dieser Virenklasse erkannt werden (Katoh, I., et al., 1985, Virol., 145, 280 - 292; Kohl, N. E., et al., 1988, Proc. Natl. Acad. Sci. USA, 85, 4686 - 4690; Crowford, S. and Goff, S.P., 1985, J. Virol., 53, 899 - 907). Durch Röntgenstrukturanalysen und molekularbiologische Studien konnte weiters gezeigt werden, daß die Protease von HIV I dem Asp-Typ angehört, sich selbst am Vorläuferprotein prozessieren kann (auch in rekombinanten prokaryontischen Systemen), "in trans" spezifisch Peptide spalten kann und als aktive Protease in einer homodimeren Form vorliegt (Navia, M. A. et al., 1989, Nature, 337, 615 - 620; Meek, T.D., et al., 1989, Proc. Natl. Acad. Sci. USA, 86, 1841 - 1845; Katoh, I. et al., 1985, loc. cit.).

Auch im picornaviralen System sind derzeit verschiedenste anorganische und organische Verbindungen, sowie Peptidderivate und Proteine bekannt, die eine inhibitorische Wirkung auf das proteolytische Processing dieser Viren besitzen. Der Effekt dieser Substanzen beruht auf der direkten Wechselwirkung mit den Proteasen (Kettner, C. A. et al., 1987, US Patent Nr.: 4,652,552; Korant, B. D. et al., 1986, J. Cell. Biochem. 32, 91 -95) und/oder auf dem indirekten Weg der Wechselwirkung mit Substraten dieser Proteasen (Geist, F. C., et al., 1987, Antimicrob. Agents Chemother. 31, 622 - 624; Perrin, D. D. und Stünzi, H., 1984, Viral Chemotherapy 1, 288 - 189). Das Problem bei den meisten dieser Substanzen ist die relativ hohe Konzentration, die zur Inhibierung nötig ist und die zum Teil große Toxizität dieser Verbindungen.

Wie bereits dargelegt wurde, ist der Infektionsablauf durch Picornaviren entscheidend von den viralen Enzymen abhängig. Da gerade diese Enzyme besonders gut konserviert und in ihren Eigenschaften bei verschiedenen Rhinoviren sehr ähnlich sind, bieten sie sich geradezu als Ziel eines chemotherapeutischen Eingriffs an, wie z. B. das virale Enzym P2A. Der chemotherapeutische Ansatz ist die Inhibierung der enzymatischen Aktivität durch spezifische Inhibitoren.

Inhibiert man die erste proteolytische Aktivität, die P2A-Aktivität, so unterbindet man jeden weiteren Reifungsprozeß des viralen Systems. Auf Grund der ausgeprägten Homologie der P2A-Region von HRV2 nicht nur zu anderen Rhinoviren, sondern auch zu Vertretern anderer Gruppen der Picornaviridae (unpublizierte Daten A. Palmenberg), ist es durchaus denkbar, daß ein Inhibitor gegen HRV2-P2A auch auf andere Picornaviren anwendbar ist.

Ein Testsystem für Inhibitoren der ersten proteolytischen Aktivität konnte etabliert werden (Sommergruber, W. et al., loc. cit.).

Durch dieses System konnte gezeigt werden, daß:

- das Polypeptid 2A von HRV2 eine Protease ist, welche bei Expression als Substratenzym in E. coli bestehend aus einem Fusionsanteil der MS2 Polymerase (98 Aminosäuren), der Region für das virale Hüllenprotein VP1 und der Protease 2A seinen eigenen N-Terminus erkennt und sich vom Precursor abspaltet;
- E. coli Extrakte, welche die aktive Protease 2A enthalten, spezifisch ein 16 Aminosäuren langes Peptid, das die native Spaltregion zwischen P1 und P2 in symmetrischer Art darstellt, spalten können;
- durch Deletion der letzten 10 C-terminalen Aminosäuren der Protease 2A, die proteolytische Funktion zerstört wird;
- durch Austausch des hochkonservierten Arg 134 gegen Gln im C-Terminus von 2A ebenfalls die proteolytische Funktion unterbunden wird;
- Deletions- und Mutationsstudien im vermutlich aktiven Zentrum von 2A die proteolytische Aktivität inhibieren.

Experimentelle Daten über den katalytischen Mechanismus von Poliovirus 3C zeigten, daß die Protease 3C durch Inhibitoren für Thiolproteasen wie z.B. N-Ethylmaleimid oder Jodacetamid blockiert werden können (Pelham, H.R.B., 1978, Eur. J. Biochem., 85, 457 - 462). Auf Grund von Vergleichen der 3C Sequenzen von Picornaviren (Argos, P., et al., 1984, Nucleic Acids Res., 12, 7251 - 7267) und Punktmutationsexperimenten (Ivanoff,L., et al., 1986, loc. cit.) wurde ein Cystein-Histidin Paar als vermutetes aktives Zentrum für die Cysteinprotease definiert.

Durch Vergleich mit anderen Picornaviren und weiteren Inhibitorstudien (Parks, G.D., et al., 1986, J. Virol., 60, 376 - 384; König, H. und Rosenwirth, B., 1988, J. Virol., 62, 1243 - 1250) sowie direkte Mutagenese des vermuteten aktiven Zentrums der Protease 2A von HRV2 (Sommergruber, W., et al., 1989, Virol., 169, 68 - 77) ist es sehr wahrscheinlich, daß auch 2A ein Cystein als Nukleophil besitzt. Ein Austausch des Cysteins 106 von HRV2-2A gegen ein Serin im vermuteten aktiven Zentrum des Enzyms zerstört die Aktivität (Sommergruber, W. et al., loc. cit).

Ein Vergleich der letzten 50 Aminosäuren mit dem aktiven Zentrum von 2A mit der SWISSPROT Sequenzdatenbank ergibt große Ähnlichkeiten des 2A Proteins mit Serinproteasen und nicht mit Cysteinproteasen. Das Cystein agiert offenbar im aktiven Zentrum einer Serinprotease. Der Austausch des Serins durch ein Cystein in Subtilisin (Philipp, M., et al., 1971, Methods in Enzymology, 19, "Proteolytic Enzymes", Colowick, S.P. and Kaplan, N. O.) zum Thiosubtilisin durch chemische Modifikation erhält die Esteraseaktivität, bei Trypsin wird durch gerichtete Mutagenese des Serins in ein Cystein die Aktivität um einen Faktor 100 000 gesenkt (Higaki, J. N., et al., 1987, Cold Spring Harbor Symp. Quant. Biol., 52, 615 - 621).

Zu einem ähnlichen Ergebnis, wenn auch weniger signifikant, führt ein Vergleich des 3C Proteins mit anderen Proteasen (Gorbalenya, A. E. et al., 1986, FEBS Lett., 194, 253 -257). Es wird sogar ein gemeinsamer Vorläufer für Serin- und Cysteinproteasen postuliert, dem die viralen Proteasen noch sehr ähnlich sind (Gorbalenya` A. E., et al., 1989, FEBS Lett., 243, 103 - 114). Eine klare Unterscheidung zwischen den beiden viralen Proteasen 2A und 3C treffen Bazan und Fletterick. Sie finden starke Strukturhomologie der 3C verschiedener Picornaviren und verwandter Pflanzenviren mit der Chymotrypsinfamilie der Serinproteasen bei Vergleich von Kristallstrukturen von Chymotrypsin mit Sekundärstrukturvorhersagen der viralen Enzyme. Die für die Struktur entscheidenden $\beta$-Faltblattkonformationen sind konserviert, Insertionen und Deletionen treten nur in den verbindenden Schleifen auf (Bazan, J. F. und Fletterick, R. J., 1988, Proc. Natl. Acad. Sci. USA, 85, 7872 - 7876). Das Protein 2A weist strukturelle Gemeinsamkeiten mit der Subtilisinfamilie der Serinproteasen auf. Auf Grund dieser Vergleiche bilden wahrscheinlich die Aminosäuren His 18, Asp 35 und Cys 106 das aktive Zentrum von HRV2-2A.

Die Proteine mit der höchsten Ähnlichkeit mit den letzten 50 Aminosäuren von 2A waren: Thaumatin I und II von "Thaumatococcus daniellii Benth", der Frucht einer einkeimblättrigen westafrikanischen Buschpflanze (Van der Wel, H., 1972, FEBS Lett., 21, 88 - 90; Iyengar, R. B., et al., 1979, Eur. J. Biochem., 90, 195 - 204), von denen in der Literatur ihre außerordentliche Süßkraft bekannt ist. Bei der Reinigung eines verwandten Proteins, Monellin, wird eine proteolytische Aktivität beschrieben (Morris, J. A. und Cagan, R. H., 1972, Biochem. Biophys. Acta, 261, 114 -122; Van der Wel, H., 1972, Europ. J. Biochem., 31, 221 -

6

225), die möglicherweise integraler Bestandteil dieser süßen Proteine ist. Vor kurzem wurde die ausgeprägte Ähnlichkeit dieser Proteine mit einem Proteaseinhibitor beschrieben (Richardson, M., et al., 1987, Nature, 327, 432 - 434), der zu einer Gruppe von Proteinen gehört, die bei Befall einer Pflanze durch Insekten, Mikroorganismen oder Viren induziert wird. Einige dieser in Pflanzen induzierten Proteine haben ebenfalls die Funktion eines Proteinaseinhibitors bzw. einer Protease (Cornelissen, B. J. C. et al., 1986, Nature, 321, 531 -532).

Eine Aufgabe der vorliegenden Erfindung bestand darin, Systeme bereitzustellen, die es ermöglichen, in der gesamtkodierenden Region, insbesondere der Spaltregion der Protease 2A beliebige Punktmutationen bzw. Deletionen einzufügen.

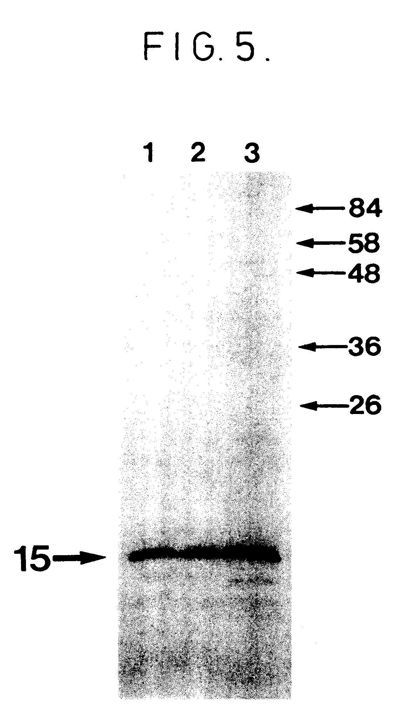

Gelöst wurde diese Aufgabe durch die Bereitstellung von Expressionssystemen, die unter Beibehaltung der nativen Aminosäuresequenz geänderte Nukleotidsequenzen aufweisen. Ausgehend vom Expressionsvektor pEx2A wurden modifizierte Plasmide etabliert. Diese modifizierten Expressionssysteme weisen unter Beibehaltung der nativen Aminosäuresequenz singuläre Signalsequenzen für Restriktionsenzyme auf, die mit Hilfe von synthetischen doppelsträngigen Oligonukleotiden eingebracht wurden. Es wurden die Systeme pEx2A/21 sowie pEx2A/II erhalten, deren Expressionsprodukte sich identisch verhielten wie die des pEx2A (s. Fig. 5, Spur 2 und 3).

Diese neuen Expressionssysteme ermöglichen es unter Ausnutzung der neugenerierten Restriktionsstellen Mutationsoligonukleotide einzubauen, die einerseits zur Feinanalyse der Spaltstelle verwendet wurden, andererseits für Deletionsstudien herangezogen werden können, um die kleinste noch proteolytisch aktive Region von HRV2-2A zu ermitteln.

Diese Systeme, insbesondere die mutierten Systeme sind weiterhin als Testsysteme zu verwenden, um die intramolekulare Aktivität der Protease, die "cis"-Aktivität zu untersuchen, indem die Auswirkungen der eingeführten Mutationen auf die "cis"-Aktivität direkt bestimmt werden können. Gleichzeitig können die Auswirkungen dieser Mutationen auf die "trans"-Aktivität bestimmt werden.

Die Mutationen der $P_1$ Position der Spaltstelle der Protease 2A von HRV2 (im folgenden HRV2-2A genannt) wurden entweder im Expressionsvektorsystem pEx18521 oder pEx2A bzw. dessen Derivat pEx2A/21 durchgeführt.

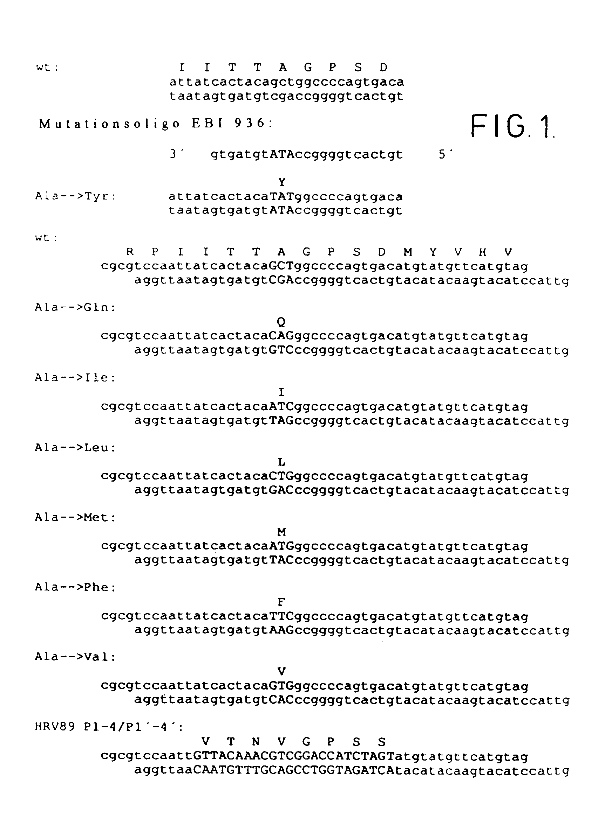

Zunächst wurde das Alanin der $P_1$ Stelle in HRV2 gegen ein Tyrosin ausgetauscht. Diese Mutation führt zu einem Y/G-Aminosäurepaar an der 2A-Spaltstelle, wie sie in Poliovirus vorkommt (Toyoda, H. et al., 1986, Cell, 45, 761 - 770). Ein die Mutation (Ala --> Tyr) enthaltendes PstI/HindIII Fragment wurde dazu verwendet, das Wildtypfragment von pEx18521 zu ersetzen. Das pEx2A/21 wurde dazu verwendet, um mit Hilfe von doppelsträngigen Oligonukleotiden (siehe Fig. 1) weitere Mutationen in und um die Spaltstelle einzuführen. Dabei repräsentiert z. B. ein doppelsträngiges Oligonukleotid jene Aminosäuren in Position $P_{1-4}$ und $P_1'_{-4}'$ wie sie im Serotyp HRV89 vorgefunden werden (siehe Fig. 1).

Konstruktionen, die die Aminosäuren Glutamin, Leucin, Methionin, Phenylalanin und Tyrosin an der $P_1$ Position aufweisen, wurden ebenso effizient gespalten wie der Wildtyp, was aus dem Fehlen der Banden des ungespaltenen Materials bei 65Kd (im Fall der Tyrosinmutation bei 75Kd) hervorgeht. Die Mutanten, welche Valin oder Isoleucin an der $P_1$ Stelle aufweisen, wurden jedoch nicht vollständig prozessiert. Bei Verwendung der Valin enthaltenden Konstruktion wurde 25% des induzierten Proteins nicht prozessiert; befindet sich ein Isoleucinrest an dieser Stelle, so wurde sogar 50% des induzierten Materials nicht gespalten. Eine deutliche Reduktion der Spalteffizienz findet man auch bei Verwendung derjenigen Aminosäuren in Position $P_{1-4}$ und $P_1'_{-4}'$ wie sie im Serotyp HRV89 vorgefunden werden (ca. 65-70% des induzierten Proteins wird nicht prozessiert).

Zusammenfassend ist zu sagen, daß die Aminosäuren Isoleucin und Valin offensichtlich nicht sehr gut in das aktive Zentrum der HRV2-2A passen; dies ist umso bemerkenswerter, als Valin jene Aminosäure ist, welche in der P1 Stelle von HRV89 anzutreffen ist. Möglicherweise stört die Methylgruppe des $\beta$-C-Atoms die Topographie des aktiven Zentrums bzw. eine zur proteolytischen Spaltung notwendige strukturelle Veränderung des Substrates. Es ist daher anzunehmen, daß Unterschiede in den Strukturen der aktiven Zentren der 2A zwischen den Serotypen HRV2 und HRV89 bestehen.

Es wurde daher der Alaninrest gegen einen Threoninrest ausgetauscht, da Threonin eine Hydroxylgruppe am $\beta$-C-Atom besitzt. Das Expressionsprodukt der Konstruktion mit dem Ala-->Thr Austausch an der $P_1$ Stelle wurde gut gespalten: die Hydroxylgruppe am $\beta$-C-Atom ist offensichtlich im Gegensatz zur Methylgruppe von Valin und Isoleucin nicht groß genug, um die Spalteffizienz zu beeinflussen.

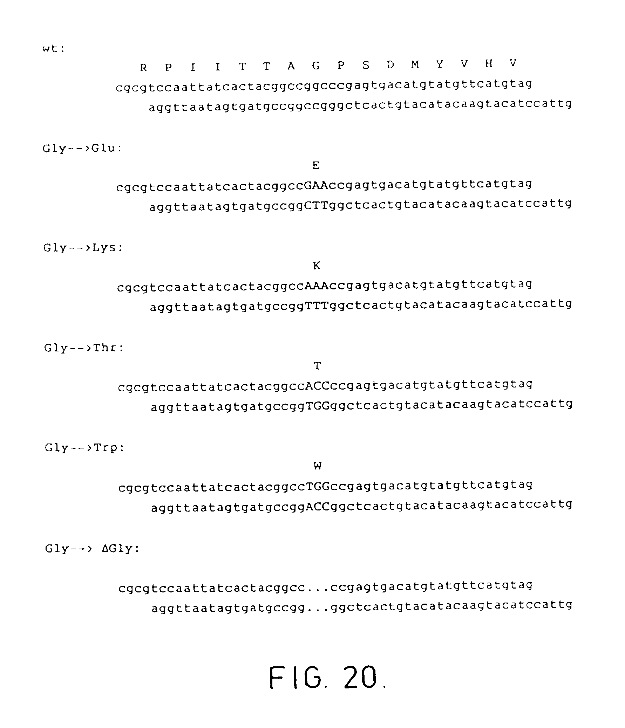

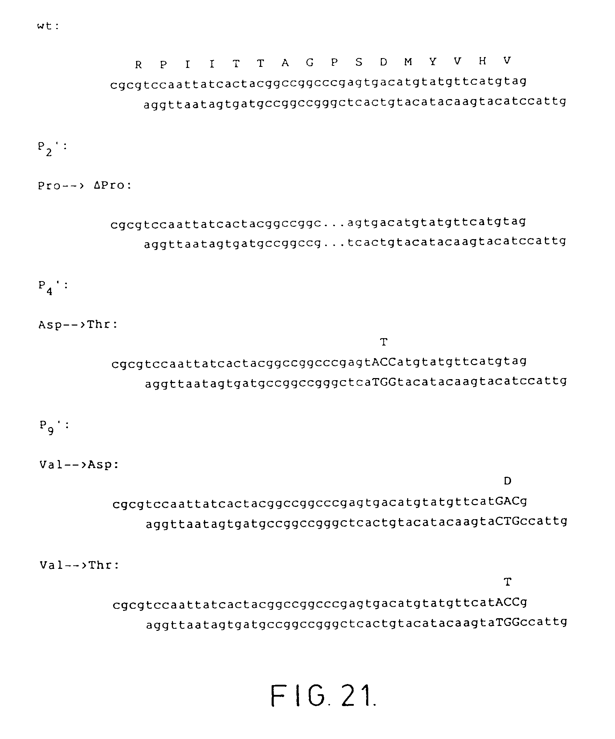

Um den Einfluß von Mutationen an den $P_1'$, $P_2'$, $P_4'$ und $P_9'$-Stellen auf die Spalteffizienz zu untersuchen, wurden die in Fig. 20 und Fig. 21 dargestellten Oligonukleotide in die jeweiligen Stellen eingeführt. Konstruktionen, die die Aminosäuren Tryptophan, Lysin, Threonin und Glutaminsäure an der $P_1'$ Stelle aufweisen, wurden nicht gespalten, was aus dem Fehlen der dem gespaltenen Material entsprechenden Bande bei 50kd hervorgeht. Eine Konstruktion, in der das Glycin an der $P_1'$ Stelle deletiert worden

war, wurde ebenfalls nicht gespalten. Aus diesen Ergebnissen kann abgeleitet werden, daß, im Gegensatz zu der $P_1$ Stelle, Änderungen in der $P_1'$ Stelle zu einem Polypeptid führen, das nicht gespalten werden kann. Die Konstruktion, die eine Deletion des Prolinrestes an der $P_2'$ Stelle besitzt, wurde nicht gespalten. Der Austausch des Valinrestes an der $P_9'$ Stelle durch Asparaginsäure führte zu einer 30% Reduktion der Spalteffizienz, mit Threonin wurde hingegen die Effizienz nicht beeinflußt. Die Anwesenheit eines Threoninrestes an der $P_4'$ Stelle zeigte auch keinerlei Einfluß auf die Wirkung der HRV2-2A Protease. Diese Ergebnisse deuten darauf hin, daß die negative Ladung der Asparaginsäure an der $P_4'$ Stelle nicht essentiell für die Proteaseaktivität ist, daß aber eine negative Ladung an der $P_9'$ Stelle die proteolytische Spaltung beeinträchtigt. Ein Valin/Threonin Austausch an der $P_9'$ Stelle beeinflußt die Aktivität nicht.

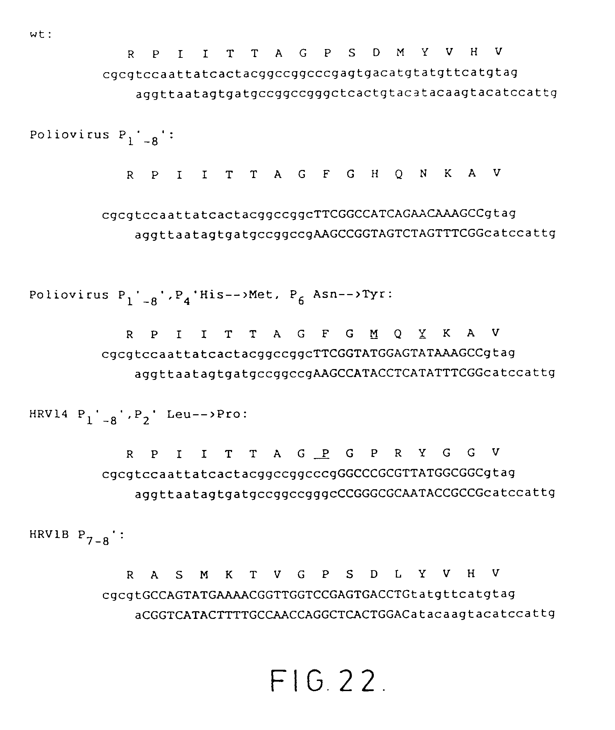

Desweiteren wurde dieses System verwendet, um den Einfluß der Sequenz zwischen $P_1'$ und $P_9'$ auf die proteolytische Aktivität von HRV2-2A zu untersuchen. Als Ausgangsbasis dienten die natürlichen Sequenzen, die in HRV14 und Poliovirus vorkommen (die Sequenzen von Poliovirus Serotyp 1, 2 und 3 (Sabin-Stämme) sind in dieser Region identisch). Die Sequenzen für die Mutagenese sind in Figur 22 gezeigt. Zu erkennen ist eine sehr grosse Variabilität in dieser Region; untersucht werden sollte daher, ob die HRV2-2A solche Spaltstellen erkennen kann. Das exprimierte Protein der Konstruktion mit der $P_1'$ - $P_9'$ ($P_2'$ Leu -> Pro) Sequenz von HRV14 wurde nicht prozessiert. Eine Spaltung des exprimierten Proteins wurde weder in der Konstruktion beobachtet, die die $P_1'$ - $P_9'$ Sequenz von Poliovirus besitzt, noch in einer sehr ähnlichen Konstruktion, die eine Variante der $P_1'$ - $P_9'$ Poliosequenz darstellt( $P_4'$ His -> Met, $P_6'$ Asn -> Tyr).

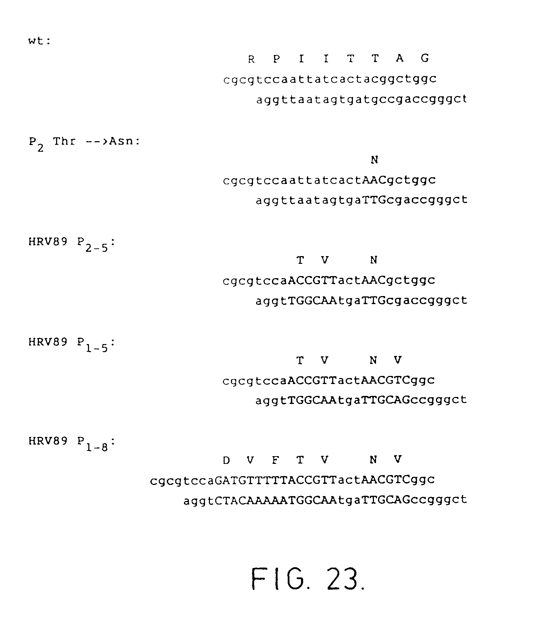

Wie gezeigt werden konnte, wird das exprimierte Protein einer Konstruktion mit Valin an der $P_1$ Stelle oder mit jenen Aminosäuren, die zwischen $P_{1-4}/P_1'_{-4}$'in HRV89 zu finden sind, nur zu 50% gespalten (Beispiel 6). Dieser Befund wurde durch den Austausch von Aminosäuren an weiteren Positionen des Spaltsignals ausgedehnt, um den Einfluß der einzelnen Aminosäuren in dieser Region zu untersuchen. Ein Vergleich der Aminosäuresequenzen der beiden HRV-Serotypen ist in Figur 23 zu sehen. Aus der Spaltung der exprimierten Proteine geht klar hervor, daß die Protease HRV2-2A jene Spaltstellen, die die Aminosäuren von HRV89 aufweisen, nicht gut erkennen kann. Hierfür verantwortlich ist zur Hauptsache die Anwesenheit von Valin an der $P_1$ Stelle, wie aus dem Unterschied der Mutationen HRV89 $P_{2-5}$ und $P_{1-5}$ zu ersehen ist. Zum Beispiel ist eine Reduktion der Spaltung um 30% durch den Alanin --> Valin Austausch zu sehen. Um dieses Ergebnis zu untermauern, wurde jene Sequenz eingebaut, die der der Spaltstellenregion des humanen Rhinovirus Serotyp 1B entspricht:

```
Arg-Pro-Ile-Ile-Thr-Thr-Ala-Gly-Pro-Ser-Asp-Met-Tyr-Val-
His-Val · gegen
```

```
Arg-Ala-Ser-Met-Lys-Thr-Val-Gly-Pro-Ser-Asp-Leu-Tyr-Val-
His-Val.
```

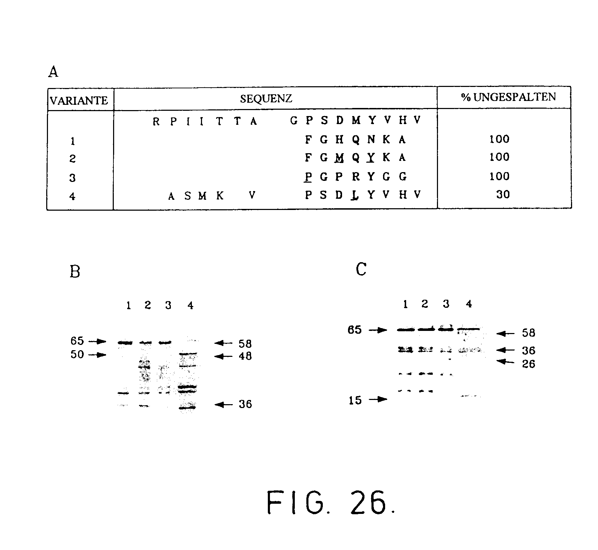

Die Sequenz hat an der $P_1$ Stelle einen Valinrest; das Expressionsprodukt wurde nur zu 65% gespalten, wie in Figur 26 zu sehen ist.

Gegenstand der vorliegenden Erfindung sind daher auch Oligonukleotide, die für eine modifizierte VP1/P2A Region kodieren, wobei die Modifikationen bevorzugt an der Spaltstelle oder in der Spaltregion der Protease 2A vorliegen und die "cis"-Aktivität beeinflussen.

Gegenstand der vorliegenden Erfindung sind auch die von den Oligonukleotiden abgeleiteten Oligopeptide; Oligopeptide, die Mutationen aufweisen, die nicht gut in das aktive Zentrum der Protease 2A passen. Bevorzugte Mutationen sind die Aminosäuren Valin oder Isoleucin an der P1-Stelle und Val-Thr-Asn-Val-Gly-Pro-Ser-Ser an der $P_{1-4}/P_1'_{-4}$'-Stelle. Auch die hiervon abgeleiteten Peptidomimetika, die beispielsweise Etherbindungen aufweisen, Peptide die mit Marker versehen sind oder beispielsweise D-Aminosäuren aufweisen, sind Gegenstand der vorliegenden Erfindung. Besonders geeignet sind diese Verbindungen als Leitsubstanzen bei der Wirkstoffindung zur Beeinflussung der "cis"-Aktivität der Protease 2A, insbesondere als Leitsubstanzen für Inhibitoren. Hergestellt werden können die Oligopeptide nach an sich bekannten Verfahren, wie zum Beispiel der "solid phase"- Methode nach Merrifield. Die Herstellung der von Oligopeptiden abgeleiteten Peptidomimetika ist dem Fachmann an sich bekannt. Beispielhaft verwiesen sei auf die Literaturstelle J.-L. Fauchère, Advances in Drug Research, Vol. 15 (1986), Seite 29-69.

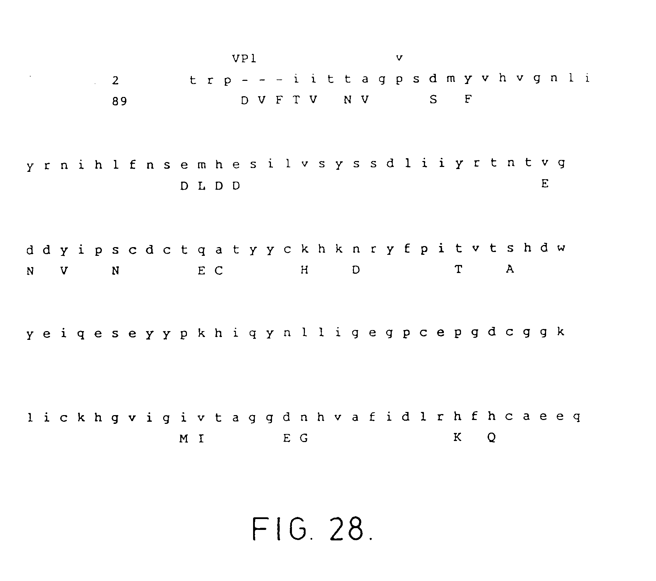

Um zu zeigen, daß auch das 2A Gen von HRV89 (EPA 261 403) für eine Protease kodiert, wurde eine DNA Konstruktion hergestellt, in der ein DNA Fragment, das der Region des 2A Gens entspricht, an das für das HRV2-VP1 kodierende DNA Fragment gebunden wurde. Nach Expression dieses Hybrids im pEx System kann aus der Länge des Fusionsproteins darauf geschlossen werden, ob, das 2A Protein von HRV89 als Protease fungiert. Die Unterschiede im 2A Gen zwischen HRV2 und HRV89 sind in Figur 28 zu sehen. Zwei Mutanten wurden außerdem konstruiert, um den Einfluß einzelner Aminosäuren auf die Spalteffizienz zu prüfen.

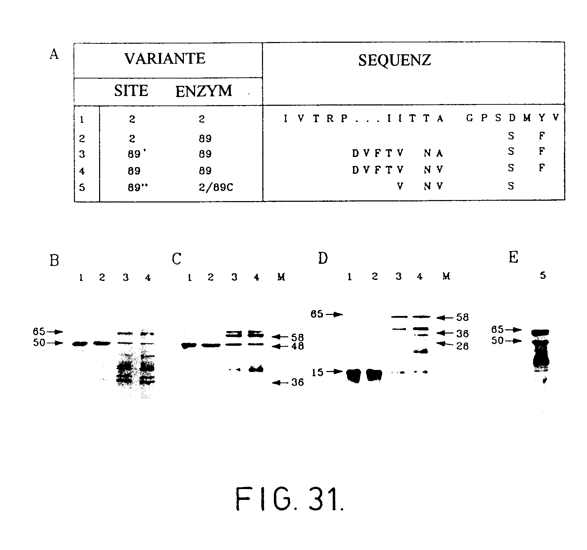

Die Analyse der Expressionsprodukte der drei Konstruktionen mit der HRV89-2A ist in Figur 31 zu sehen. Das Fusionsprotein der Mutante, die die HRV2 Spaltstelle (pEx2A/S2/89) besitzt, wird fast vollständig gespalten; das HRV89-2A Enzym kann daher die HRV2 Spaltstelle genauso effizient wie das HRV2-2A Enzym erkennen. Das ungespaltene Produkt wandert bei der Konstruktion mit dem HRV89-2A etwas langsamer, obwohl die beiden 2A-Proteine die gleiche Zahl an Aminosäuren besitzen. Möglicherweise haben die 13 Unterschiede in der Aminosäuresequenz einen Einfluß auf die Mobilität des 2A Polypeptids. Die Interpretation der Ergebnisse mit den anderen zwei Konstruktionen mit der HRV89-2A (pEx2A/S89/89 und pEx2A/S89'/89) wurde dadurch erschwert, daß zusätzlich zu den erwarteten 65kD und 50kD Produkten zwei andere Banden mit Molekulargewichten von 62kD und 60kD gefunden wurden. Eine genauere Überprüfung der Banden von der Mutante pEx2A/S2/89 mit der HRV2 Spaltstelle zeigte, daß diese Banden auch bei dieser Konstruktion vorhanden waren. Diese Banden wurden nicht von anti-PC20 erkannt und besitzen daher nicht das C-terminale Ende vom 2A Protein; es scheint daher möglich, daß die Banden durch ein Pausieren der Ribosomen oder einen Kettenabbruch in der Region vor dem C-Terminus des Polypeptids verursacht wurde. Obwohl es nur vier Unterschiede in dieser Region zwischen den 2A Polypeptiden des HRV2 und des HRV89 auf der Proteinebene gibt, ist die "Codon-Usage" sehr verschieden. Die Analyse der Expressionsprodukte von der Intermediärkonstruktion pEx2A/S89''/C89 zeigte auch ein etwas größeres, ungespaltenes Produkt und die Anwesenheit von zwei zusätzlichen Banden, was darauf hinweist, daß die Gründe dafür mit dem Bereich der letzten 20 Aminosäuren in Zusammenhang stehen.

Trotz der Anwesenheit der zwei zusätzlichen Banden ist es offensichtlich, daß in dem hier beschriebenen Expressionssystem die HRV89-2A die HRV2-2A Spaltstelle besser erkennen kann als ihre eigene, was durch den höheren Anteil an ungespaltenem Produkt bei den Konstruktionen pEx2A/S89'/89 und pEx2A/S89/89 zu erkennen ist. Offenbar ist dabei die Anwesenheit von Alanin an der $P_1$ Stelle für die Spaltung durch HRV2-2A ausschlaggebend. Bei der HRV89-2A ist hingegen die Situation etwas anders: das HRV89-2A Enzym scheint seine eigene Spaltstelle nicht besonders gut zu erkennen. Es ist möglich, daß in vivo Teile vom 2B für die Aktivität von HRV89-2A notwendig sind, möglicherweise haben sie einen Einfluß auf die Konformation des 2A-Polypeptids. Auch die Konformation der Kapsidproteine von HRV89 könnte eine Rolle bei der Spaltung spielen. Denkbar wäre es, daß der langsamen Spaltung eine biologische Funktion z. B. bei der Virusreplikation zukommt, ist es doch eine Tatsache, daß der HRV89 Serotyp deutlich langsamer wächst als der HRV2 Serotyp.

Wie bereits erwähnt, ist die P2A des Poliovirussystems dafür verantwortlich, daß ein oder mehrere Regulationsfaktoren der Translation der Wirtszelle während der Infektion verändert werden. Diese Befunde lassen sich auch auf das rhinovirale System übertragen (Etchison, D., Foud, S. (1985) J. Virol. 54, S. 634-638). Die für diese Veränderung der Regulationsverfahren verantwortliche "trans-"Aktivität der P2A ließ sich im Rhinovirussystem für die Protease 2A nachweisen.

Die Beeinflussung oder Inhibierung dieses Prozesses würde einen therapeutisch positiven, wenn auch untergeordneten Effekt auf das virale Geschehen ausüben.

Eine weitere Aufgabe der vorliegenden Erfindung bestand daher darin, die für die "trans"-Aktivität erforderlichen Parameter zu ermitteln.

Gelöst wurde diese Aufgabe durch die Bereitstellung verschiedener Peptidsubstrate und die Ermittlung verschiedener Inkubationsbedindungen.

Die Versuche zeigen, daß die Spalteffizienz der HRV2-2A in trans wesentlich von der Länge des verwendeten Spaltpeptids abhängt und die Spaltspezifität weniger durch das Spaltsignal selbst determiniert ist, als vielmehr die Aminosäuresequenz der Spaltregion einen wesentlichen Einfluß auf die "Erkennbarkeit" einer Spaltstelle ausübt.

Gegenstand der vorliegenden Erfindung sind daher auch Oligopeptide, die von der Protease 2A mit unterschiedlicher Effizienz gespalten werden. Besonders bevorzugt solche Oligopeptide, die zumindest fünf Aminosäuren der C-terminalen Region von VP1 und acht Aminosäuren vom N-Terminus der Protease 2A oder jeweils sechs Aminosäuren aus diesen Regionen aufweisen. Sie können auf ihre Eignung als kompetitive Inhibitoren der "trans"-Aktivität untersucht werden und gegebenenfalls therapeutisch bei rhinoviralen Infektionen eingesetzt werden. Besonders geeignet sind diese Verbindungen jedoch als Leitsubstanzen bei der Wirkstoffindung zur Beeinflussung der "trans"-Aktivität der Protease 2A, insbesondere als Leitsubstanzen für Inhibitoren. Hergestellt werden können die Oligopeptide nach an sich bekannten Verfahren, wie zum Beispiel der "solid phase"- Methode nach Merrifield.

Die vorliegenden Ergebnisse geben wertvolle Anhaltspunkte für die Struktur/Wirkungs-bezogene Suche nach Wirkstoffen, die die "cis"- und/oder "trans"-Aktivität der rhinoviralen Proteasen therapeutisch wirksam beeinflussen.

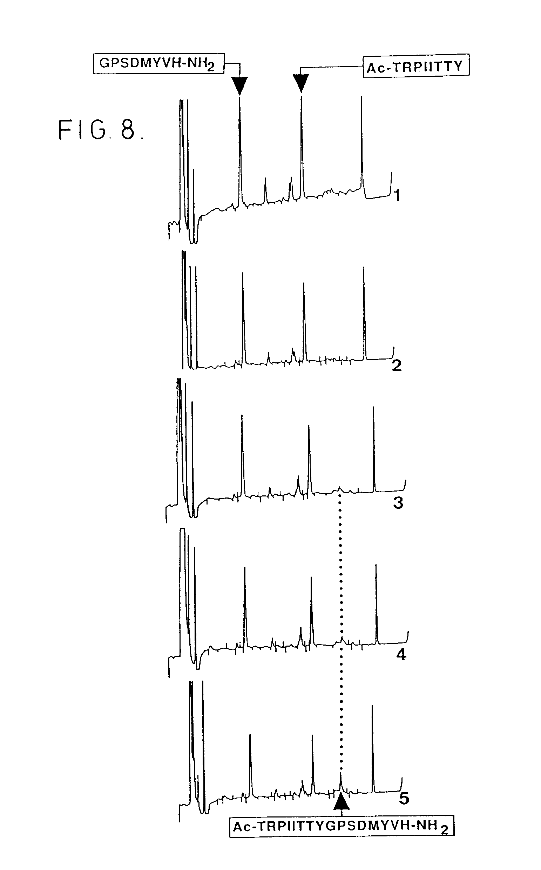

Bei der Austestung von verschiedenen Inkubationsbedingungen konnte gezeigt werden, daß in Anwesenheit von 2,5 M NaCl im "trans"-Aktivitätspuffer (siehe Tab. 1) eine vollständige Spaltung des Peptidsubstrats durch HRV2-2A erfolgt und keinerlei unspezifischer Abbau der Peptidsubstrate und deren Spaltprodukte mehr eintritt (gezeigt am Beispiel der Transspaltung von Peptid K; siehe Fig. 8). Drei Gründe können für diesen Salzeffekt angeführt werden:

a) Beeinflussung der Löslichkeit bzw. Stabilisierung der Peptidsubstrate, deren Spaltprodukte und der HRV2-2A im Zellextrakt durch hohe Salzkonzentrationen.

b) Unterdrückung des unspezifischen Abbaus der Peptide, deren Spaltprodukte und der HRV2-2A durch Inhibition der wirtszellspezifischen E. coli Proteasen im Zellextrakt.

c) Förderung einer geeigneten Struktur der HRV2-2A, die die "trans"-Aktivität begünstigt bzw. die Substratbindung erleichtert.

Zur weiteren Charakterisierung der HRV2-2A wurde der Einfluß verschiedener Detergentien, Lösungs- und Denaturierungsmittel auf die Effizienz der "trans"-Aktivität von HRV2-2A untersucht. Dabei stellte sich heraus, daß sämtliche verwendeten Detergentien und sogar Glycerin einen drastischen negativen Einfluß auf die "trans"-Aktivität von HRV2-2A ausüben (Tab. 1). Selbst bei niedrigsten Verdünnungsstufen wurde -mit Ausnahme von DMSO- eine 30%-ige Inhibierung festgestellt. Substanzen wie Ammoniumsulfat, Guanidinium-Hydrochlorid und Harnstoff zeigten dagegen einen vergleichsweise wesentlich geringeren Einfluß auf die "trans"-Aktivität. Die Verwendung von hohen Ionenstärken inhibierte die "trans"-Aktivität nicht, sondern führte im Gegenteil zu einer erhöhten Effizienz der "trans"-Aktivität (100% Spaltung des Peptidsubstrats, Unterdrückung des unspezifischen Abbaus der Peptidspaltprodukte usw.).

Diese Ergebnisse können erste Hinweise über den Mechanismus der "trans"-Aktivität von HRV2-2A geben und erlauben eine Aussage über eine mögliche strukturelle Beschaffenheit der Protease 2A während der Spaltung von Peptiden in trans.

- Einerseits ist bekannt, daß die Anwesenheit von hohen Salzkonzentrationen (2,5 M NaCl) die Ausbildung von nicht-monomeren Strukturen (z.B. dimeren Strukturen) begünstigt,

- andererseits aber verhindern milde Detergentien diese Struktur (z.B. Tween, NP40, Glycerin usw.) was sich wie im oben gezeigten Fall in der Reduktion der "trans"-Aktivität äußert.

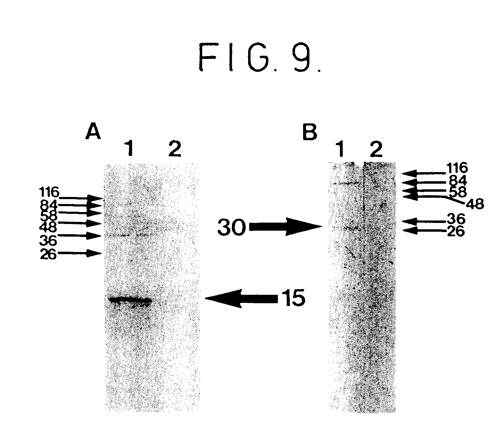

Die Möglichkeit, daß die HRV2-2A in einer multimeren Form vorliegt wird durch die Tatsache untermauert, daß bei einer Auftrennung von HRV2-2A enthaltenden E. coli Zellextrakten auf einem nicht-denaturierenden Proteingel und anschließendem Immunblot mit anti-PC20 die Protease nur mehr in einer höhermolekularen, ihrer dimeren Form entsprechenden molekularen Masse nachgewiesen werden kann (siehe Fig. 9).

Wie durch Deletionsstudien und in vitro Mutagenese des C-terminalen Endes der Protease 2A bereits gezeigt werden konnte, ist diese Region für die "cis"-Aktivität essentiell (Sommergruber, W. et al., loc. cit.). Anti-PC20, ein polyklonales Antiserum, das gegen die letzten 20 Aminosäuren der nativen Protease 2A gerichtet ist, übt jedoch keinen Einfluß auf die "trans"-Aktivität von 2A aus. Dies bedeutet, daß:

a) entweder der C-Terminus von HRV2-2A für die "trans"-Aktivität nicht essentiell ist oder daß

b) der C-terminale Bereich unter diesen Bedingungen für das Antiserum nicht zugänglich ist; d.h. die Antikörper können aus strukturellen Gründen nicht an die antigene Stelle binden.

Die vorliegenden Ergebnisse geben wertvolle Anhaltspunkte für die strukturwirkungsbezogene Suche nach Wirkstoffen, die die "trans"-Aktivität der viralen Proteasen, insbesondere der rhinoviralen Proteasen therapeutisch wirksam beeinflussen.

Gegenstand der vorliegenden Erfindung sind insbesondere ein:
DNA-Molekül, das für die VP1/P2A-Region des rhinoviralen Systems kodiert und in dem Bereich der Protease 2A nicht - Wildtyp - singuläre Signalsequenzen für Restriktionsenzyme aufweist, wobei vorzugsweise als rhinovirales System der HRV2- oder der HRV89-Serotyp verwendet wird und die Signalsequenzen vorzugsweise für die Restriktionsenzyme XmaI, SmaI, XmaIII, AvaI, NaeI, MluI, EagI, BglII oder SnaBI erkennbar sind.

Besonders bevorzugt ist ein DNA-Molekül, das das Expressionsplasmid pEx2A/21, pEx2A/II, pEx2A/P$_{1-4}$/1$_{-4}$'/89, pEx2A/S89''/C89, pEx2A/S89/89, pEx2A/S2/89 oder pEx2A/S89/89 ist.

Weiterhin sind Oligonukleotide Gegenstand der Erfindung, die für eine modifizierte VP1/P2A Region des rhinoviralen Systems kodieren, wobei die Modifikationen in der gesamtkodierenden Region, vorzugsweise an der Spaltstelle oder in der Spaltregion der Protease 2A vorliegen und vorzugsweise deren "cis"-Aktivität beeinflussen, sowie Oligonukleotide, die für eine modifizierte VP1/P2A Region des rhinoviralen Systems kodieren, wobei die Modifikationen zu einer Störung des aktiven Zentrums der rhinoviralen Protease 2A führen.

Bevorzugt sind Oligonukleotide, bei denen

- die Modifikationen für die Aminosäuren Val, Ile, Tyr, Gln, Leu, Met, Phe, Thr insbesondere für Valin oder Isoleucin an der oder um die $P_1$-Stelle der Protease HRV2-2A kodieren,
- die Modifikationen für Trp, Lys, Thr, Glu oder $\Delta$ Gly an der $P_1$'-Stelle der Protease HRV2-2A kodieren,
- die Modifikationen für $\Delta$ Pro, Asp, Thr, insbesondere für die Deletion von Prolin oder für Asparaginsäure um die $P_1$'-Stelle der Protease HRV2-2A kodieren,
- die Modifikationen für die Aminosäuren V-T-N-V-G-P-S-S an der $P_{1-4}/P_1$'$_{-4}$-Stelle der Protease HRV2-2A kodieren,
- die Modifikationen für die Aminosäuren G-F-G-H-Q-N-K-A an der $P_1$'$_{-8}$' Stelle der Protease HRV2-2A kodieren,
- die Modifikationen für die Aminosäuren G-F-G-M-Q-Y-K-A an der $P_1$'$_{-8}$' Stelle der Protease HRV2-2A kodieren,
- die Modifikationen für die Aminosäuren G-P-G-P-R-Y-G-G an der $P_1$'$_{-8}$' Stelle der Protease HRV2-2A kodieren,
- die Modifikationen für die Aminosäuren R-A-S-M-K-T-V-G-P-S-D-L-Y-V-H an der $P_{7-8}$' Stelle der Protease HRV2-2A kodieren,
- die Modifikation für die Aminosäure Asparagin an der $P_2$ Stelle der Protease HRV2-2A kodiert,
- die Modifikationen für die Aminosäuren T-V-T-N-A an der $P_{1-5}$ Stelle der Protease HRV2-2A kodieren,
- die Modifikationen für die Aminosäuren T-V-T-N-V an der $P_{1-5}$ Stelle der Protease HRV2-2A kodieren oder
- die Modifikationen für die Aminosäuren D-V-F-T-V-T-N-V an der $P_{1-8}$ Stelle der Protease HRV2-2A kodieren,
- die Modifikationen für die Aminosäure Alanin an der $P_1$ Stelle der Protease HRV89-2A oder für die Aminosäuren A-T-T-I-I an der $P_{1-5}$ Stelle der Protease HRV89-2A kodieren.

Gegenstand der vorliegenden Erfindung sind weiterhin Testsysteme, die in einem geeigneten Expressionssystem eines der obengenannten DNA-Moleküle enthalten.

Besonders bevorzugt sind die Testsysteme pEx2A/21, pEx2A/II, pEx2A/$P_{1-4}$/$_1$'$_{-4}$'/89, pEx2A/S89''/C89, pEx2A/S89/89, pEx2A/S2/89 oder pEx2A/S89/89, sowie diese Systeme, bei denen unter Ausnutzung der nicht - Wildtyp - singulären Signalsequenzen eines der obengenannten Oligonukleotide eingefügt ist.

Gegenstand der vorliegenden Erfindung sind Oligopeptide, die von einem der obengenannten Oligonukleotide kodiert wird, sowie davon abgeleitete Peptidomimetika; Oligopeptide, die von der Protease 2A in trans gespalten werden, vorzugsweise Oligopeptide, die zumindest fünf Aminosäuren der C-terminalen Region von VP1 und acht Aminosäuren vom N-Terminus der Protease HRV2-2A oder jeweils sechs Aminosäuren aus diesen Regionen aufweisen, besonders bevorzugt die Oligopeptide H, A, B, C, G, F oder K.

Diese Oligopeptide oder davon abgeleitete Peptidomimetika können Verwendung finden als Arzneimittel oder als Prophylaktika beziehungsweise sie können verwendet werden zur Herstellung von Arzneimitteln.

Gegenstand der Erfindung sind auch Verfahren zur Herstellung eines DNA-Moleküls, das für die VP1/P2A Region des rhinoviralen Systems kodiert, dadurch gekennzeichnet, daß in den Bereich der Protease 2A nicht - Wildtyp - singuläre Signalsequenzen für Restriktionsenzyme eingeführt werden, wobei als rhinovirales System vorzugsweise der HRV2- oder HRV89-Serotyp verwendet wird und die Signalsequenzen vorzugsweise für die Restriktionsenzyme XmaI, SmaI, XmaIII, AvaI, NaeI, MluI, EagI, BglII oder SnaBI erkennbar sind;

Verfahren zur Herstellung eines Oligonukleotids, das für eine modifizierte VP1/P2A Region des rhinoviralen Systems kodiert, dadurch gekennzeichnet, daß Modifikationen in der gesamtkodierenden Region, vorzugsweise in die Spaltstelle oder in die Spaltregion der Protease 2A eingefügt werden, wobei die Modifikationen vorzugsweise die "cis"-Aktivität der Protease 2A beeinflussen, besonders bevorzugt zu einer Störung des aktiven Zentrums der rhinoviralen Protease 2A führen, insbesondere Verfahren, bei denen

- die Modifikationen für die Aminosäuren Val, Ile, Tyr, Gln, Leu, Met, Phe, Thr insbesondere für Valin oder Isoleucin an der oder um die $P_1$-Stelle der Protease HRV2-2A kodieren,
- die Modifikationen für Trp, Lys, Thr, Glu oder $\Delta$ Gly an der $P_1$'-Stelle der Protease HRV2-2A kodieren,
- die Modifikationen für $\Delta$ Pro, Asp, Thr, insbesondere für die Deletion von Prolin oder für Asparaginsäure um die $P_1$'-Stelle der Protease HRV2-2A kodieren,
- die Modifikationen für die Aminosäuren V-T-N-V-G-P-S-S an der $P_{1-4}/P_1$'$_{-4}$'-Stelle der Protease HRV2-2A kodieren,

11

- die Modifikationen für die Aminosäuren G-F-G-H-Q-N-K-A an der $P_1'_{-8}'$ Stelle der Protease HRV2-2A kodieren,
- die Modifikationen für die Aminosäuren G-F-G-M-Q-Y-K-A an der $P_1'_{-8}'$ Stelle der Protease HRV2-2A kodieren,
- die Modifikationen für die Aminosäuren G-P-G-P-R-Y-G-G an der $P_1'_{-8}'$ Stelle der Protease HRV2-2A kodieren,
- die Modifikationen für die Aminosäuren R-A-S-M-K-T-V-G-P-S-D-L-Y-V-H an der $P_{7-8}'$ Stelle der Protease HRV2-2A kodieren,
- die Modifikation für die Aminosäure Asparagin an der $P_2$ Stelle der Protease HRV2-2A kodiert,
- die Modifikationen für die Aminosäuren T-V-T-N-A an der $P_{1-5}$ Stelle der Protease HRV2-2A kodieren,
- die Modifikationen für die Aminosäuren T-V-T-N-V an der $P_{1-5}$ Stelle der Protease HRV2-2A kodieren,
- die Modifikationen für die Aminosäuren D-V-F-T-V-T-N-V an der $P_{1-8}$ Stelle der Protease HRV2-2A kodieren,
- die Modifikationen für die Aminosäure Alanin an der $P_1$ Stelle der Protease HRV89-2A kodieren oder
- für die Aminosäuren A-T-T-I-I an der $P_{1-5}$ Stelle der Protease HRV89-2A kodieren;

Verfahren zur Herstellung eines Testsystems, dadurch gekennzeichnet, daß in ein geeignetes Expressionssystem eines der genannten DNA-Moleküle eingefügt wird; vorzugsweise ein Verfahren bei dem in dem Expressionsplasmid pEx2A/21, pEx2A/II, pEx2A/$P_{1-4}/_1'_{-4}'$/89, pEx2A/S89''/C89, pEx2A/S89/89, pEx2A/S2/89 oder pEx2A/S89/89 unter Ausnutzung der nicht - Wildtyp - singulären Signalsequenzen eines der genannten Oligonukleotide eingefügt wird.

Unter "Testsytem" sind rekombinante Expressionssysteme zu verstehen, die einerseits zur Produktion von Wildtyp-HRV2 2A und deren Mutanten (insbesondere Mutanten des aktiven Zentrums und der Spaltstellenregion), andererseits als Testsysteme zum Auffinden geeigneter Inhibitoren verwendet werden können, wobei Teilsequenzen dieser Systeme als Grundlage zur Synthese von therapeutisch wirksamen Oligonukleotiden und Oligopeptiden dienen.

**Legende zu den Figuren**

In den Figuren lautet der "single-letter-code" für die Aminosäuren:
A, Alanin; D, Asparaginsäure; E, Glutaminsäure; F, Phenylalanin; G, Glycin; H, Histidin; I, Isoleucin; K, Lysin; L, Leucin; M, Methionin; N, Asparagin; P, Prolin; Q, Glutamin; R, Arginin; S, Serin; T, Threonin; V, Valin; W, Tryptophan; Y, Tyrosin.

Wenn nicht anders angegeben, wird bei der Angabe der Mutationen in der Spaltstellenregion Bezug genommen auf den Wildtyp des HRV2 bzw. HRV89 (HRV2:$P_1$ = Ala; $P_1'$ = Gly; HRV89:$P_1$ = Val; $P_1'$ = Gly).

Figur 1 zeigt die Sequenz der Oligonukleotide wie sie zur Mutagenese der Spaltstellenregion von HRV2-2A verwendet wurden.

Die Kleinbuchstaben geben die ursprüngliche Sequenz der cDNA von HRV2 wieder, bzw. die Sequenz des Expressionsplasmids pEx2A/21, einer Restriktionsenzym-modifizierten Variante von pEx2A (siehe Beispiel 2).

Großbuchstaben weisen auf die jeweilige Mutation hin. wt bedeutet Wildtyp; HRV89 $P_{1-4}/P_1'_{-4}'$ entspricht der Spaltstellenregion wie sie im humanen Rhinovirus vom Serotyp 89 vorgefunden wird.

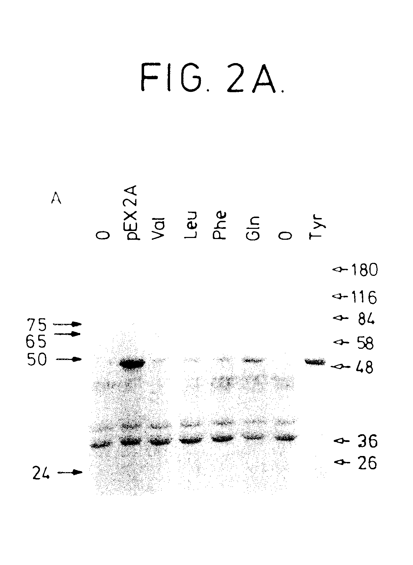

Figur 2A zeigt die Expressionsprodukte der Mutagenese des C-Terminus von VP1; Austausch von Ala gegen Val, Leu, Phe, Gln und Tyr in der $P_1$ Position.

| | |
|---|---|
| Bild A: | Coomassie Blau gefärbtes 12,5% SDS-Polyacrylamidgel. |
| 0: | nicht induzierter pEx2A/21 |
| pEx2A: | induziertes Ausgangsplasmid pEx2A/21 Val, Leu, Phe, Gln: Mutanten von pEx2A/21 mit geänderter Aminosäure in Position P1 (Ala-->Val, Leu,Phe und Gln) |
| Tyr: | Mutante von pEx18521 (Ala-->Tyr in P1-Position); diese Vektorkonstruktion weist einen Teil von 2B auf, der die Erkennung durch anti-PC20 behindert |
| 75 kd | unprozessiertes Expressionsprodukt von pEx18521 |
| 65 kd | unprozessiertes Expressionsprodukt von pEx2A und Mutanten |
| 50 kd | prozessiertes Expressionsprodukt von pEx18521, pEx2A und Mutanten; entspricht dem Fusionsanteil (98 Aminosäuren der MS2-Polymerase), dem C-terminalen Ende von VP3 und dem gesamten VP1 |
| 24 kd | Spaltprodukt von pEx18521, bestehend aus HRV2-2A und dem N-terminalen Abschnitt von 2B |

15 kd      HRV2-2A

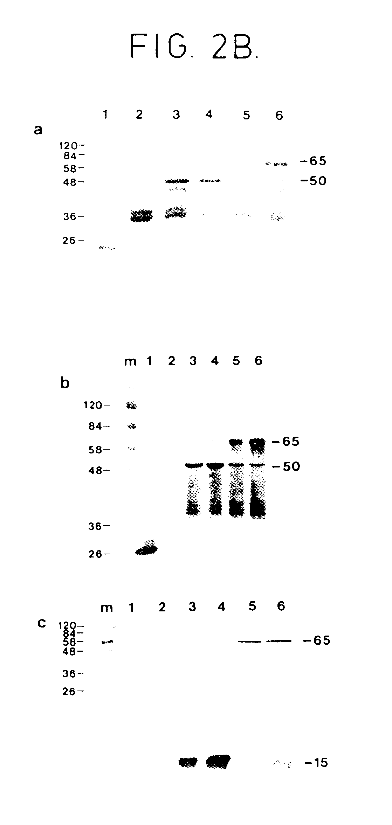

Figur 2B zeigt die Expressionsprodukte der Mutagenese des Spaltsignals für 2A; Austausch von Ala gegen Met und Ile in der Position P1 sowie Austausch der Spaltregion von HRV2 (Ile-Thr-Thr-Ala-Gly-Pro-Ser-Asp) gegen die Spaltregion von HRV89 (Val-Thr-Asn-Val-Gly-Pro-Ser-Ser); betrifft die Positionen $P_{1-4}/P_1'-4'$.

    a:           Coomassie Blau gefärbtes 10% SDS-Polyacrylamidgel

    b:           Western blot eines identischen Gels mit anti-MS2-Pol

    c:           Western blot eines 15% SDS-Polyacrylamidgels mit anti-PC20

Spur m:      Markerproteine

Spur 1:       pVP2D2 (MS2Pol-VP2 Fusionsprotein als positive Kontrolle für den anti-MS2-Pol Antikörper)

Spur 2:       pEx2A/21 nicht induziert

Spur 3:       pEx2A/21 induziert

Spur 4:       pEx2A/21 mit Mutation Ala-->Met in Position P1

Spur 5:       pEx2A/21 mit Mutation Ala-->Ile in Position P1

Spur 6:       pEx2A/21 mit Austausch der Spaltregion HRV2 gegen HRV89; (ITTAGPSD-->VTNVGPSS) in den Positionen $P_{1-4}/P_1'-4'$ (detaillierte Beschreibung siehe Figur 1 bzw. Beispiel 1)

65 kd      unprozessiertes Expressionsprodukt von pEx2A/21 und Mutanten von pEx2A/21

50 kd      prozessiertes Expressionsprodukt von pEx2A/21 und Mutanten

15 kd      HRV2-2A

Figur 3 zeigt schematisch die Neuschaffung von Erkennungsstellen für Restriktionsenzyme um die Spaltstelle von HRV2-2A und im kodierenden Teil von HRV2-2A.

Nicht fett gedruckte Restriktionsenzyme geben die ursprünglich schon in pEx2A vorhandenen Erkennungssequenzen wieder; eingerahmte, fettgedruckte weisen auf die neu eingeführten Erkennungssequenzen in pEx2A/21 und pEx2A/II hin.

AS bedeutet Aminosäuren.

Figur 4 zeigt das doppelsträngige Oligonukleotid zur Herstellung von pEx2A/21, einer Restriktions-modifizierten Variante von pEx2A.

Das Oligonukleotid ist von einer Acc I bzw. einer BstE II Stelle flankiert; die fettgedruckten Restriktionsstellen wurden mit Hilfe der geänderten Nukleotidsequenz des Mutationsoligos unter Beibehaltung der ursprünglichen Aminosäuresequenz in die DNA eingebracht.

Kleinbuchstaben weisen auf die ursprüngliche Sequenz der HRV2-cDNA hin; Großbuchstaben zeigen die modifizierte Sequenz an. Mit einem Strich versehene Nukleotide geben die Erkennungssequenz für das jeweilige Restriktionsenzym an.

Figur 5 zeigt den Western blot eines 15% SDS-Polyacrylamidgels unter Verwendung von anti-PC20.

    Spur 1:      Expressionsprodukt von pEx2A

    Spur 2:      Expressionsprodukt von pEx2A/21, einer Mutante von pEx2A mit neuen singulären Restriktionsstellen in und um die Spaltregion von HRV2-2A.

    Spur 3:      Expressionsprodukt von pEx2A/II, einer Mutante von pEx2A, welche neue singuläre Restriktionsstellen innerhalb der kodierenden Region von HRV-2A aufweist

Der linke Pfeil bei 15 Kd weist auf das durch den Peptidantikörper spezifisch nachgewiesene Expressionsprodukt (HRV2-2A) hin. Rechts sind die molekularen Größenmarker (in Kd) angeführt. In allen drei Fällen ist die HRV2-2A nach wie vor proteolytisch aktiv, wird von anti-PC20 erkannt und zeigt ein Expressionsprodukt von gleicher Größe.

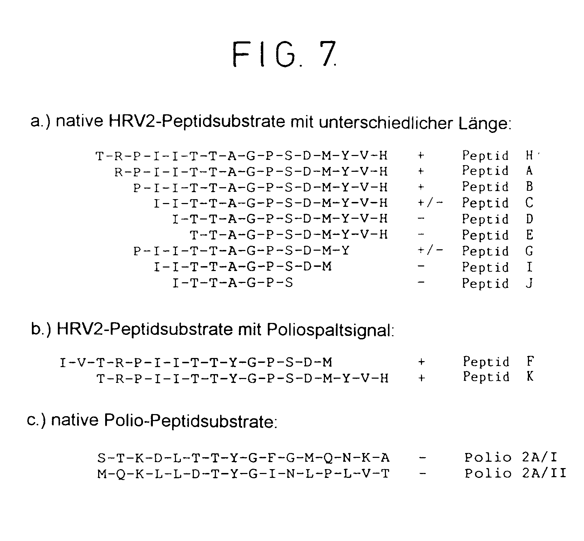

Figur 6 zeigt 3 doppelsträngige Oligonukleotide zur Herstellung von pEx2A/II, einer Mutante mit 2 neuen singulären Restriktionsstellen innerhalb der für die HRV2-2A kodierenden Region.

Die 3 Mutationsoligonukleotide wurden mittels überhängender Enden (durch * oder # gekennzeichnet), welche keine Restriktionsstellen darstellen, aneinanderligiert. Nach erfolgter Ligation kann das ca. 270 bp lange Fragment mit den Restriktionsenzymen BstE II und Apa I nachgeschnitten werden. Kleinbuchstaben weisen auf die ursprüngliche Nukleotidsequenz der HRV2-cDNA hin; Großbuchstaben zeigen die mutierte Sequenz an. Fettgedruckte Restriktionsstellen entsprechen den neu generierten Erkennungssequenzen.

Figur 7 zeigt verschiedene Peptidsubstrate und ihren Einfluß auf die Effizienz der "trans"-Aktivität von HRV2-2A.

    +        Spaltung mit großer Effizienz und vergleichbarer Kinetik.

    -        Peptid kann unter Standardbedingungen (siehe Beispiel 3) nicht gespalten werden.

    +/-      Peptid wird nur mit geringer Effizienz gespalten.

Das Peptid Polio 2A/I repräsentiert die Spaltstelle VP1/2A für die Protease 2A von Poliovirus Typ 1 und 2A/II stellt die alternative Spaltstelle für die Protease 2 A in 3CD für Poliovirus Typ 1 dar.

Figur 8 zeigt HPLC Profile von Peptid K (TRPIITTYGPSDMYVH; tR = 21,6) und dessen Spaltprodukte TRPIITTY (tR = 18,6) und GPSDMYVH (tR = 12,2) nach Inkubation in Überständen von pEx2A; der "trans"-Aktivitätstest wurde wie in Beispiel 3 beschrieben durchgeführt wobei verschiedene NaCl-Konzentrationen verwendet wurden:

1 2,5 M NaCl

2 0,5 M NaCl

3 50 mM NaCl

4 5 mM NaCl

5 0,5 mM NaCl

Die gepunktete Linie weist auf die Position des Peaks hin, der das ungespaltene Peptidsubstrat (tR = 21,6) repräsentiert.

Figur 9 zeigt den Immunblot eines denaturierenden und eines nicht-denaturierenden Proteingels von löslichen Fraktionen aus pEx2A und pEx34b.

| | | |
|---|---|---|
| Figur 9A | Immunblot eines denaturierenden 15% Acrylamidgels mit Hilfe des Peptidantikörpers PC20 |
| Figur 9B | Immunblot eines nicht denaturierenden 15% Acrylamidgels mit-Hilfe des Peptidantikörpers PC20 |
| Spur 1 | löslicher Anteil des E.coli Extraktes von pEx2A |
| Spur 2 | löslicher Anteil des E.coli Estraktes von pEx34b (negative Kontrolle für PC20) |

Die kleinen schmalen Pfeile weisen auf die Position der molekularen Größenmarker hin.

Die dicken Pfeile bei 15 kd auf dem denaturierenden Proteingel (monomere Form) bzw. bei 30 kd auf einem nicht-denaturierenden Proteingel (mögliche dimere Form weisen auf die Position der HRV2-2A spezifischen Bande am Westernblot hin.

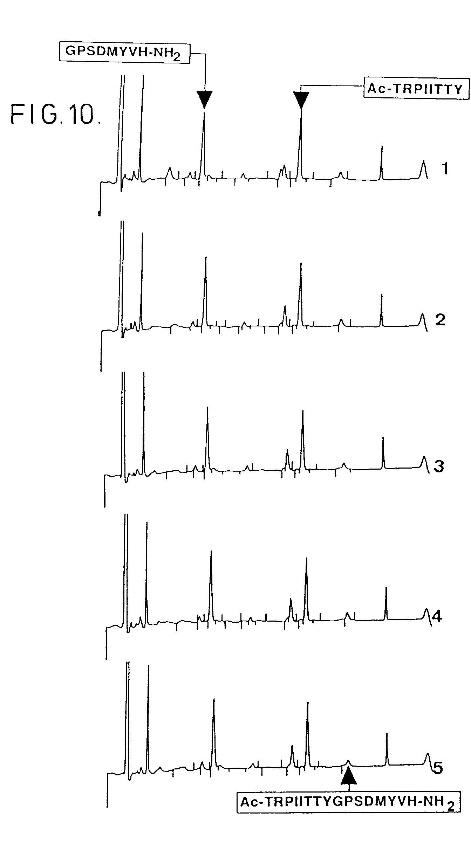

Figur 10 zeigt HPLC-Profile von Peptid K (TRPIITTYGPSDMYVH; tR = 21,6) und dessen Spaltprodukte TRPIITTY (tR = 18,6) und GPSDMYVH (tR = 12,2).

Der "trans"-Aktivitätstest wurde wie in Beispiel 3 beschrieben durchgeführt mit Ausnahme von 150 mM NaCl im "trans"-Aktivitätspuffer und einer 30 min Präinkubation des Zellextraktes von pEx2A in Anwesenheit verschiedener Konzentrationen von anti-PC20. Dabei wurden folgende Verdünnungsstufen von PC20 eingesetzt.

| Spur 1 | 1/1 | PC20 |
|---|---|---|
| Spur 2 | 1/10 | PC20 |
| Spur 3 | 1/100 | PC20 |
| Spur 4 | 1/1000 | PC20 |
| Spur 5 | 1/10 000 | PC20 |

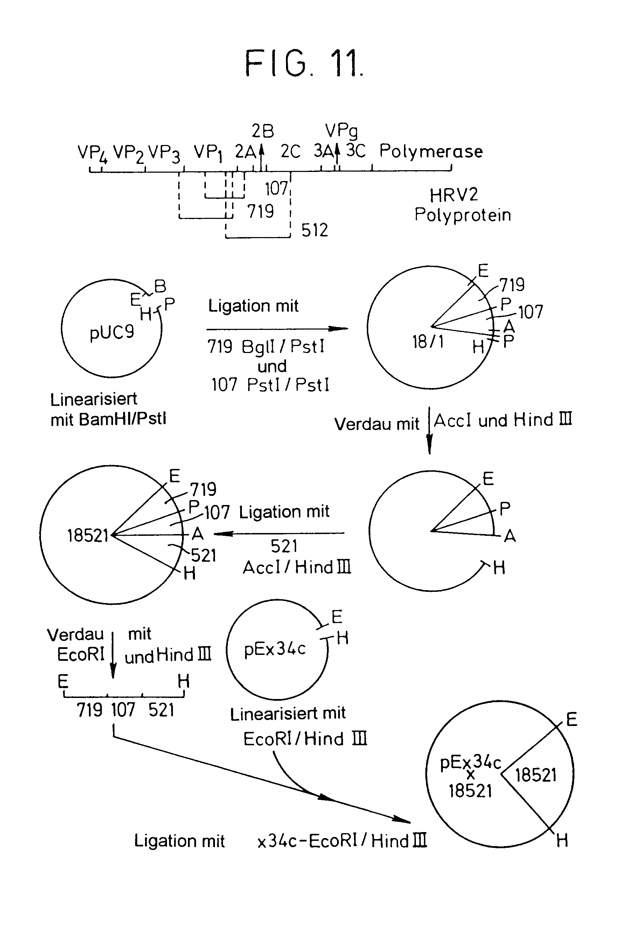

Figur 11: Konstruktion von pEx34c x 18521

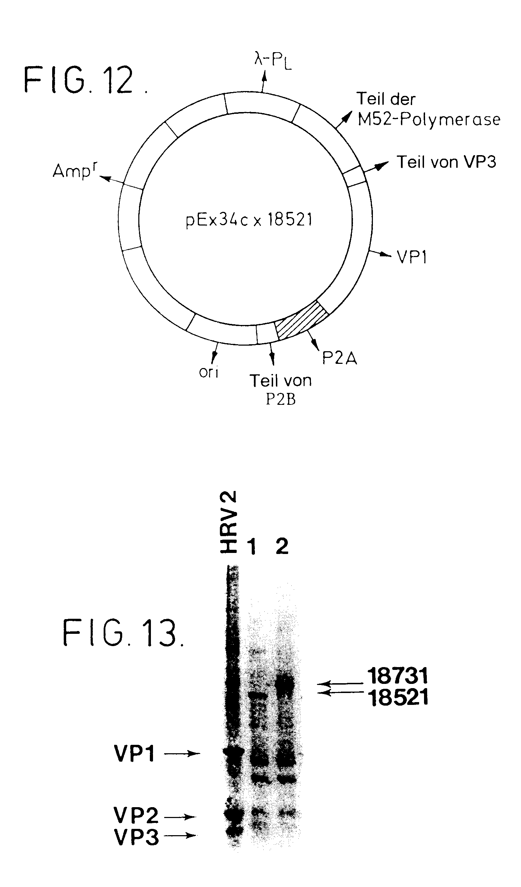

Figur 12: Aufbau des Expressionsplasmides pEx34c x 18521

Figur 13: Elektrophoretische Trennung der Expressionsprodukte von pEx34c x 18521 (Spur 1) und pEx34c x 18731 (Spur 2), sowie der viralen Hüllenproteine von HRV2 (Spur HRV2) auf einem 10%igen SDS-Polyacrylamidgel und Anfärbung mit Coomassie-Brillant-Blau.

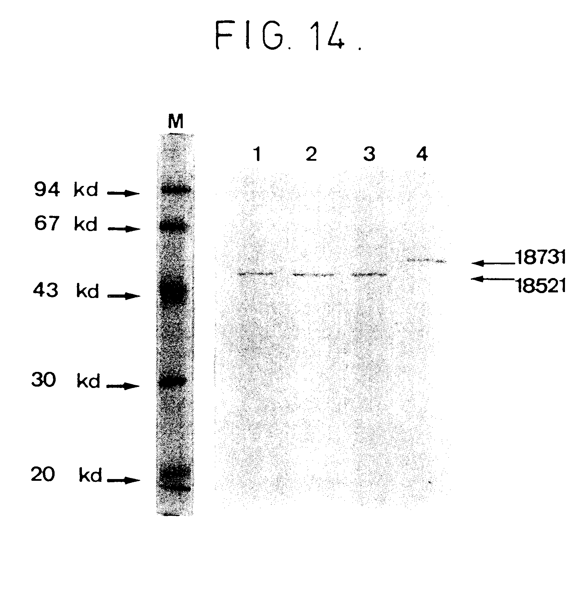

Figur 14: Western blot der Expressionsprodukte von pEx34c x 18521 bzw. 18731 mit einem polyklonalen anti-VP1 Serum; in Spur 1 bis 3 ist das Expressionsprodukt von 3 verschiedenen Klonen von 18521, in Spur 4 von 18731 aufgetrennt. Die nur schwach erkennbaren höhermolekularen Banden in Spur 1 bis 3 repräsentieren das noch unprozessierte Expressionsprodukt von 18521.

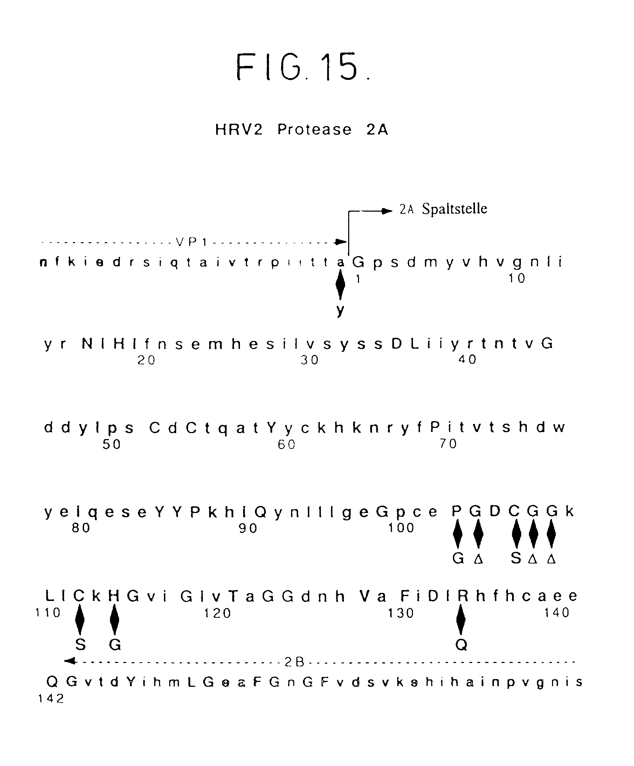

Figur 15: Aminosäuresequenz der HRV2-Protease-2A-Region (fette Buchstaben). Die Großbuchstaben weisen auf die zwischen Rhino-, Polio- und Coxsackieviren identischen Aminosäuren hin. Doppelpfeile zeigen die Position und die Art des Austausches bzw. der Deletion an, die zur Charakterisierung des wahrscheinlich aktiven Zentrums und des C-Terminus der Protease 2A von HRV2 herangezogen wurden.

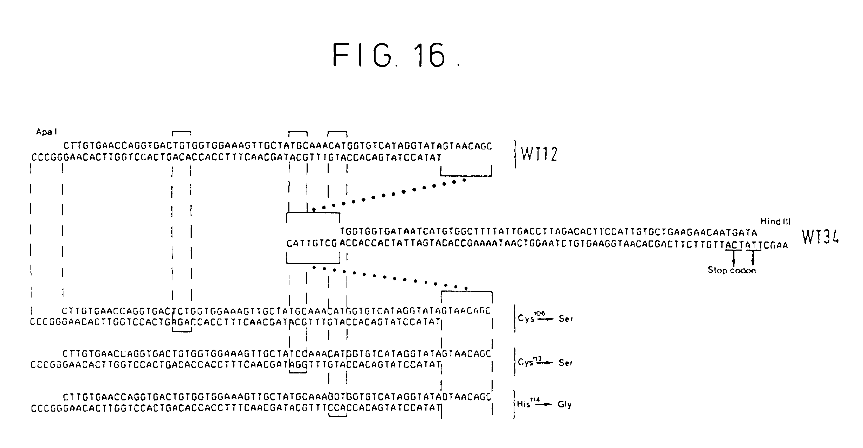

Figur 16: Oligonukleotidkassette zur Mutagenese des wahrscheinlich aktiven Zentrums der Protease 2A von HRV2. Durch Kombination der beiden doppelsträngigen Oligonukleotide WT12 + WT34 erhält man die kodierende Region für die Wildtyp-Protease 2A. Nach der letzten Aminosäure von 2A (Glutamin 142) wurden zwei Stoppkodons eingefügt. Kombiniert man das doppelsträngige Oligonukleotid WT34 anstelle von WT12 mit einem Oligonukleotid, das aufgrund seiner geänderten Basensequenz eine geeignete Mutation oder Deletion besitzt, kann man jede gewünschte Veränderung in der Aminosäuresequenz in

EP 0 410 147 B1

dieser Region herbeiführen (hier gezeigt am Beispiel für die Konstruktion der Wildtyp-2A und den Mutationen für Cys106-->Ser, Cys112-->Ser und His114-->Gly).

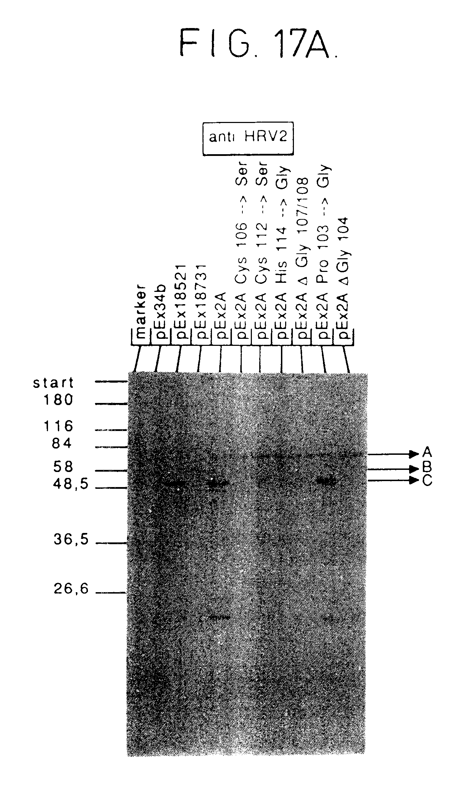

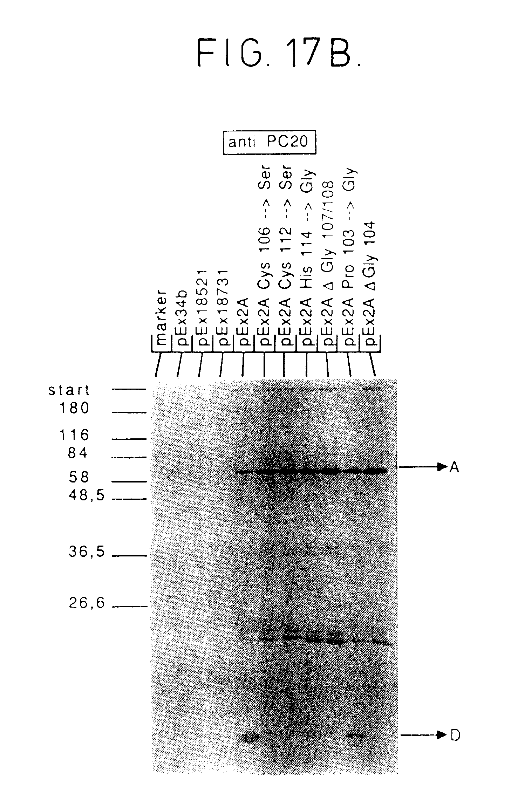

Figur 17 zeigt das Proteinmuster von pEx34c (Negativkontrolle), pEx18521, pEx18731, pEx2A und sämtlichen Mutanten von pEx2A im wahrscheinlich aktiven Zentrum von 2A.

Bande A    entspricht dem unprozessierten Expressionsprodukt (65 K) von pEx2A bzw. von den Mutanten von pEx2A

Bande B    entspricht dem Expressionsprodukt (58 K) von pEx18731

Bande C    entspricht dem prozessierten Expressionsprodukt (50 K; MS2-Polymeraseanteil + C-terminaler Teil von VP3 + gesamtes VP1) von pEx18521, pEx2A und pEx2A[Pro103-->Gly]

Bande D    entspricht der reifen Protease 2A (15 K)

Fig. 17a: Coomassie Blue; mit Coomassie Blue gefärbtes Proteingel

Fig. 17b: Anti HRV2; Western blot des gleichen Gels mit einem polyklonalen Antiserum gegen HRV2

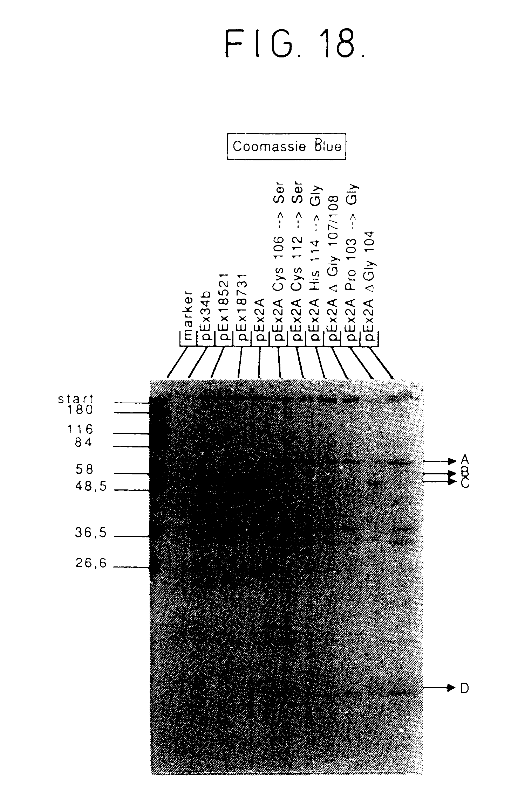

Fig. 18: Anti PC20; Western blot des gleichen Gels mit einem polyklonalen Antiserum gegen PC20 (Peptid das aus den letzten 20 Aminosäuren von 2A aufgebaut ist; siehe Beispiel 7)

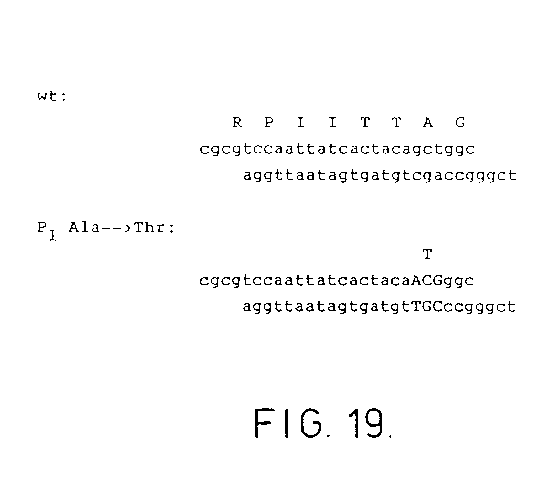

Figur 19 zeigt die Sequenz der Oligonukleotide, wie sie zur Mutagenese der $P_1$ Stelle von HRV2-2A verwendet wurde. Die Kleinbuchstaben geben die ursprüngliche Sequenz der cDNA von HRV2 wieder, bzw. die Sequenz des Expressionsplasmids pEx2A/21, einer Restriktionsenzym-modifizierten Variante von pEx2A (siehe Beispiel 2).

Großbuchstaben weisen auf die jeweilige Mutation hin. wt bedeutet Wildtyp.

Figur 20 zeigt die Sequenz der Oligonukleotide wie sie zur Mutagenese der $P_1$' Stelle von HRV2-2A verwendet wurde.

Die Kleinbuchstaben geben die ursprüngliche Sequenz der cDNA von HRV2 wieder, bzw. die Sequenz des Expressionsplasmids pEx2A/21, einer Restriktionsenzym-modifizierten Variante von pEx2A (siehe Beispiel 2).

Großbuchstaben weisen auf die jeweilige Mutation, die Punkte auf das Deletieren einer Aminosäure hin. wt bedeutet Wildtyp.

Figur 21 zeigt die Sequenz der Oligonukleotide wie sie zur Mutagenese der $P_2$', $P_4$' und $P_9$' Stellen von HRV2-2A verwendet wurde. Die Kleinbuchstaben geben die ursprüngliche Sequenz der cDNA von HRV2 wieder bzw. die Sequenz des Expressionsplasmids pEx2A/21, einer Restriktionsenzym-modifizierten Variante von pEx2A (siehe Beispiel 2).

Großbuchstaben weisen auf die jeweilige Mutation, die Punkte auf das Deletieren einer Aminosäure hin. wt bedeutet Wildtyp.

Figur 22 zeigt die Sequenz der Oligonukleotide, wie sie zur Mutagenese der P' Region bzw. P und P' Regionen von HRV2-2A verwendet wurden. Die Kleinbuchstaben geben die ursprüngliche Sequenz der cDNA von HRV2 wieder bzw. die Sequenz des Expressionsplasmids pEx2A/21, einer Restriktionsenzym-modifizierten Variante von pEx2A (siehe Beispiel 2).

Großbuchstaben weisen auf die jeweilige Mutation hin. wt bedeutet Wildtyp. Poliovirus $P_1$'$_{-8}$' entspricht der P' Region, wie sie in allen drei Poliovirustypen (Sabin-Stämme) vorgefunden wird. Poliovirus $P_1$'$_{-8}$', $P_4$' His-->Met, $P_6$' Asn-->Tyr entspricht der modifizierten Poliovirussequenz, mit den zwei Mutationen. HRV14 $P_1$'$_{-8}$', $P_2$' Leu-->Pro entspricht der Sequenz der P'Region wie sie im humanen Rhinovirus Serotyp 14 vorgefunden wird, mit einer Mutation. HRV1B $P_{7-8}$' entspricht der Sequenz der Spaltstelle wie sie im humanen Rhinovirus Serotyp 1B vorkommt.

Figur 23 zeigt die Sequenz der Oligonukleotide wie sie zur Mutagenese der Spaltstellenregion von HRV2-2A verwendet wurde. Die Kleinbuchstaben geben die ursprüngliche Sequenz der cDNA von HRV2 wieder bzw. die Sequenz des Expressionsplasmids pEx2A/21, einer Restriktionsenzym- modifizierten Variante von pEx2A (siehe Beispiel 2).

Großbuchstaben weisen auf die jeweilige Mutation hin. wt bedeutet Wildtyp. Diejenigen Aminosäuren, die der mutierten Sequenz von HRV2-2A entsprechen, sind angegeben.

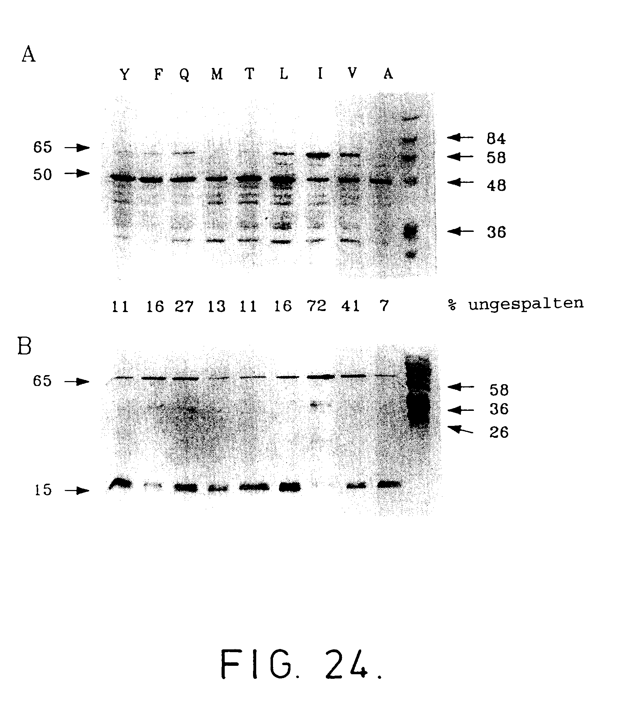

Figur 24 zeigt die Expressionsprodukte der Mutagenese der $P_1$ Stelle der Spaltstelle von HRV2-2A (d. h. die letzte Aminosäure von VP1). A (Ala) ist im Wildtyp pEx2A/21 anzutreffen: die Substitutionen Ala gegen Tyr, Phe, Gln, Met, Leu, Ile und Val sind im Beispiel 6 beschrieben, der Austausch Ala gegen Thr ist im Beispiel 8 beschrieben.

Bild A:    Coomassie Blau gefärbtes 10% SDS-Polyacrylamidgel.

65kD    unprozessiertes Expressionsprodukt der Mutanten

50kD    prozessiertes Expressionsprodukt der Mutanten; entspricht dem Fusionsanteil (98 Aminosäuren der MS2-Polymerase), dem C-terminalen Ende von VP3 und dem gesamten VP1

15kD    HRV2-2A

Der Prozentsatz von ungespaltenem Produkt wurde durch Densitometrie festgestellt und ist zwischen Bild A und B angegeben.

Bild B: Western Blot eines 15% Polyacrylamidgels inkubiert mit dem anti-PC20 Antikörper.

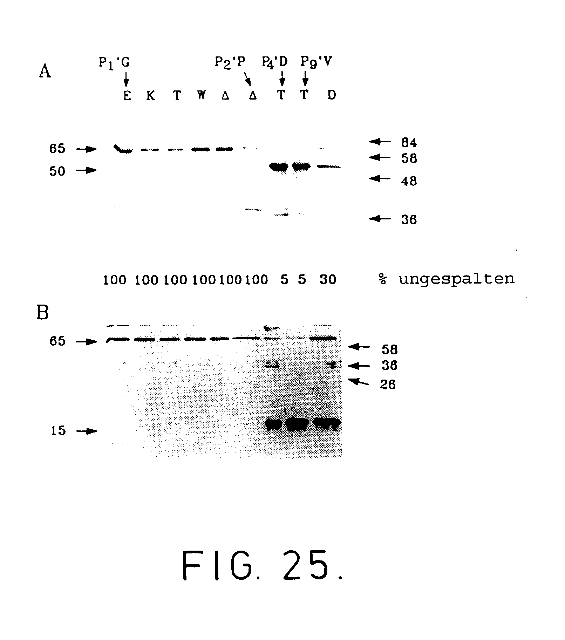

Figur 25 zeigt die Expressionsprodukte der Mutagenese der $P_1'$, $P_2'$, $P_4'$ und $P_9'$ Stellen der Spaltstelle von HRV2-2A (d.h. den N-Terminus von 2A selbst). Austausch von Gly gegen Glu, Lys, Thr und Trp in der $P_1$ Stelle, von Asp gegen Thr in der $P_4'$ Stelle und von Val gegen Asp und Thr in der $P_9'$ Stelle. Deletionen von Gly in der $P_1'$ und Pro in der $P_2'$ Stelle.

Bild A: Coomassie Blau gefärbtes 10% SDS-Polyacrylamidgel.

65kD unprozessiertes Expressionsprodukt der Mutanten

50kD prozessiertes Expressionsprodukt der Mutanten; entspricht dem Fusionsanteil (98 Aminosäuren der MS2-Polymerase), dem C-terminalen Ende von VP3 und dem gesamten VP1

15kD HRV2-2A

Der Prozentsatz von ungespaltenem Produkt wurde durch Densitometrie festgestellt.

Bild B: Western Blot eines 15% Polyacrylamidgels inkubiert mit dem anti-PC20 Antikörper.

Figur 26 zeigt die Expressionsprodukte der Mutagenese der P' bzw. der P und P' Regionen von HRV2-2A; Austausch der P' Region von HRV2 gegen die P'Region von Poliovirus; gegen die P' Region von Poliovirus mit einem Austausch an der $P_4'$ Stelle (His--> Met) und mit einem Austausch an der $P_6'$ Stelle (Asn--> Tyr); gegen die P' Region von HRV14 mit einem Austausch an der $P_2'$ Stelle (Leu--> Pro), sowie der Austausch der P und P' Regionen gegen die von HRV1B.

Tabelle A

| Sequenz der Mutationen verglichen mit der HRV2-2A Spaltstelle. |
|---|
| Spur 1: pEx2A/21 mit der $P_1'_{-7}'$ Region von Poliovirus Serotyp 1 |
| Spur 2: pEx2A/21 mit der $P_1'_{-7}'$ ($P_4'$ His-->Met, $P_6'$ Asn-->Tyr) Region von Poliovirus Serotyp 1 |
| Spur 3: pEx2A/21 mit der $P_1'_{-7}'$ Region ($P_2'$ Leu-->Pro) von HRV14 |
| Spur 4: pEx2A/21 mit der $P_{1-7}/P_1'_{-8}'$ Region von HRV 1B |

Die unterstrichenen Aminosäuren in den Varianten 2 und 3 weisen daraufhin, daß es sich dabei um Unterschiede zu der Wildtyp Poliovirus Serotyp 1 bzw. zu der Wildtypsequenz von HRV 14 handelt. Die unterstrichene Aminosäure in Variante 4 zeigt einen Unterschied an dieser Position gegenüber der Sequenz von HRV2. Die Reduktion der Spalteffizienz (in Prozent) die durch Densitometrie ermittelt wurde, ist jeweils angegeben.

Bild B: Coomassie Blau gefärbtes 10% SDS-Polyacrylamidgel.

Der Prozentsatz von ungespaltenem Produkt wurde durch Densitometrie festgestellt.

Bild C: Western Blot eines 15% Polyacrylamidgels inkubiert mit dem anti-PC20 Antikörper.

65kD unprozessiertes Expressionsprodukt der Mutanten

50kD prozessiertes Expressionsprodukt der Mutanten; entspricht dem Fusionsanteil (98 Aminosäuren der MS2-Polymerase), dem C-terminalen Ende von VP3 und dem gesamten VP1

15kD HRV2-2A

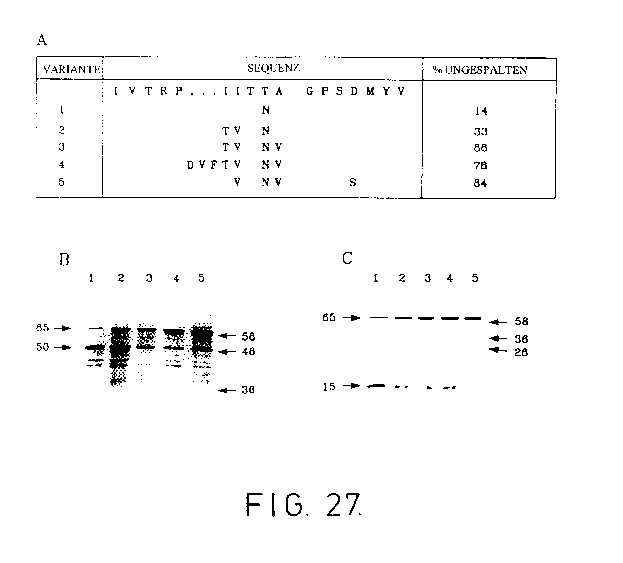

Figur 27 zeigt die Expressionsprodukte der Mutagenese der P Region von HRV2-2A; Austausch gegen jene Aminosäuren, die in der P Region von HRV89 vorkommen. Die genauen Änderungen sind in Figur 22 zu sehen.

Tabelle A

| Sequenz der Mutationen verglichen mit der HRV2-2A Spaltstelle. |
|---|
| Spur 1: pEx2A/21 mit dem Austausch an der $P_2$ Stelle Thr-->Asn |
| Spur 2: pEx2A/21 mit der $P_{2-5}$ Region von HRV89 |
| Spur 3: pEx2A/21 mit der $P_{1-5}$ Region von HRV89 |
| Spur 4: pEx2A/21 mit der $P_{1-8}$ Region von HRV89 |
| Spur 5: pEx2A/21 mit der $P_{1-4}/P_1'_{-4}'$ Region von HRV89 (siehe Beispiel 6) |

Die Reduktion der Spalteffizienz (in Prozent) die durch Densitometrie ermittelt wurde, ist jeweils angegeben.

Bild B: Coomassie Blau gefärbtes 10% SDS-Polyacrylamidgel.

Die Reduktion der Spalteffizienz wurde durch Densitometrie bestimmt und ist (in Prozent) angegeben.

Bild C:     Western Blot eines 15% Polyacrylamidgels inkubiert mit dem anti-PC20 Antikörper.

65kD     unprozessiertes Expressionsprodukt von den Mutanten

50kD     prozessiertes Expressionsprodukt von den Mutanten; entspricht dem Fusionsanteil (98 Aminosäuren der MS2-Polymerase), dem C-terminalen Ende von VP3 und dem gesamten VP1

15kD     HRV2-2A

Figur 28 zeigt die Aminosäureunterschiede zwischen HRV2-2A und HRV89-2A. Die kleinen Buchstaben stellen die Aminosäuresequenz der letzten 8 Reste von VP1 von HRV2 und die Gesamtsequenz der HRV2-2A dar. Die Aminosäuren, die in HRV89-2A unterschiedlich sind, sind unterhalb der HRV2 Sequenz in Großbuchstaben angegeben. Die Spaltstelle ist mit einem Pfeil gekennzeichnet.

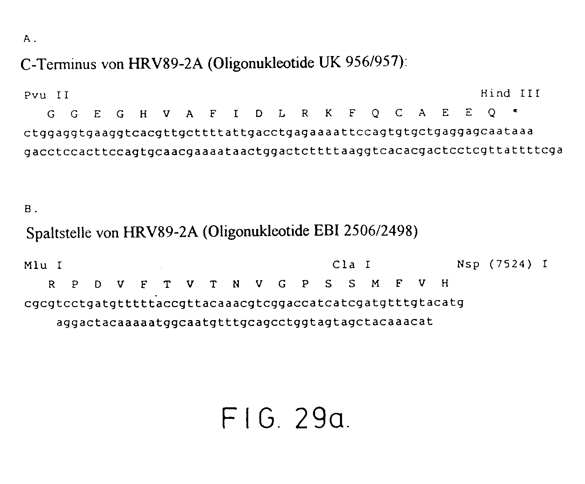

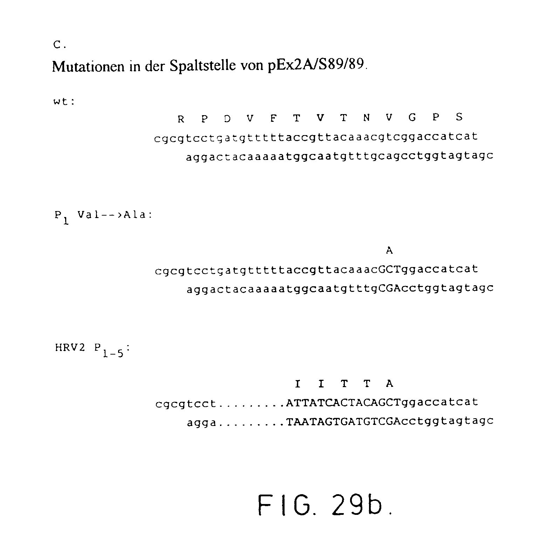

Figur 29 zeigt die Sequenzen der Oligonukleotide, die im Beispiel 9 verwendet wurden.

A. Pvu II/Hind III Oligonukleotidkassette zur Substitution der 21 Aminosäuren am C-Terminus von pEx2A/P$_{1-4}$/P$_1$'$_{-4}$'/89 durch diejenigen von 2A des HRV Serotyp 89. Nach der letzten Aminosäure von 2A wurde ein Stopkodon eingefügt (mit Stern gekennzeichnet).

B. Mlu I/Nsp (7524) I Oligonukleotidkassette zur Einführung der 0,342kb Nsp (7524) I/Pvu II DNA Fragment in das mit Mlu I und Pvu II geschnittene pEx2A/S89''/C89 Plasmid.

C. Oligonukleotide, wie sie zur Mutagenese der 2A Spaltstelle im Plasmid pEx2A/S89/89 verwendet wurden. Die Kleinbuchstaben geben die ursprüngliche Sequenz der cDNA von HRV89 wieder bzw. die Sequenz der Restriktionsvariante pEx2A/S89/89.

Großbuchstaben weisen auf die jeweilige Mutation hin, die Punkte auf die Deletion einer Aminosäure. wt bedeutet Wildtyp.

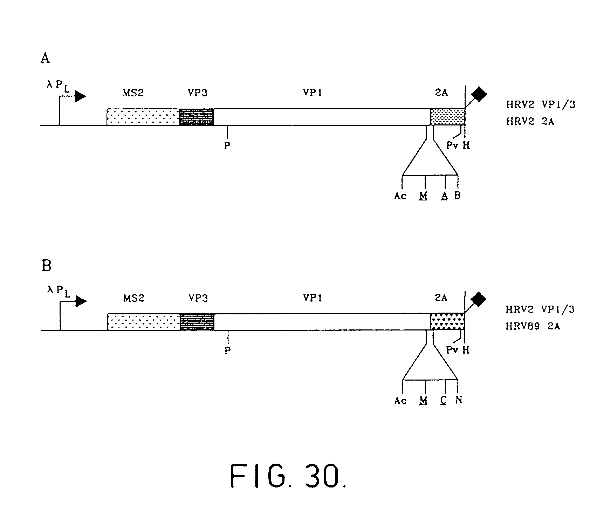

Figur 30 zeigt die Restriktionschnittstellen, die verwendet wurden, um das Plasmid pEx2A/S89/89 zu konstruieren. Abkürzungen der Restriktionenzyme: A,Ava I; Ac, Acc I; B, Bst EII; C, Cla I; Hind III; Mlu I; N, Nsp (7524) I; P, Pst II; Pv, Pvu II.

Figur 31 zeigt die Expressionsprodukte der Plasmide pEx2A/S89/89 und pEx2A/S89''/C89 bzw. von zwei Spaltstellenmutanten von Plasmid pEx2A/S89/89 (pEx2A/S2/89 und pEx2A/S89'/89).

Tabelle A: Sequenz der Mutationen verglichen mit der HRV2-2A Spaltstelle. Die jeweilige Spaltstelle und das Enzym in den Konstruktionen ist angegeben. 89'und 89'' weisen darauf hin, daß die Spaltstellen in diesen Konstruktionen Mutationen gegenüber dem HRV89 Wildtyp aufweisen.

Spur 1: pEx2A/21.

Spur 2: pEx2A/S2/89 mit der Spaltstellensequenz von HRV2 und der 2A Sequenz von HRV89.

Spur 3: pEx2A/S89'/89 mit dem Austausch an der P1 Stelle Val-->Ala.

Spur 4: pEx2A/S89/89 mit der Spaltstellensequenz von HRV89 und der 2A Sequenz von HRV89.

Spur 5: pEx2A/S89''/C89.

Bild B: Coomassie Blau gefärbtes 10% SDS-Polyacrylamidgel.
Bild C: Western Blot eines 10% Polyacrylamidgels inkubiert mit dem anti-MS2-Pol Antikörper.
Bild D: Western Blot eines 15% Polyacrylamidgels inkubiert mit dem anti-PC20 Antikörper.
Bild E: Western Blot eines 10% Polyacrylamidgels inkubiert mit dem anti-MS2-Pol Antikörper.

| EMPIGEN | 50% | 5% | 0,5% | 0,05% | 0,005% |
|---|---|---|---|---|---|
| (Alkyl-dimethyl-ammonium-betain) | 100% | 100% | 100% | 45% | 35% |
| NP40 | 50% | 5% | 0,5% | 0,05% | 0,005% |
| (Octyl-phenol-ethylenoxid) | 100% | 50% | 45% | 45% | 45% |
| Tween 20 | / | 5% | 0,5% | 0,05% | 0,005% |
| (Polyoxyethlensorbitan-Monolaurat) | / | 55% | 45% | 40% | 30% |
| Glycerin | 40% | 4% | 0,4% | 0,04% | 0,004% |
| | 80% | 35% | 35% | 35% | 35% |
| Ammoniumsulfat | 1,9 M | 0,4 M | 40 mM | 4 mM | 0,4 mM |
| | 10% | 0% | 0% | 0% | 0% |
| Guanidinium-Hydrochlorid | 3,5 M | 0,7 M | 70 mM | 7 mM | 0,7 mM |
| | 100% | 90% | 50% | 0% | 0% |
| Harnstoff | 3,5 M | 0,7 M | 70 mM | 7 mM | 0,7 mM |
| | 100% | 20% | 5% | 0% | 0% |
| DMSO | 10% | 1% | 0,1% | 0,01% | 0,001% |
| (Dimetylsulfoxid) | 25% | 10% | >5% | >5% | >5% |
| NaCl | 2,5 M | 0,5 M | 50 mM | 5 mM | 0,5 mM |
| | 0% | 0% | 0-5% | 5% | 5-20% |

Tabelle 1

Der Einfluß verschiedener Denaturierungs- und Solubilisierungsmittel
auf die "trans"-Aktivität von HRV2-2A im "trans cleavage assay"
unter Verwendung von Peptid K (siehe Text) als Substrat. Das obere
Kästchen einer Zeile gibt die Konzentration der jeweiligen Substanz
an (in Molarität oder v/v-Prozent). Der gesandete untere Teil zeigt
den jeweils ermittelten Grad der Inhibierung an bezogen auf das
Verhältnis der Peakflächen von Peptid-Substrat zu Peptid-
Spaltprodukten (methodische Details siehe Text).
0% bedeutet, daß kein ungespaltenes Peptidsubstrat mehr nachweisbar
ist.

18

Beispiel 1:

Herstellung eines aktiven und inaktiven P2A-Enzymsubstrates von HRV2 zur Expression in E. coli

Ausgehend von pUC9 und den cDNA-Klonen von HRV2 (Fig. 11) wurde ein Expressionssystem für P2A konstruiert. Zunächst wurden ca. 10 μg pUC9 durch Doppelverdau mit BamHI und PstI in der Polylinkerregion geöffnet. Die linearisierte Form von pUC9 wurde von Spuren des ungeschnittenen Plasmids mit Hilfe von Whatman DE81- Papier getrennt (Dretzen, G.M., Bellard, P., Sassone-Corsi und Chambon, P.; 1981, Anal. Biochem. 112, 295 - 298). Dazu wurde nach Auftrennung der DNA-Fragmente am Agarosegel vor und hinter die zu isolierende DNA-Bande ein Schlitz geschnitten, in den jeweils ein Streifen DE81-Papier gesteckt wurde. Die Elektrophorese wurde weitergeführt (Gel soll nicht mit Puffer bedeckt sein), bis das gewünschte DNA-Fragment vollständig an den vorderen DE81-Streifen gebunden worden war. Der hintere DE81-Streifen verhinderte eine Verunreinigung durch größere DNA-Fragmente. Das DE81-Papier mit dem gebundenen DNA-Fragment wurde in ein 1,5 ml Eppendorfröhrchen (mit einem Ausflußloch im Gefäßboden und darübergelagerter Polyallomerwolle) transferiert und zweimal 5 min mit je 400 μl Waschpuffer (0,2M NaCl, 25mM TrisHCl pH = 8,0, 1mM EDTA) gewaschen, wobei durch kurze Zentrifugation (ca. 1 sek) die Waschlösung im zweiten darunter befindlichen Eppendorfgefäß aufgefangen wurde. Anschließend wurde die gebundene DNA zweimal durch 15 Minuten dauernde Inkubationen in je 200 μl Elutionspuffer (1M NaCl, 25mM TrisHCl pH = 7,5, 1mM EDTA) vom DE81-Papier gewaschen. Die 400 μl Eluat wurden 10 min in der Eppendorfzentrifuge (15000g) zentrifugiert, um Papierstückchen zu entfernen. Der Überstand wurde vorsichtig in ein neues Eppendorfgefäß transferiert, mit 800 μl 96% Ethanol versetzt, bei -20°C ausgefällt (ca. 2 Stunden), zweimal mit 70% Ethanol gewaschen und getrocknet. Parallel dazu wurde das Plasmid von Klon 719 mit BglII und PstI und das Plasmid von Klon 107 mit PstI verdaut (Fig. 11). Diese beiden Plasmide sind pBR322-Vektoren und enthalten HRV2-cDNA-Fragmente, welche über homopolymere G-C-Regionen in pBR322 insertiert worden waren. Das BglII/PstI-Fragment von Klon 719 repräsentiert die HRV2-cDNA Region von 2145 - 2421 (siehe Fig. 11); das PstI/PstI-Fragment von Klon 107 deckt die anschließende Region 2421-3100 ab. Diese beiden Fragmente wurden in die BamHI und PstI Stelle von pUC9 insertiert, wobei beide Schnittstellen (BglII und BamHI) zerstört wurden. Diese Konstruktion wurde p18 genannt (siehe Fig. 11).

Um für die Transformation kompetente Zellen zu erhalten, wurde eine modifizierte Vorschrift von Mandel und Higa (Mandel, M. und Higa, A., 1970, J. Mol. Biol. 53, 159-162) angewandt. Dazu wurden 0,5 ml einer "über Nachtkultur" von E. coli Stamm HB 101 in 50 ml LB-Medium (10 g/l Trypton, 5 g/l Hefeextrakt, 10 g/l NaCl) überimpft, bis zu einer OD600 von ca. 0,4 hochgezüchtet und anschließend 5 min bei 5 k und 4°C pelletiert. Die Bakterien wurden dann in 25 ml 0,1M MgCl$_2$ (eiskalt) vorsichtig resuspendiert, 5 min auf Eis gestellt und abermals 5 min bei 5 k und 4°C zentrifugiert. Das Pellet wurde in 25 ml 0,1M CaCl$_2$ - (eiskalt) resuspendiert, 4 Stunden auf Eis gestellt und 5 min bei 5 k und 4°C zentrifugiert. Die Zellen wurden in 2,5 ml 1x Lagerpuffer (0,1M CaCl$_2$/Glycerin = 4/1% v/v) aufgenommen, 20 min auf Eis gestellt, in 100 μl Portionen aliquotiert und in flüssigem Stickstoff schockgefroren, sowie bei -80°C gelagert. Zu 100 μl auf Eis aufgetauter kompetenter Zellsuspension wurden 5 μl des oben beschriebenen Ligationsansatzes zugegeben, die Zellen 1 Stunde auf Eis und 2 min bei 42°C inkubiert und abschließend 5 min auf Eis gestellt. Vor dem Ausplattieren der Zellen wurden 900 μl LB-Medium zugesetzt, 10 min bei 37°C inkubiert und je 200 μl Zellsuspension auf LB-Agar-Platten (1,5%iger Agar in LB-Medium mit 100 mg/l Ampicillin) aufgebracht und über Nacht inkubiert. Mit dem Plasmid p18 wurden wie oben angeführt kompetente JM 101 Zellen transformiert. Einige der erhaltenen Amp$^r$ Klone wurden einer Restriktionsanalyse unterworfen und von einem der positiven Klone (18/1) wurde Plasmid-DNA im Großmaßstab gewonnen (T. Maniatis et al. 1982, Molecular Cloning: A Laboratory Manual; Cold Spring Harbor, 86ff). Das Plasmid wurde anschließend über eine Sephacryl-S-1000 Säule gereinigt (Durchmesser 0,9cm, Länge 20cm). Als Elutionspuffer wurde ein TE-Puffer verwendet. Das Eluat wurde in ca. 0,5 ml Fraktionen aufgetrennt und die einzelnen Fraktionen bei 260 nm ausgemessen (üblicherweise erscheint der Plasmidpeak zwischen der 9. und 14. Fraktion). Der Beginn des RNA-Peaks ist ab der 17. Fraktion zu erwarten (OD über 3,0). Die in Frage kommenden Fraktionen wurden vereinigt, lyophilisiert, das Plasmid in 0,5 ml TE-Puffer aufgenommen, 5 min bei 65°C inkubiert, mit Phenol/Chloroform und Chloroform extrahiert, ausgefällt, zentrifugiert, getrocknet und in 100 μl TE-Puffer gelöst. Etwa 10 μg von Plasmid 18/1 wurden mit AccI und HindIII (die AccI Stelle stammt von der HRV2-cDNA, die HindIII Stelle von der Polylinkeregion von pUC9) geschnitten. Parallel dazu wurde der Klon 521 mit AccI und HindIII verdaut. Das AccI/HindIII-Fragment von Klon 521 umfaßt die HRV2-cDNA von Nukleotidnummer 3075-3698 (Fig. 11). Das AccI/HindIII-Fragment von 521 wurde in 18/1 (AccI/HindIII) ligiert und damit kompetente JM 101, wie oben beschrieben transformiert. Die erhaltenen Kolonien wurden durch Restriktionsanalyse überprüft (EcoRI, PstI, AccI und HindIII) und die Plasmid DNA einiger Klone wurde sequenziert. Ein Klon, der die HRV2-Sequenz von 2145 - 3698 und den richtigen Leserahmen aufwies, wurde 18521 benannt und zur Expression ausgewählt. In der Konstruktion 18521 sind die homopolymeren G-C-Regionen, die noch von der Klonierung in pBR322 herstammen 5'seitig von der HindIII Stelle vorhanden. p18521 wurde mit EcoRI und HindIII geschnitten, das Fragment mit DE81 Papier isoliert und in pEx34c (EcoRI/HindIII) durch Ligation insertiert. pEx34 ist ein 3,0 kb Expressionsvektor (ein Derivat von pPLc24; E. Remault, P. Stanssens und W. Fiers; 1981, Gene 15, 81-93) welcher folgende Abschnitte enthält:

- die prokaryontische ribosomale Bindungsstelle
- einen Teil der kodierenden Region für die ersten 98 N-terminalen Aminosäuren der MS2-Polymerase; dieser Fusionsanteil weist hydrophobe und basische Aminosäuren auf und bewirkt die Herabsetzung der Löslichkeit des Fusionsproteins und erhöht die Stabilität des exprimierten Produktes in der Zelle.
- das Fusionsprotein steht unter der Kontrolle des linken lambda-Promotors
- eine kleine Polylinkerregion in 3 verschiedenen Leserahmen (pExa,b und c) mit Schnittstellen für EcoRI, BamHI und HindIII ermöglicht das Einsetzen von geeigneten DNA-Fragmenten in Phase hinter dem Fusionsproteinanteil
- die "ori"- und die Ampicillin-resistenz-region von pBR322.

E. coli W6(lambda) exprimiert konstitutiv das Gen für den Wildtyp lambda-Repressor und eignet sich zum Hochzüchten der pEx-Plasmide. E. coli 537 dagegen trägt die cI 853-lambda- Repressor-Mutation (inaktiv bei 42°C) auf einem weiteren Plasmid, das auch ein Kanamycin Resistenzgen trägt (K. Strebel, E. Beck, K. Strohmaier und H. Schaller; 1986, J. Virol. 57, 983-991).

Durch Insertion des EcoRI/HindIII Fragmentes von p18521 in pEx34c konnte ein Expressionssystem erhalten werden, welches die Region: (VP3)-VP1-P2A-(P2B) von HRV2 (2145-3698; siehe Fig. 12) umfaßt. Dieses Expressionssystem produziert ein als Substrat dienendes virales Polypeptid, das gleichzeitig auch P2A-Protease-Aktivität aufweist.

Um nun ein inaktives Enzymsubstrat für P2A zu erhalten wurde der Expressionsvektor pEx34c x 18521 in E. coli W6(lambda) hochgezüchtet. Wie oben beschrieben wurde der Vektor nach der Großmaßstabpräparationstechnik aus 500 ml einer Übernachtkultur isoliert. 2 µg von pEx34c x 18521 wurden mit HindIII verdaut und mit Hilfe von DE81 wie oben beschrieben gereinigt. Anschließend wurde wie folgt der linearisierte Vektor mit Ba131 - Nuklease verdaut: ca. 1 µg des mit HindIII linearisierten Vektors wurden mit 1U Bal31-Nuklease (Biolabs) in 20 mM TrisHCl pH = 8, 600 mM NaCl, 12 mM MgC12 und 1 mM EDTA inkubiert. Aliquote Proben wurden nach 1-2-3-4-5-6 und 8 Minuten entnommen und durch Zugabe von EDTA (Endkonzentration 30 mM) wurde der Verdau gestoppt. Die DNA wurde durch Ethanolpräzipitation wiedergewonnen und 100 ng des Plasmids wurden mit 100 U T4-Ligase in 10 mM TrisHCl pH = 7,5, 6 mM MgC12, 6 mM BME und 1 mM ATP über Nacht bei 15°C inkubiert. Der T4-DNA-Ligaseansatz wurde direkt zur Transformation von E. coli W6(lambda) verwendet. Einige der Klone wurden gepickt und die jeweilige Plasmid DNA wurde nach Maxam und Gilbert sequenziert (Maxam, A. und Gilbert, W., 1980, Nucleic Acids Res. 65, 499 - 560). Ein Klon dessen cDNA mit der HRV2-Nukleotidnummer 3321 (siehe Skern, T. et al., 1985, Nucleic Acids Res. 13, 2111 - 2126) endet, wurde 18731 benannt. Diese Deletionsmutante von 18521 wurde als inaktives P2A-Enzymsubstrat zu Expressionsstudien herangezogen.

Beispiel 2:

Expression und Nachweis der Fusionsproteine

Das Plasmid pEx34c x 18521 und Plasmide von Klonen des Bal31-Verdaus von pEx34c x 18521 wurden in E. coli Zellen transferiert. Die Zellen wurden über Nacht bei 28°C in LB-Medium (+100 mg Ampicillin/1 und 25 mg Kanamycin/1) hochgezüchtet. Die Kulturen wurden anschließend 1:5 mit vorgewärmten (42°C) LB-Medium ohne Antibiotika verdünnt (Induktion des lambda-PL-Promotors) und für 2 Stunden unter heftigem Schütteln bei 42°C inkubiert. Nach 2 Stunden wurden die Zellen von 1 ml der Kultur geerntet (2 min in der Eppendorfzentrifuge, 4°C) und in 500 µl kalten Sonikierungspuffer (150 mM NaCl, 50 mM TrisHCl pH = 8 und 1 mM EDTA) resuspendiert. Das Aufbrechen der Zellen wurde mit Hilfe eines M. S. E. Ultrasonic Power Gerätes vorgenommen (3 mal je 5 sek; 1,7 Amp.), wobei zwischen den einzelnen Sonikierungen eine Pause von 45 sek eingeschaltet wurde, um ein Überhitzen der Proben zu vermeiden. Unlösliches Material wurde anschließend durch 2 min Zentrifugation in der Eppendorfzentrifuge gewonnen und die Pellets in 200 µl Sample buffer (4% SDS, 125 mM TrisHCl pH = 6,8, 10% BME, 10% Glycerin und 0,02% Bromphenolblau) gelöst. Nach Erhitzen auf 95°C für 4 min, wurden je 10 µl der Proben auf einem 10% SDS-PAA-Gel (Lämmli, U. K., 1970, Nature 227, 680-685) aufgetrennt. Kontrollexperimente zeigten, daß alle exprimierten Proteine im Sonikierungspuffer unlöslich sind. Die Gele wurden anschließend mit Coomassie Brilliant Blue wie folgt gefärbt:
- Färbung: 30- 60 min in 50% Methanol, 10% Essigsäure und 0,1% Coomassie Brilliant Blue
- Entfärbung: über Nacht in 5% Methanol und 10% Essigsäure
- Glycerinbad: 30 min in 7% Essigsäure und 2% Glycerin
- Ethanolbad: 1 bis 2 min in 96% Ethanol
- Trocknen: auf 3MM Papier; 2-3 Stunden bei 80°C auf Geltrockner (Hoefer, SE 1160)

In Fig. 13 ist ein typisches Bild einer Coomassie Brilliant Blue Anfärbung einer Expression von pEx34c x 18521 in 537 zu sehen. Ebenfalls aufgetrennt wurde die Deletionsmutante 18731 (siehe Fig. 13). pEx34c x 18731 endet bei Nukleotidnummer 3321 und besitzt daher das wahrscheinlich aktive Zentrum von P2A nicht mehr.

Um die antigenische Spezifität dieser exprimierten Formen von 18521 und 18731 aufzuzeigen wurde ein Western blot mit Hilfe eines polyklonalen Serums gegen VP1 durchgeführt (ATCC-VR-1112 AS/GP; VP1 ist integraler Bestandteil beider Expressionsplasmide pEx34c x 18521 bzw. 18731). Der Western blot wurde wie folgt durchgeführt:

Für den Elektrotransfer der aufgetrennten Proteine vom Gel auf die Nitrozellulose wurden 4 Lagen 3MM Papier (Whatman) und 1 Lage Nitrozellulose (Schleicher und Schuell, BA85, 0,45 um) genau den Abmessungen des Trenngels entsprechend zurechtgeschnitten und in Transferpuffer präinkubiert:

20 mM Tris-Base

150 mM Glycin

20% Methanol p.a.

(pH = 8,8; braucht nicht mehr titriert zu werden).

Die Zusammenstellung des "Transfer-Sandwich" erfolgte nach dem unten angeführten Schema (Luftblasen vermeiden!):

- Pol --> scotch brite --> 2 Lagen 3 MM --> Gel --> Nitrozellulose --> 2 Lagen 3 MM --> scotch brite --> + Pol

Das Proteingel wurde ebenfalls vor dem Zusammenbau 2 min in Transferpuffer äquilibriert. Der Transferpuffer kann auch als 10 x Lösung (24,2g Tris und 112,6 g Glycin pro Liter ohne Methanol) hergestellt werden. Der Transfer wurde im Transferpuffer bei ca. 1 Ampere, 2 Stunden in einer Protein-Blot-Apparatur in Anwesenheit von 0,1% Empigen BB (Alkyldimethylammoniumbetain; Nr. 62 852, Marchon France S.A.) durchgeführt (R.E. Mandrell et al.; J. Immunol. Meth. 67, S. 1 (1984)). Die Effizienz des Transfers wurde an Hand der vorgefärbten Markerproteine überprüft.

Die Filter mit den daraufgebundenen Proteinen wurden über Nacht bei Raumtemperatur in 50 ml "Blocking Solution", das ist PBS:

137,O mM NaCl

2,7 mM KCl

8,0 mM $Na_2HPO_4$

1,5 mM $KH_2PO_4$

0,5 mM $MgCl_2.6H_2O$

1,0 mM $CaCl_2.2H_2O$

mit 1% BSA, 1% Tween 20 (Polyoxyethylen(20)-sorbitanmonolaurat) und 10% Hitze-inaktiviertem fötalem Kälberserum (HIFKS), gebadet.

Das polyklonale Antiserum gegen VP1 (ATCC VR-1112 AS/GP) wurde vor Verwendung mit einem E. coli - Lysat präinkubiert, um E. coli - spezifische Antikörper zu entfernen. Dazu mischte man gleiche Volumina Antiserum und E. coli-Zellysat, inkubierte 2 Stunden bei Raumtemperatur und über Nacht bei 4°C. Die kreuzreagierenden E. coli Proteine wurden als Immunopräzipitat durch Zentrifugation (5 min, Eppendorfzentrifuge) vom Überstand getrennt. Das Nitrozellulosefilter wurde anschließend 3 Stunden in "Blocking Solution" mit dem polyklonalen Antikörper (präinkubiertes polyklonales Antiserum 1/500 bis 1/1000 in "Blocking Solution" verdünnt) in einer Plexiglasbox auf einer Wippe inkubiert. Danach wurde das Filter unter fließendem Leitungswasser gut gespült (ca. 15 min) und dreimal je 15 min mit 50 ml PBS (+1% Tween 20) gewaschen. Im letzten Schritt wurde das Filter in ca. 50 ml "Blocking Solution" mit den alkalische Phosphatase konjugierten Kaninchen-Anti-Antikörper (1/5000 bis 1/7500 in "Blocking Solution" verdünnt) 3 Stunden bei Raumtemperatur inkubiert. Abschließend wurde das Filter wieder unter fließendem Leitungswasser gut gespült (15 min) und dreimal wie oben mit je 5o ml PBS (+1% Tween 20) gewaschen. Die Anfärbung erfolgte in 10 ml Phosphatasepuffer:

100 mM TrisHCl pH = 9,5

100 mM NaCl

5 mM MgCl$_2$
in Anwesenheit der Farbstoffe Nitro-blue-Tetrazolium (NBT; 165 $\mu$g/ml) und
5-Bromo-4-chloro-3-indolyl-Phosphat (BCIP; 82,5 $\mu$g/ml). Das alkalische Phosphatase konjugierte Anti-Kaninchen-IgG (Fc), sowie die Farbstoffe NBT und BCIP stammen von Promega Biotec (Protoblot TM-System). Die Färbereaktion wurde ebenfalls durch Spülen unter fließendem Leitungswasser nach ca. 1 min abgestoppt. In Fig. 14 ist ein typisches Bild eines Western blots von pEx34c x 18521 bzw. 18731 wiedergegeben.

Beispiel 3:

Etablierung eines Expressionssystems zur Darstellung ursprünglicher Protease 2A, sowie Analyse von Punktmutanten in dem wahrscheinlich aktiven Zentrum von 2A.

Um die Rolle einiger hochkonservierter Aminosäuren in der Region des wahrscheinlich aktiven Zentrums zu untersuchen (siehe Fig. 15), wurde unter Verwendung einer Oligonukleotidkassette die in vitro Mutagenese und die Deletion einzelner Aminosäuren in dieser Region durchgeführt. Parallel dazu wurde diese Methode verwendet, um ursprüngliche Protease 2A zu exprimieren.

Ausgehend von pEx18521 wurde durch Verdau mit Apa I (Nukleotidnummer 3458 der HRV2 cDNA) und Hind III (Restriktionsschnittstelle stammt aus der Polylinkerregion des Vektors; siehe Beispiel 1) nach Vorschrift des Herstellers (Biolabs) ein 264 bp langes DNA-Fragment gewonnen, welches durch zwei doppelsträngige Oligonukleotide WT 12 und WT 34 mit Apa I/Hind III "sticky ends" ersetzt wurde (siehe Fig. 16). Zunächst wurde je 1 $\mu$g der einzelsträngigen Oligonukleotide WT1, WT2, WT3 und WT4 in jeweils 10 $\mu$l (20 mM TrisHCl pH = 7,5, 10 mM MgC12, 20 mM DTT und 1 mM ATP) mit je 2 U T4-Polynukleotidkinase (Biolabs) 30 min bei 37°C kinasiert. Anschließend wurden die Kinaseansätze von WT1 und WT2, bzw. von WT3 und WT4 vereinigt, 10 min bei 68°C, 30 min bei 45°C, 10 min bei Raumtemperatur und anschließend kurz auf Eis inkubiert. Die dermaßen kinasierten und hybridisierten Oligonukleotide wurden vereinigt, mit einer 10 mM ATP Lösung wurde die Konzentration auf 1 mM eingestellt und für die Ligation mit 28 U T4-DNA-Ligase (Boehringer Mannheim) versetzt. Die Ligation selbst wurde zunächst 2 Stunden bei 20°C durchgeführt, dann wurden nochmals 7 U T4-DNA-Ligase zugesetzt und der Ligationsansatz 40 Stunden bei 14°C inkubiert. Anschließend wurde der Ligationsansatz bei 70°C 10 min inkubiert, mit einer 1M NaCl-Lösung auf 100 mM gebracht und mit 50 U Apa I und 20 U Hind III wurden die entstandenen multimeren Formen des Oligonukleotids "nachgeschnitten". Die Reinigung und Isolierung des korrekten, doppelsträngigen Apa I/Hind III Fragmentes, sowie die Rückklonierung dieses Fragmentes in pEx18521 (mit Apa 1 und Hind III geschnitten) und die Identifikation mittels Sequenzierung wurde, wie in Beispiel 1 beschrieben, durchgeführt. Ein positiver Klon wurde ausgewählt und zu Expressionsstudien sowie zu "trans"-Aktivitätstests herangezogen. Man erhielt auf diese Weise die Klone pEx2A, pEx2A Cys 106 --> Ser, - Cys 112 --> Ser bzw. - His 114 --> Gly. Die Induktion der Expression erfolgte genau wie in Beispiel 2 beschrieben und erbrachte das in Fig. 17 und 18 gezeigte Ergebnis.

Beispiel 4:

Produktion des Peptidantikörpers PC20

Die letzten 20 Aminosäuren der Protease 2A stellen eine potentielle antigene Determinante dar. Ein Peptid (PC20), welches genau diese Aminosäuresequenz aufwies, wurde synthetisiert und zur Induktion von Antikörpern in Kaninchen verwendet:
570 $\mu$g dieses Peptids wurden in 0,4 ml PBS Lösung aufgenommen und in einer 5 ml Spritze aufgezogen. In einer zweiten 5 ml Spritze wurden 0,5 ml Freund'sches Adjuvans (CAF; GIBCO) aufgezogen und die beiden Komponenten wurden anschließend mit Hilfe eines Dreiweg-Sperrhahnventils vermischt, bis eine Emulsion gebildet wurde. Das Kaninchen wurde im Ohr arteriell punktiert, um ein Pre-Serum für die Negativkontrolle zu erhalten. Die Immunisierung erfolgte durch subkutane Injektion der Peptid/CFA Mischung an 4 verschiedenen Stellen (0,2 ml/Injektionsstelle) des Rückenbereiches. Nach 5 Wochen wurde mit 1,2 ml Peptidlösung durch Injektion von jeweils 0,2 ml intramuskulär in den Rückenteil "geboostet". Acht Tage später wurde das Blut durch Herzpunktion entnommen. Das Blut ließ man bei Zimmertemperatur gerinnen, Fibrin und geformte Bestandteile wurden mit einem sterilen Stäbchen entfernt und das Blut bei 2000 rpm zentrifugiert. Das Serum wurde aliquotiert und bei -18°C gelagert.

Beispiel 5:

Einführung neuer singulärer Erkennungssequenzen für Restriktionsenzyme in dem für die C-terminale Region von VP1 und die HRV2-2A kodierenden Abschnitt des Expressionsvektors pEx2A.

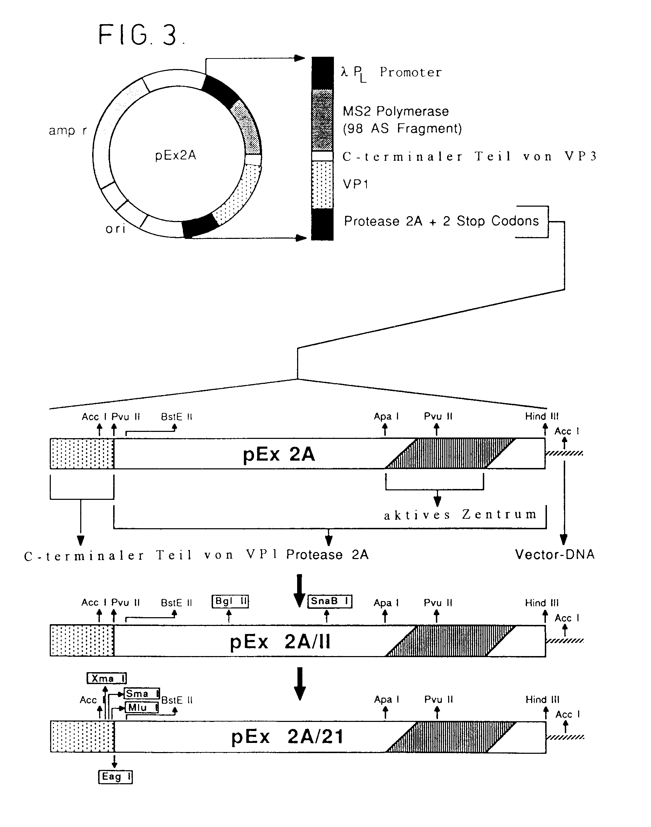

Im Zuge der Analyse der Spaltspezifität und zur Einführung von Deletionen im kodierenden Bereich für HRV2-2A wurde ausgehend vom Expressionsvektor pEx2A (siehe Beispiel 3) ein modifiziertes Plasmid etabliert, das in der kodierenden Region um die Spaltstelle und in dem Bereich für die Protease 2A neue singuläre Erkennungssequenzen für Restriktionsenzyme aufweist. Dieses modifizierte Expressionssystem weist unter Beibehaltung der nativen Aminosäuresequenz eine geänderte Nukleotidsequenz auf, die mit Hilfe von synthetischen doppelsträngigen Oligonukleotiden eingebracht wurde (Fig. 3).

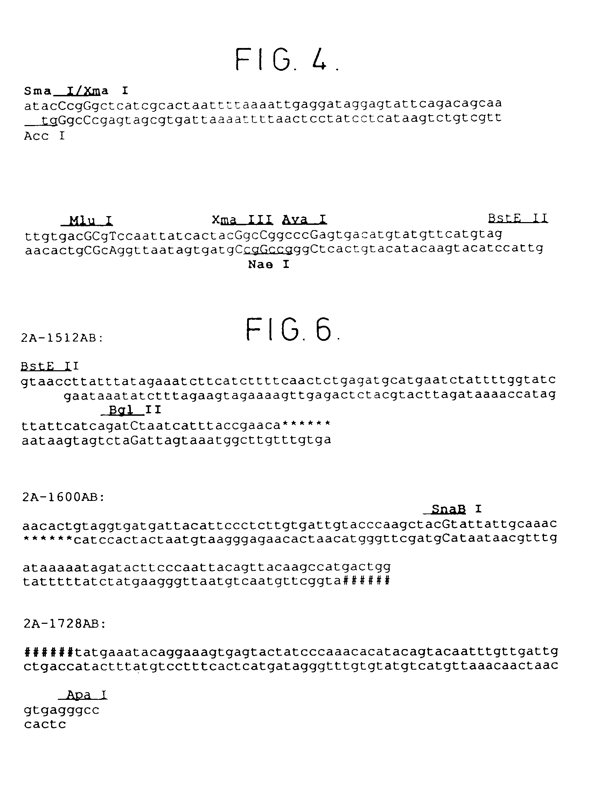

1. Ausgehend vom Expressionsplasmid pEx2A wurde ein AccI Fragment (ca. 700 bp) unter Standardbedingungen isoliert. Dieses Fragment umfaßt den C-Terminus von VP1, die gesamte kodierende Region von HRV2-2A (inklusive der Stoppkodons nach der letzten Aminosäure für die HRV2-2A), sowie Teile der Vektor-DNA (siehe Fig. 11). Das gereinigte AccI-Fragment von pEx2A wurde anschließend mit BstEII nachgeschnitten. Das größere BstEII/AccI-Fragment (ca. 635 bp) wurde vom kleineren AccI/BstEII-Fragment (ca. 115 bp) abgetrennt, gereinigt und unter Standardbedingungen mit einem synthetischen doppelsträngigen Oligonukleotid (ca. 115 bp; siehe Fig. 4), welches dem kleinen AccI/BstEII-Fragment entspricht aber eine modifizierte Nukleinsäuresequenz aufweist, ligiert. Das neu rekombinierte AccI-Fragment wurde mit AccI nachgeschnitten, um multimere Formen zu zerstören, und in den ursprünglichen mit AccI linearisierten pEx2A-Vektor ligiert und in kompetente E. coli W6(1) transformiert. Einige 100 Ampicillin-resistente Klone wurden erhalten und aufgrund von Restriktionsenzymanalysen 3 Klone ausgewählt und sequenziert. Ein Klon pEx2A/21 wurde wie oben beschrieben in E. coli 537 transformiert und exprimiert, wobei das Expressionsprodukt von pEx2A/21 ein identes Verhalten auf einem Western blot zeigt wie das von pEx2A (siehe Fig. 5, Spur 2).

2. Um geeignete neue singuläre Restriktionsschnittstellen in den mittleren für die HRV2-2A kodierenden Bereich von pEx2A zu erhalten, wurde zunächst die Vektor-DNA von pEx2A mit BstEII und ApaI verdaut und vom entstandenen BstEII/ApaI- Fragment (ca. 270 bp) auf Agarosegelen abgetrennt. Sowohl ApaI als auch BstEII sind Erkennungssequenzen für Restriktionsenzyme, welche ursprünglich in der cDNA von HRV2 anzutreffen sind (Positionsnummer 3458 für ApaI und 3188 für BstEII siehe deutsche Patentanmeldung P 35 05 148.5). Parallel dazu wurden 3 doppelsträngige Oligonukleotide (2A-1512AB, 2A-1600AB und 2A-1728AB), die die Region zwischen der ApaI und der BstEII Stelle von HRV2-2A repräsentieren (siehe Fig.64), ligiert, mit ApaI/BstEII nachgeschnitten und in den mit ApaI und BstEII linearisierten pEx2A-Vektor eingebaut. Dieser modifizierte pEx2A-Vektor wurde verwendet, um kompetente E. coli W6-(1) zu transformieren. Von einigen 100 Klonen wurden 4 aufgrund des Restriktionsmusters ausgewählt, sequenziert und damit kompetente E. coli 537 transformiert. Das Expressionsmuster eines Klons (pEx2A/II, siehe Fig. 5, Spur 3) zeigte identes Verhalten wie das von pEx2A (siehe Fig. 5, Spur 1). Wie in Fig. 3 gezeigt, weist das neu eingebrachte BstEII/ApaI-Fragment singuläre Schnittstellen für BglII und SnaBI auf.

Diese beiden neuen Expressionsvektoren pEx2A/21 und pEx2A/II, ermöglichen es unter Ausnützung der neugenerierten Restriktionsstellen Mutationsoligonukleotide einzubauen, welche einerseits zur Feinanalyse der Spaltstelle (siehe Beispiel 6) verwendet wurden, andererseits für Deletionsstudien herangezogen werden können, um die kleinste noch proteolytisch aktive Region von HRV2-2A zu ermitteln.

Beispiel 6:

Mutationsanalyse der P1 Stelle der Protease 2A von HRV2.

Die Mutationen der P1 Position der Spaltstelle der Protease 2A von HRV2 (im folgenden HRV2-2A genannt) wurden entweder im Expressionsvektorsystem pEx18521 oder pEx2A durchgeführt.

1. Ala --> Tyr Mutation an der Spaltstelle von HRV2-2A unter Verwendung des Expressionsvektorsystems pEx18521.

Zunächst wurde das Alanin der P1 Stelle in HRV2 gegen ein Tyrosin ausgetauscht. Diese Mutation führt zu einem Y/G-Aminosäurepaar an der 2A-Spaltstelle, wie sie in Poliovirus vorkommt (Toyoda, H. et al., 1986, Cell, 45, 761 - 770). Ausgehend von dem isolierten 1,3 kb PstI/HindIII Fragment des Expressionsvektors pEx18521 wurde dieses Fragment in einen BLUESCRIPT-Vektor (Stratagene Cloning Systems) eingebaut. Von diesem neu rekombinierten Plasmid wurde mit Hilfe von Standardmethoden bzw. nach der

EP 0 410 147 B1

Vorschrift des Herstellers (Amersham kit: "Oligonucleotide-directed in vitro mutagenesis system") die Einzelstrangform isoliert. Die Punktmutation der Einzelstrangform wurde mit Hilfe des Oligonukleotids EBI 936 (siehe Fig. 1) nach der Vorschrift des Herstellers (Amersham kit: "Oligonucleotide-directed in vitro mutagenesis kit") durchgeführt. Rekombinante Plasmide, die die Mutation enthielten, wurden durch Sequenzierung verifiziert. Das die Mutation (Ala --> Tyr) enthaltende PstI/HindIII Fragment wurde isoliert und dazu verwendet, das Wildtypfragment von pEx18521 zu ersetzen.

2. Mutationen an der Spaltstelle von HRV2-2A unter Verwendung des Expressionsvektors pEx2A.

Ein Derivat des Vektors pEx2A, nämlich pEx2A/21, welches unter anderem eine neu generierte MluI Erkennungssequenz an Position 3137 der HRV2-cDNA enthält (siehe Beispiel 5), wurde dazu verwendet, um mit Hilfe von doppelsträngigen Oligonukleotiden (siehe Fig. 1) weitere Mutationen in und um die Spaltstelle einzuführen, wobei die neu geschaffene Restriktionsstelle MluI und die ursprünglich in der HRV2-cDNA vorhandene BstEII-Stelle verwendet wurden. Dabei repräsentiert z. B. ein doppelsträngiges Oligonukleotid jene Aminosäuren in Position P1-4 und P1'-4' wie sie im Serotyp HRV89 vorgefunden werden (siehe Fig. 1).

Die Präparation der Vektor-DNA, die Integration des doppelsträngigen Oligonukleotids und die DNA-Sequenzierung wurden nach Standardmethoden durchgeführt. Die Expression der mutierten Proteine wurde, wie beschrieben, in E. coli 537 bzw. AR58 vorgenommen. Die Expressionsprodukte wurden auf SDS-Polyacrylamidgelen analysiert (siehe Fig. 2A und 2B). Identische Polyacrylamidgele wurden einer Western-blot Analyse unterworfen, wobei einerseits ein monoklonaler Mausantikörper, der gegen den N-terminalen Teil der MS2-Polymerase gerichtet ist, verwendet wurde (z. B. der anti-MS2-Pol; Hansen, J. et al., Embo Journal, Vol. 7, 1785-1791 (1988)); andererseits wurde ein polyklonales Kaninchenantiserum eingesetzt, das ein Peptid erkennt, welches aus den letzten 20 Aminosäuren der ursprünglichen HRV2-2A besteht (im folgenden anti-PC20 genannt). Konstruktionen, die die Aminosäuren Glutamin, Leucin, Methionin, Phenylalanin und Tyrosin an der P1 Position aufweisen, wurden ebenso effizient gespalten wie der Wildtyp, was aus dem Fehlen der Banden des ungespaltenen Materials bei 65Kd (im Fall der Tyrosinmutation bei 75Kd) hervorgeht. Die Mutanten, welche Valin oder Isoleucin an der P1 Stelle aufweisen, wurden jedoch nicht vollständig prozessiert. Bei Verwendung der Valin enthaltenden Konstruktion wurde 25% des induzierten Proteins nicht prozessiert; befindet sich ein Isoleucinrest an dieser Stelle, so wurde sogar 50% des induzierten Materials nicht gespalten. Eine deutliche Reduktion der Spalteffizienz findet man auch bei Verwendung derjenigen Aminosäuren in Position P1-4 und P1'-4' wie sie im Serotyp HRV89 vorgefunden werden (ca. 65-70% des induzierten Proteins wird nicht prozessiert).

Zusammenfassend ist zu sagen, daß die Aminosäuren Isoleucin und Valin offensichtlich nicht sehr gut in das aktive Zentrum der HRV2-2A passen; dies ist umso bemerkenswerter, als Valin jene Aminosäure ist, welche in der P1 Stelle von HRV89 anzutreffen ist. Möglicherweise stört die Methylgruppe des $\beta$-C-Atoms die Topographie des aktiven Zentrums bzw. eine zur proteolytischen Spaltung notwendige strukturelle Veränderung des Substrates. Es ist daher anzunehmen, daß Unterschiede in den Strukturen der aktiven Zentren der 2A zwischen den Serotypen HRV2 und HRV89 bestehen.

Beispiel 7:

Der Einfluß unterschiedlicher Peptidsubstrate und verschiedener Inkubationsbedingungen auf die Effizienz der "trans"-Aktivität von HRV2-2A in vitro.

Die in diesem Beispiel verwendeten Peptide wurden zum Teil von der Firma "Multiple Peptide Systems" (San Diego; California, USA) erworben oder nach der "solid phase" Methode (Merrifield, R.B. 1963, J. Am. Chem. Soc., 85, 2149 - 2154) synthetisiert. Die Peptide wurden anschließend über "reverse phase" HPLC gereinigt (0,1% Trifluoressigsäure und Acetonitril als mobile Phase) und mit Hilfe von HPLC Analyse, "fast atom bombardment mass spectrometry (FAB-MS) und Aminosäuresequenzierung (Hunkapiller, M.W. and Hood, L.E., 1983, Science, 219, 650 - 659) identifiziert. Wenn nicht anders angegeben weisen alle verwendeten Peptide aus Stabilitätsgründen einen mit einer Acetylgruppe blockierten N-Terminus auf, der C-Terminus liegt immer als Säureamid vor.

Die Induktion der 2A Expressionssysteme im E. coli Stamm 537 erfolgte wie in Beispiel 2 beschrieben. Nach 2 Stunden Inkubation bei 42°C wurden die Zellen von 1 ml der Kultur geerntet (30 sek in der Eppendorfzentrifuge` 4°C) und in 500 ul "trans"-Aktivitätspuffer (50 mM Tris HCl pH = 8,0, 2,5 M NaCl und 1 mM EDTA) resuspendiert. Das Aufbrechen der Zellen wurde mit Hilfe eines M.S.E. Ultrasonic Power Gerätes vorgenommen (30 sek; im Eiswasserbad).

Unlösliches Material wurde durch Zentrifugation bei 100 000 g (4°C, 30 min in der Ultrazentrifuge;

24

Rotortype Ti 50, Beckmann) entfernt und 100 ul dieses Überstandes wurden jeweils mit 5 ul einer wässrigen Peptidlösung (4 mg/ml) versetzt und 30 min bei 37°C inkubiert. Die Reaktion wurde durch Zugabe des gleichen Volumens an 1 M $HClO_4$ abgestoppt. Die Proben wurden anschließend 20 min auf Eis inkubiert, 10 min bei 4°C zentrifugiert (Eppendorfzentrifuge), mit gleichem Volumen an 1,4 M $K_2HPO_4$ versetzt, 5 min bei Raumtemperatur inkubiert und das entstandene Präzipitat durch Zentrifugation entfernt (5 min Eppendorfzentrifuge). Der das Peptid enthaltende Überstand wurde anschließend über "reverse phase" HPLC aufgetrennt. Im folgenden sind alle wichtigen HPLC-Daten für die Peptidanalyse aufgelistet:

HPLC Anlage:

| Automatischer Injektor: | Waters WISP 710 A |
|---|---|
| HPLC Pumpen: | Waters Modell 510 (2 Pumpen Mischung hochdruckseitig) |
| Gradientensteuerung: | Absorbance Detector 214 nm Extended Module: 280 nm (Filtergerät) |
| Waters Temperatur Controller (30°C) Integrator: | Waters QA 1 Data System (ident mit Hewlett Pakkard: 3390 A Integrator) |

HPLC Bedingungen:

Säule:      Bakerbond C18 5 mm 4,6*250 mm Fotometerempfindlichkeit: 214 nm: 0,5 AUFS 280 nm: 0,05 AUFS

Schreiber:   Empfindlichkeit 10 mV

Vorschub: 1 cm/min

Fluß: 1ml/min

Temperatur der Säule: 30°C

Einspritzmenge der Probe: 150 ul

Laufmittel A: Dest. Wasser + 0,1% Trifluoressigsäure

Laufmittel B: Acetonitril + 0,1% Trifluoressigsäure

Gradientenbedingungen:

| Isokratisch | 1 Minute | 90% A | 10% B |
|---|---|---|---|
| Linearer Gradient | 27 Minuten | 60% A | 40% B |
| Linearer Gradient | 1 Minute | 20% A | 80% B |
| Isokratisch | 5 Minuten | 20% A | 80% B |

Integratoreinstellungen:

Attenuation: 8

Threshold: 7

Chart Speed: 0,5 cm/min (von 8-30 min, sonst 0,2 cm/min)

Vorbereitung der HPLC-Proben:

Die Proben wurden am Schüttelfinger geschüttelt, 10 sek im Ultraschallbad behandelt, zentrifugiert (Eppendorfzentrifuge) und der Überstand injiziert.

Die Identifizierung der Peptide und deren Spaltprodukte erfolgte bei Verwendung des ursprünglich 16 Aminosäuren langen Peptid-Substrats (Ac-TRPIITTAGPSDMYVH-NH$_2$, Peptid H) durch ein Referenzpeptid (GPSDMYVH-NH$_2$), das das C-terminale Spaltprodukt darstellt. Die Spaltung des Peptids H, welche spezifisch zwischen Alanin und Glycin erfolgt, diente bei allen Untersuchungen als Bezugssystem. Bei Verwendung modifizierter Peptidsubstrate wurden die C-terminalen Spaltprodukte durch N-terminale Sequenzierung (Hunkapiller, M. W. and Hood, L. E. loc. cit.), die am N-Terminus mit einer Acetylgruppe blockierten N-terminalen Spaltprodukte durch Beckman Aminosäureanalyse identifiziert.

1. Ausgehend von dem Standardpeptid H wurde zunächst die Frage der Peptidlänge und ihr Einfluß auf die Effizienz der "trans"-Aktivität untersucht. Eine asymmetrische Verkürzung der Peptide am N-Terminus (siehe Fig. 7; Peptide H, A, B, C, D und E) zeigte, daß zumindest 5 Aminosäuren der P-Region (entsprechen den letzten C-terminalen Aminosäuren von VP1) für eine Spaltung in trans vorhanden sein müssen. Bei einer symmetrischen Verkürzung der Spaltpeptide (siehe Fig. 7; Peptide G, I und J) wurde bei einer Peptidlänge von 7 und 10 Aminosäuren (Peptide J und I) keine Spaltung, bei Verwendung eines 12 Aminosäuren langen Peptidsubstrats (Peptid G) nur eine geringfügige Spaltung beobachtet.

2. Die in Poliovirus zwischen VP1 und 2A von Polio-2A durchgeführte Spaltung erfolgt zwischen Tyrosin und Glycin. Eine weitere Spaltung wird bei Poliovirus in der 3CD Region ebenfalls zwischen Tyrosin und Glycin durchgeführt (Toyoda, H. et al.; loc. cit.). Die Frage, ob HRV2-2A das Polio-Spaltsignal akzeptiert, wurde mit Hilfe weiterer Peptidsubstrate beantwortet (siehe Fig.7; Peptide F und K). Ersetzt man das Spaltsignal Ala/Gly für die HRV2-2A durch das für die Polio-2A (Tyr/Gly) ohne alle anderen Aminosäuren auszutauschen, so können diese beiden Peptide mit der gleichen Effizienz wie die nativen Peptidsubstrate vergleichbarer Länge von HRV2-2A gespalten werden. Verwendet man jedoch Peptide (siehe Fig. 7; Peptide Polio 2A/I und 2A/II), welche nicht nur das Spaltsignal sondern die gesamte ursprüngliche Spaltregion von Poliovirus repräsentieren, so erfolgt keine Spaltung durch HRV2-2A. Das Peptid Polio 2A/I repräsentiert die Spaltstelle VP1/2A für die Protease 2A von Poliovirus Typ 1 und 2A/II stellt die alternative Spaltstelle für die Protease 2 A in 3CD für Poliovirus Typ 1 dar.

3. Bei der Austestung von verschiedenen Inkubationsbedingungen konnte gezeigt werden, daß in Anwesenheit von 2,5 M NaCl im "trans"-Aktivitätspuffer (siehe Tab. 1) eine vollständige Spaltung des Peptidsubstrats durch HRV2-2A erfolgt und keinerlei unspezifischer Abbau der Peptidsubstrate und deren Spaltprodukte mehr eintritt (gezeigt am Beispiel der Transspaltung von Peptid K; siehe Fig. 8). Bei diesen Untersuchungen wurde aus Gründen der besseren Stabilität bzw. Ausbeute bei der Peptidsynthese anstelle des ursprünglichen Peptids H das Peptid K eingesetzt. Alle unten angeführten Ergebnisse mit Peptid K können jedoch bei Verwendung des Peptids H mit gleicher Effizienz reproduziert werden.

4. Zur weiteren Charakterisierung der HRV2-2A wurde der Einfluß verschiedener Detergentien, Lösungs- und Denaturierungsmittel auf die Effizienz der "trans"-Aktivität von HRV2-2A untersucht. Der "trans"-Aktivitätstest wurde unter den oben angegebenen Standardbedingungen (mit Ausnahme von nur 0,5 M NaCl im "trans"-Aktivitätspuffer) durchgeführt. Als Substrat dieser Testserie wurde das Peptid K verwendet. Dabei stellte sich heraus, daß sämtliche verwendeten Detergentien wie Empigen (Alkyl-dimethyl-ammonium-betain), NP40 (Octyl-phenol-ethylenoxid), TWEEN 20 (Polyoxyethylensorbitan-Monolaurat), DMSO (Dimethylsulfoxid) und sogar Glycerin einen drastisch negativen Einfluß auf die "trans"-Aktivität von HRV2-2A ausüben (Tab. 1). Selbst bei niedrigsten Verdünnungsstufen wurde -mit Ausnahme von DMSO- eine 30%-ige Inhibierung festgestellt. Substanzen wie Ammoniumsulfat, Guanidinium-Hydrochlorid und Harnstoff zeigten dagegen einen vergleichsweise wesentlich geringeren Einfluß auf die "trans"-Aktivität. Die Verwendung von hohen Ionenstärken (bis zu 2,5 M NaCl) inhibierte die "trans"-Aktivität nicht. Die Anwesenheit hoher NaCl-Konzentrationen führte im Gegenteil zu einer erhöhten Effizienz der "trans"-Aktivität (100% Spaltung des Peptidsubstrats, Unterdrückung des unspezifischen Abbaus der Peptidspaltprodukte usw.).

5. Das polyklonale Antiserum anti-PC20, welches gegen die letzten 20 Aminosäuren der ursprünglichen Protease 2A gerichtet ist, erkennt HRV2-2A auf Western-blots (Sommergruber, W. et al., loc. cit.). Um den Einfluß von anti-PC20 auf die Effizienz der "trans"-Aktivität festzustellen, wurden steigende Mengen an anti-PC20 zu den unter Standardbedingungen (mit Ausnahme von nur 150 mM NaCl) durchgeführten "trans"-Aktivitätstests zugesetzt (siehe Fig. 10). Dieser Versuch, bei welchem das Peptid K als Peptidsubstrat verwendet wurde, zeigte, daß anti-PC20 keinen Einfluß auf die "trans"-Aktivität von 2A ausübt.

Beispiel 8:

Mutationsanalyse der Spaltstelle der Protease 2A von HRV2.

1. Mutationen der $P_1$ Stelle der Protease 2A von HRV2 (im folgenden HRV2-2A genannt).

Es wurde der Alaninrest gegen einen Threoninrest ausgetauscht, da Threonin eine Hydroxylgruppe am $\beta$-C-Atom besitzt.

Ein Derivat des Vektors pEx2A, nämlich pEx2A/21, welches unter anderem eine neu generierte Mlu I Erkennungssequenz an Position 3137 der HRV2-cDNA und eine neu eingeführte Ava I Stelle (Position 3163, siehe Figur 4) enthält, wurde verwendet, um mit Hilfe eines doppelsträngigen Oligonukleotids (siehe Figur 19) in die $P_1$ Stelle die Ala-->Thr Mutation einzuführen, wobei die neu geschaffenen Restriktionsstellen Mlu I und Ava I genutzt wurden. Für die Konstruktionen wurden das Oligonukleotid aus Figur 19 mit dem 0,45kb Mlu I /Hind III Fragment und dem 3,7kb Ava I /Hind III Fragment von pEx2A/21 ligiert.

Die Präparation der Vektor-DNA, der Einbau des doppelsträngigen Oligonukleotids und die DNA-Sequenzierung wurden nach Standardmethoden durchgeführt. Die Expression des mutierten Proteins wurde, wie beschrieben, in E. coli 537 bzw. AR58 vorgenommen. Die Expressionsprodukte wurden auf SDS-Polyacrylamidgelen analysiert (siehe Figur 24); die Auswirkung der Mutationen auf die Spaltung wurde durch Densitometrie ermittelt, indem das Verhältnis zwischen gespaltenem und nicht-gespaltenem Polypeptid gemessen wurde. Auch die im Beispiel 6 angeführten $P_1$-Mutanten wurden nach dieser Methode getestet (siehe Figur 24).

Für die Densitometrie wurde das Polyacrylamidgel zwischen zwei Blätter vorher ausgekochter Zellophanfolie (Bio-Rad) gelegt und getrocknet. Die Polypeptide auf den Spuren wurden nach Anfärben mit Coomassie Blau dann auf einem Beckmann DU8 Spectralphotometer oder einem Sebia Preference Ecran Densitometer quantitativ bestimmt. Identische Polyacrylamidgele wurden einer Western-blot Analyse unterworfen, wobei einerseits ein monoklonaler Mausantikörper, der gegen den N-terminalen Teil der MS2-Polymerase gerichtet ist, (anti-MS2-Pol; Hansen, J. et al., EMBO Journal, Vol. 7, 1785-1791 (1988)) andererseits ein polyklonales Kaninchenantiserum, das ein Peptid erkennt, welches aus den letzten 20 Aminosäuren der ursprünglichen HRV2-2A besteht (im folgenden anti-PC20 genannt; Sommergruber, W. et al., loc. cit.) eingesetzt wurde.

Das Expressionsprodukt der Konstruktion mit dem Ala-->Thr Austausch an der $P_1$ Stelle wurde gut gespalten.

2. Mutationen der $P_1'$ Stelle der Protease 2A von HRV2 .

Ein Derivat des Vektors pEx2A, nämlich pEx2A/21, welches unter anderem eine neu generierte Mlu I Erkennungssequenz an Position 3137 der HRV2-cDNA enthält, wurde verwendet, um mit Hilfe von doppelsträngigen Oligonukleotiden (siehe Figur 20) in die $P_1'$ Stelle Mutationen einzuführen, wobei die neu geschaffene Restriktionsstelle Mlu I und die ursprünglich in der HRV2-cDNA vorhandene Bst EII Stelle verwendet wurde. Die Konstruktion und die Analyse der Mutationen wurde wie oben beschrieben durchgeführt; die Analyse der Ergebnisse auf Polyacrylamidgelen ist in Figur 25 gezeigt.

Konstruktionen, die die Aminosäuren Tryptophan, Lysin, Threonin und Glutaminsäure an der $P_1'$ Stelle aufweisen, wurden nicht gespalten, was aus dem Fehlen der dem gespaltenen Material entsprechenden Bande bei 50kd hervorgeht. Eine Konstruktion, in der das Glycin an der $P_1'$ Stelle deletiert worden war, wurde ebenfalls nicht gespalten.

Das Derivat des Vektors pEx2A/21 wurde außerdem dazu verwendet, um mit Hilfe von doppelsträngigen Oligonukleotiden (siehe Figur 21) Mutationen in die $P_2'$, die $P_4'$ und die $P_9'$ Stellen der Spaltstelle der Protease 2A einzuführen. Es wurden wieder die neu geschaffene Mlu I und die urspünglich in der HRV2-cDNA vorhandene Bst EII Stelle verwendet. Die Konstruktion und die Analyse der Mutationen wurde wie oben beschrieben durchgeführt; die Analyse der Ergebnisse auf Polyacrylamidgelen ist in Figur 25 gezeigt. Die Konstruktion, die eine Deletion des Prolinrestes an der $P_2'$ Stelle besitzt, wurde nicht gespalten. Der Austausch des Valinrestes an der $P_9'$ Stelle gegen Asparaginsäure führte zu einer 30% Reduktion der Spalteffizienz, durch Threonin an derselben Stelle wurde hingegen die Effizienz nicht beeinflußt. Die Anwesenheit eines Threoninrestes in der $P_4'$ Stelle zeigte auch keinerlei Einfluß auf die Aktivität der HRV2-2A Protease.

Desweiteren wurde dieses System verwendet, um den Einfluß der Sequenz zwischen $P_1'$ und $P_9'$ auf die proteolytische Aktivität von HRV2-2A zu untersuchen. Als Ausgangsbasis dienten die natürlichen Sequenzen, die in HRV14 und Poliovirus vorkommen (die Sequenzen von Poliovirus Serotyp 1, 2 und 3 (Sabin-Stämme) sind in dieser Region identisch). Die Sequenzen für die Mutagenese sind in Figur 22 gezeigt. Die Oligonukleotide in Figur 22 wurden wie oben beschrieben dazu in den Vektor pEx2A/21 eingebracht und die exprimierten Proteine analysiert (siehe Figur 26). Das exprimierte Protein der Konstruktion mit der $P_1'$ - $P_9'$ ($P_2'$ Leu -> Pro) Sequenz von HRV14 wurde nicht prozessiert. Eine Spaltung des exprimierten Proteins wurde weder in der Konstruktion beobachtet, die die $P_1'$ - $P_9'$ Sequenz von Poliovirus besitzt, noch in einer sehr ähnlichen Konstruktion, die eine Variante der $P_1'$ - $P_9'$ Poliosequenz darstellt ( $P_4'$ His -> Met, $P_6'$ Asn -> Tyr).

3. Spaltung der 2A Protease von HRV2 an der Spaltstelle von HRV89

Nochmals wurden Mutanten mit Hilfe des Vektors pEx2A/21 hergestellt, wobei statt der Bst EII Stelle, die neu eingeführte Ava I Stelle (Position 3163, siehe Figur 4) verwendet wurde. Für die Konstruktionen wurden die Oligonukleotide in Figur 23 mit dem 0,45kb Mlu I / Hind III Fragment und dem 3,7kb Ava I /Hind III Fragment von pEx2A/21 ligiert. Die Konstruktion und Analyse der Mutationen wurde wie oben durchgeführt; die daraus resultierenden Polyacrylamidgele sind in Figur 27 gezeigt. Aus der Spaltung der exprimierten Proteine geht klar hervor, daß die Protease HRV2-2A jene Spaltstellen, die die Aminosäuren von HRV89 aufweisen, nicht gut erkennen kann. Um dieses Ergebnis zu untermauern, wurde jene Sequenz

27

eingebaut, die der der Spaltstellenregion des humanen Rhinovirus Serotyp 1B entspricht:


```
Arg-Pro-Ile-Ile-Thr-Thr-Ala-Gly-Pro-Ser-Asp-Met-Tyr-Val-
His-Val  gegen


Arg-Ala-Ser-Met-Lys-Thr-Val-Gly-Pro-Ser-Asp-Leu-Tyr-Val-
His-Val.
```

Die Sequenz hat an der $P_1$ Stelle einen Valinrest; das Expressionsprodukt wurde nur zu 65% gespalten, wie in Figur 26 zu sehen ist.

Beispiel 9:

Expression der Protease 2A von HRV89 und Untersuchung der Spaltspezifität.

1. Expression der Protease 2A von HRV89 (im folgenden HRV89-2A genannt).

Um zu zeigen, daß auch das 2A Gen von HRV89 (EPA 261 403) für eine Protease kodiert, wurde eine DNA Konstruktion hergestellt, in der ein DNA Fragment, das der Region des 2A Gens entspricht, an das für das HRV2-VP1 kodierende DNA Fragment gebunden wurde. Nach Expression dieses Hybrids im pEx System kann aus der Länge des Fusionsproteins darauf geschlossen werden, ob das 2A Protein von HRV89 als Protease fungiert. Die Unterschiede im 2A Gen zwischen HRV2 und HRV89 sind in Figur 28 zu sehen.

Um diese Konstruktion zu erhalten, wurde folgender Weg beschritten. Als Ausgangsplasmid wurde das im Beispiel 6 beschriebene Derivat von pEx2A/21 verwendet, das im Bereich der Spaltstelle in den Positionen $P_{1-4}/P_1'_{-4}'$ für eine Aminosäuresequenz kodiert, wie sie im Serotyp HRV89 vorkommt (im folgenden pEx2A/$P_{1-4}$/$P_1'_{-4}'$/89genannt). Zuerst wurde das Pvu II/Hind III Fragment am C-Terminus der HRV2-2A (siehe Fig. 30) durch ein synthetisches Oligonukleotid ersetzt, das die Sequenz der HRV89-2A besitzt (Figur 29). Das erhaltene Plasmid (pEx2A/S89''/C89 genannt) wurde dann mit Mlu I und Pvu II verdaut und mit einem synthetischen Mlu I/Nsp (7524) I Oligonukleotid (siehe Figur 29) und einem Nsp (7524) I/Pvu II Fragment ligiert, wie in Figur 30 dargestellt. Das synthetische Oligonukleotid kodiert für die Spaltstelle, wie sie im Serotyp HRV89 vorgefunden wird, und das Nsp (7524) I/Pvu II Fragment kodiert für die HRV89-2A von Aminosäure 9 bis 121. Dieses Fragment kann beispielsweise aus dem Klon HRV89/68 gewonnen werden, der in der EPA 261 403 beschrieben worden ist.

Dabei wurde ein Plasmid erhalten, das pEx2A/S89/89 benannt wurde (siehe Figur 29 und Figur 30). Zwei weitere Mutanten von pEx2A/S89/89 wurden konstruiert, um den Einfluß der einzelnen Aminosäuren auf die Spalteffizienz zu überprüfen. Wiederum wurden die Mutanten durch den Einbau von doppelsträngigen Oligonukleotiden erhalten, wobei die oben erwähnte Mlu I Restriktionsstelle und eine neu im Mlu I/Nsp (7524) I Oligonukleotid vorhandene Cla I Restriktionsstelle genutzt wurde (siehe Figur 29). In einer Mutation (im folgenden pEx2A/S2/89 genannt) wurde die HRV2 Spaltstelle wiederhergestellt, in der zweiten (im folgenden pEx2A/S89'/89 genannt) wurde ein Austausch von Valin gegen Alanin an der P1 Stelle durchgeführt (siehe Figur 29).

Die Präparation der Vektor-DNA, der Einbau der doppelsträngigen Oligonukleotide und die DNA-Sequenzierung wurden nach Standardmethoden durchgeführt. Die Expression des mutierten Proteins wurde, wie oben beschrieben, in E. coli 537 bzw. AR58 vorgenommen. Die Expressionsprodukte wurden auf SDS-Polyacrylamidgelen aufgetrennt und mit Coomassie Blau gefärbt (Figur 31). Identische Polyacrylamidgele wurden einer Western-blot Analyse unterworfen, wobei einerseits ein monoklonaler Maus-Antikörper, der gegen den N-terminalen Teil der MS2-Polymerase gerichtet ist (anti-MS2-Pol; Hansen, J. et al., EMBO Journal, Vol. 7, 1785-1791 (1988)), andererseits ein polyklonales Kaninchen-Antiserum (anti-PC20) eingesetzt wurde.

Die Analyse der Expressionsprodukte der drei Konstruktionen mit der HRV89-2A ist in Figur 31 zu sehen. Das Fusionsprotein der Mutante, die die HRV2 Spaltstelle (pEx2A/S2/89) besitzt, wird fast vollständig gespalten; das HRV89-2A Enzym kann daher die HRV2 Spaltstelle genauso effizient wie das HRV2-2A Enzym erkennen. Das ungespaltene Produkt wandert bei der Konstruktion mit dem HRV89-2A etwas langsamer, obwohl die beiden 2A-Proteine die gleiche Zahl an Aminosäuren besitzen. Möglicher-

28

weise haben die 13 Unterschiede in der Aminosäuresequenz einen Einfluß auf die Mobilität des 2A Polypeptids. Die Interpretation der Ergebnisse mit den anderen zwei Konstruktionen mit der HRV89-2A (pEx2A/S89/89 und pEx2A/S89'/89) wurde dadurch erschwert, daß zusätzlich zu den erwarteten 65kD und 50kD Produkten zwei andere Banden mit Molekulargewichten von 62kD und 60kD gefunden wurden. Eine genauere Überprüfung der Banden von der Mutante pEx2A/S2/89 mit der HRV2 Spaltstelle zeigte, daß diese Banden auch bei dieser Konstruktion vorhanden waren. Diese Banden wurden nicht von anti-PC20 erkannt und besitzen daher nicht das C-terminale Ende vom 2A Protein; es scheint daher möglich, daß die Banden durch ein Pausieren der Ribosomen oder einen Kettenabbruch in der Region vor dem C-Terminus des Polypeptids verursacht wurde. Obwohl es nur vier Unterschiede in dieser Region zwischen den 2A Polypeptiden des HRV2 und des HRV89 auf der Proteinebene gibt, ist die "Codon-Usage" sehr verschieden. Die Analyse der Expressionsprodukte von der Intermediärkonstruktion pEx2A/S89''/C89 zeigte auch ein etwas größeres, ungespaltenes Produkt und die Anwesenheit von zwei zusätzlichen Banden, was darauf hinweist, daß die Gründe dafür mit dem Bereich der letzten 20 Aminosäuren in Zusammenhang stehen.

Trotz der Anwesenheit der zwei zusätzlichen Banden ist es offensichtlich, daß in dem hier beschriebenen Expressionssystem die HRV89-2A die HRV2-2A Spaltstelle besser erkennen kann als ihre eigene, was durch den höheren Anteil an ungespaltenem Produkt bei den Konstruktionen pEx2A/S89'/89 und pEx2A/S89/89 zu erkennen ist. Offenbar ist dabei die Anwesenheit von Alanin an der P1 Stelle für die Spaltung durch HRV2-2A ausschlaggebend. Bei der HRV89-2A ist hingegen die Situation etwas anders: das HRV89-2A Enzym scheint seine eigene Spaltstelle nicht besonders gut zu erkennen. Es ist möglich, daß in vivo Teile vom 2B für die Aktivität von HRV89-2A notwendig sind, möglicherweise haben sie einen Einfluß auf die Konformation des 2A-Polypeptids. Auch die Konformation der Kapsidproteine von HRV89 könnte eine Rolle bei der Spaltung spielen. Denkbar wäre es, daß der langsamen Spaltung eine biologische Funktion z. B. bei der Virusreplikation zukommt, ist es doch eine Tatsache, daß der HRV89 Serotyp deutlich langsamer wächst als der HRV2 Serotyp.

**Patentansprüche**

1. DNA-Molekül, dadurch gekennzeichnet, daß es für die VP1/P2A-Region des rhinoviralen Systems kodiert und in dem Bereich der Spaltregion der Protease 2A nicht-Wildtyp - singuläre Signalsequenzen für Restriktionsenzyme aufweist.

2. DNA-Molekül nach Anspruch 1, dadurch gekennzeichnet, daß als rhinovirales System der HRV2- oder der HRV89-Serotyp verwendet wird.

3. DNA-Molekül nach Anspruch 1, dadurch gekennzeichnet, daß die Signalsequenzen für die Restriktionsenzyme XmaI, SmaI, XmaIII, AvaI, NaeI, MluI, EagI, BgIII oder SnaBI erkennbar sind.

4. DNA-Molekül nach Anspruch 1, dadurch gekennzeichnet, daß es
   a. das Expressionsplasmid pEx2A/II ist, das, abgeleitet von pEx2A, die DNA-Sequenz

   5'-GTAACCTTATTTATAGAAATCTTCATCTTTTCAACTCTGAGA
   TGCATGAATCTATTTTGGTATCTTATTCATCAGATCTAATCATTT
   ACCGAACAAACACTGTAGGTGATGATTACATTCCCTCTTGTGATT
   GTACCCAAGCTACGTATTATTGCAAACATAAAAATAGATACTTCC
   CAATTACAGTTACAAGCCATGACTGGATGAAATACAGGAAAGTGA
   GTACTATCCCAAACACATACAGTACAATTTGTTGATTGGTGAGGG
   CC-3'

   gemäß den Oligonukleotiden Fig. 6 enthält;

b. das Expressionsplasmid pEx2A/21 ist, das, abgeleitet von pEx2A, die DNA-Sequenz

5'-ATACCCGGGCTCATCGCACTAATTTTAAAATTGAGGATAGGA

GTATTCAGACAGCAATTGTGACGCGTCCAATTATCACTACGGCCG

GCCCGAGTGACATGTATGTTCATGTAG-3'

gemäß Fig. 4 enthält;
c. das Expressionsplasmid
pEx2A($P_{1-4}$/$P_{1'-4'}$/89, das, abgeleitet von pEx2A/21, im Bereich der Aminosäurepositionen $P_{1-4}$/$P_{1'-4'}$ eine dem Serotyp HRV89 entsprechende DNA-Sequenz

5'-CGCGTCCAATTGTTACAAACGTCGGACCATCTAGTATGTATG

TTCATGTAG-3'

gemäß Fig. 1 enthält;
d. das Expressionsplasmid pEx2A/S89''/C89, das, abgeleitet von pEx2A/$P_{1-4}$/$P_{1'-4'}$/89, anstelle des C-terminalen 2A Proteasebereichs das für den Serotyp HRV89 kodierende PvuII/HindIII-DNA-Fragment mit der Sequenz

5'-CTGGAGGTGAAGGTCACGTTGCTTTTATTGACCTGAGAAAAT

TCCAGTGTGCTGAGGAGCAATAAA-3'

gemaß Fig. 29 enthält;
e. das Expressionsplasmid pEx2A/S89/89, das, abgeleitet von pEx2A/S89''/C89, eine für die Aminosäureposition 9 bis 121 der 2A Protease des Serotyps HRV89 kodierendes Nsp (7524)I/PvuII-DNA-Fragment gemäß Fig. 30 und ein für die Spaltstelle der 2A Protease des Serotyps HRV89 kodierendes DNA-Fragment mit der Sequenz

5'-CGCGTCCTGATGTTTTTACCGTTACAAACGTCGGACCATCAT

CGATGTTTGTACATG-3'

gemäß Fig. 29 enthält;
f. das Expressionsplasmid pEx2A/S2/89, das, abgeleitet von pEx2A/S89/89, ein für die Spaltstelle der 2A Protease des Serotyps HRV2 kodierendes DNA-Fragment mit der Sequenz 5'-CGCGTCCTATTATCACTACAGCTGGACCATCAT-3' gemäß Fig. 29 enthält sowie
g. das Expressionsplasmid pEx2A/S89'/89, das, abgeleitet von pEx2A/S89/89, ein für die Spaltstelle der 2A Protease kodierendes DNA-Fragment mit der Sequenz

5'-CGCGTCCTGATGTTTTTACCGTTACAAACGCTGGACCATC

AT-3'

gemäß Fig. 29 enthält.

5. Oligonukleotide, dadurch gekennzeichnet, daß sie für eine modifizierte VP1/P2A Region des rhinoviralen Systems kodieren, wobei die Modifikationen an der Spaltstelle oder in der Spaltregion der Protease 2A vorliegen.

6. Oligonukleotide nach Anspruch 5, dadurch gekennzeichnet, daß sie die "cis"-Aktivität beeinflussen.

7. Oligonukleotide nach Anspruch 5, dadurch gekennzeichnet, daß die Modifikationen zu einer Störung des aktiven Zentrums der rhinoviralen Protease 2A führen.

8. Oligonukleotide nach Anspruch 5, dadurch gekennzeichnet,
   - daß die Modifikationen für die Aminosäuren Valin oder Isoleucin an der $P_1$-Stelle der Protease HRV2-2A kodieren,
   - oder daß die Modifikationen für Trp, Lys, Thr, Glu oder ΔGly an der $P_1$'-Stelle der Protease HRV2-2A kodieren,
   - oder daß die Modifikationen für ΔPro an der $P_2$'-Stelle oder für Asparaginsäure an der $P_9$'-Stelle der Protease HRV2-2A kodieren,
   - oder daß die Modifikationen für die Aminosäuren V-T-N-V-G-P-S-S an der $P_{1-4}/P_1$'$_{-4}$'-Stelle der Protease HRV2-2A kodieren,
   - oder daß die Modifikationen für die Aminosäuren G-F-G-H-Q-N-K-A an der $P_1$'$_{-8}$' Stelle der Protease HRV2-2A kodieren,
   - oder daß die Modifikationen für die Aminosäuren G-F-G-M-Q-Y-K-A an der $P_1$'$_{-8}$' Stelle der Protease HRV2-2A kodieren,
   - oder daß die Modifikationen für die Aminosäuren G-P-G-P-R-Y-G-G an der $P_1$'$_{-8}$' Stelle der Protease HRV2-2A kodieren,
   - oder daß die Modifikationen für die Aminosäuren R-A-S-M-K-T-V-G-P-S-D-L-Y-V-H an der $P_{7-8}$' Stelle der Protease HRV2-2A kodieren,
   - oder daß die Modifikation für die Aminosäure Asparagin an der $P_2$ Stelle der Protease HRV2-2A kodiert,
   - oder daß die Modifikationen für die Aminosäuren T-V-T-N-A an der $P_{1-5}$ Stelle der Protease HRV2-2A kodieren,
   - oder daß die Modifikationen für die Aminosäuren T-V-T-N-V an der $P_{1-5}$ Stelle der Protease HRV2-2A kodieren,
   - oder daß die Modifikationen für die Aminosäuren D-V-F-T-V-T-N-V an der $P_{1-8}$ Stelle der Protease HRV2-2A kodieren.

9. Oligonukleotide nach Anspruch 5, dadurch gekennzeichnet, daß die Modifikationen für die Aminosäure Alanin an der $P_1$ Stelle der Protease HRV89-2A oder für die Aminosäuren A-T-T-I-I an der $P_{1-5}$ Stelle der Protease HRV89-2A kodieren.

10. Testsystem, dadurch gekennzeichnet, daß in einem geeigneten Expressionssystem das DNA-Molekül nach einem der Ansprüche 1 oder 2 eingefügt ist.

11. Testsystem nach Anspruch 10, dadurch gekennzeichnet, daß es das Expressionsplasmid pEx2A/21, pEx2A/II, pEx2A/$P_{1-4}$/$P_1$'$_{-4}$'/89, pEx2A/S89''/C89, pEx2A/S89/89, pEx2A/S2/89 oder pEx2A/S89'/89 gemäß Anspruch 4 ist.

12. Testsystem nach einem der Ansprüche 10 oder 11, dadurch gekennzeichnet, daß unter Ausnutzung der nicht-Wildtyp-singulären Signalsequenzen ein Oligonukleotid nach einem der Ansprüche 5 bis 9 eingefügt wird.

13. Oligopeptid, dadurch gekennzeichnet, daß es von einem der Oligonukleotide nach einem der Ansprüche 5 bis 9 kodiert wird.

14. Oligopeptid, dadurch gekennzeichnet, daß es von der Protease 2A in trans gespalten wird und nicht der Aminosäuresequenz der nativen VP1/P2A Spaltregion entspricht.

15. Oligopeptid nach Anspruch 14, dadurch gekennzeichnet, daß es zumindest fünf Aminosäuren der C-terminalen Region von VP1 und acht Aminosäuren vom N-Terminus der Protease HRV2-2A oder jeweils sechs Aminosäuren aus diesen Regionen aufweist.

16. Oligopeptid nach Anspruch 14, dadurch gekennzeichnet, daß es ein Peptid mit der Aminosäuresequenz I-V-T-R-P-I-I-T-T-Y-G-P-S-D-M oder ein Peptid mit der Aminosäuresequenz T-R-P-I-I-T-T-Y-G-P-S-D-M-Y-V-H ist.

17. Verfahren zur Herstellung eines DNA-Moleküls, das für die VP1/P2A Region des rhinoviralen Systems kodiert, dadurch gekennzeichnet, daß in den Bereich der Spaltregion der Protease 2A nicht-Wildtyp - singuläre Signalsequenzen für Restriktionsenzyme eingeführt werden.

18. Verfahren zur Herstellung eines DNA-Moleküls nach Anspruch 17, dadurch gekennzeichnet, daß als rhinovirales System der HRV2- oder HRV89-Serotyp verwendet wird.

19. Verfahren zur Herstellung eines DNA-Moleküls nach Anspruch 17 oder 18, dadurch gekennzeichnet, daß die Signalsequenzen für die Restriktionsenzyme XmaI, SmaI, XmaIII, AvaI, NaeI, MluI, EagI, BglII oder SnaBI erkennbar sind.

20. Verfahren zur Herstellung eines Oligonukleotids, das für eine modifizierte VP1/P2A Region des rhinoviralen Systems kodiert, dadurch gekennzeichnet, daß Modifikationen in die Spaltstelle oder in die Spaltregion der Protease 2A eingefügt werden.

21. Verfahren zur Herstellung eines Oligonukleotids nach Anspruch 20, dadurch gekennzeichnet, daß Modifikationen die "cis"-Aktivität der Protease 2A beeinflussen.

22. Verfahren zur Herstellung eines Oligonukleotids nach Anspruch 20 oder 21, dadurch gekennzeichnet, daß die Modifikationen zu einer Störung des aktiven Zentrums der rhinoviralen Protease 2A führen.

23. Verfahren gemäß einem der Ansprüche 20 bis 22, dadurch gekennzeichnet, daß
   - die Modifikationen für die Aminosäuren Valin oder Isoleucin an der $P_1$-Stelle der Protease HRV2-2A kodieren,
   - daß die Modifikationen für Trp, Lys, Thr, Glu oder $\Delta$Gly an der $P_1$'-Stelle der Protease HRV2-2A kodieren,
   - daß die Modifikationen für die Deletion von Prolin an Position $P_2$' oder für Asparaginsäure an der $P_9$'-Stelle der Protease HRV2-2A kodieren,
   - daß die Modifikationen für die Aminosäuren V-T-N-V-G-P-S-S an der $P_{1-4}$/$P_1$'$_{-4}$'-Stelle der Protease HRV2-2A kodieren,
   - daß die Modifikationen für die Aminosäuren G-F-G-H-Q-N-K-A an der $P_1$'$_{-8}$' Stelle der Protease HRV2-2A kodieren,
   - daß die Modifikationen für die Aminosäuren G-F-G-M-Q-Y-K-A an der $P_1$'$_{-8}$' Stelle der Protease HRV2-2A kodieren,
   - daß die Modifikationen für die Aminosäuren G-P-G-P-R-Y-G-G an der $P_1$'$_{-8}$' Stelle der Protease HRV2-2A kodieren,
   - daß die Modifikationen für die Aminosäuren R-A-S-M-K-T-V-G-P-S-D-L-Y-V-H an der $P_{7-8}$' Stelle der Protease HRV2-2A kodieren,
   - daß die Modifikation für die Aminosäure Asparagin an der $P_2$ Stelle der Protease HRV2-2A kodiert,
   - daß die Modifikationen für die Aminosäuren T-V-T-N-A an der $P_{1-5}$ Stelle der Protease HRV2-2A kodieren,
   - daß die Modifikationen für die Aminosäuren T-V-T-N-V an der $P_{1-5}$ Stelle der Protease HRV2-2A kodieren,
   - daß die Modifikationen für die Aminosäuren D-V-F-T-V-T-N-V an der $P_{1-8}$ Stelle der Protease HRV2-2A kodieren,
   - daß die Modifikationen für die Aminosäure Alanin an der $P_1$ Stelle der Protease HRV89-2A kodieren oder
   - für die Aminosäuren A-T-T-I-I an der $P_{1-5}$ Stelle der Protease HRV89-2A kodieren.

24. Verfahren zur Herstellung eines Testsystems, dadurch gekennzeichnet, daß in ein geeignetes Expressionssystem ein nach Anspruch 17 erhältliches DNA-Molekül eingefügt wird.

25. Verfahren zur Herstellung eines Testsystems nach Anspruch 24, dadurch gekennzeichnet, daß in eines der Expressionsplasmide gemäß Anspruch 4 unter Ausnutzung der nicht-Wildtyp-singulären Signalsequenzen ein nach einem der Ansprüche 20 bis 23 erhältliches Oligonukleotid eingefügt wird.

**Claims**

1. DNA molecule, characterised in that it codes for the VP1/P2A region of the rhinoviral system and has, in the region of the cleavage region of the protease 2A, non-wild type single signal sequences for restriction enzymes.

2. DNA molecule according to claim 1, characterised in that the HRV2- or the HRV89 serotype is used as the rhinoviral system.

3. DNA molecule according to claim 1, characterised in that the signal sequences for the restriction enzymes XmaI, SmaI, XmaIII, AvaI, NaeI, MluI, EagI, BglII or SnaBI can be recognised.

4. DNA molecule according to claim 1, characterised in that it is
   a. the expression plasmid pEx2A/II which, derived from pEx2A, contains the DNA sequence

   5'-GTAACCTTATTTATAGAAATCTTCATCTTTTCAACTCTGAGA
   TGCATGAATCTATTTTGGTATCTTATTCATCAGATCTAATCATTT
   ACCGAACAAACACTGTAGGTGATGATTACATTCCCTCTTGTGATT
   GTACCCAAGCTACGTATTATTGCAAACATAAAAATAGATACTTCC
   CAATTACAGTTACAAGCCATGACTGGATGAAATACAGGAAAGTGA
   GTACTATCCCAAACACATACAGTACAATTTGTTGATTGGTGAGGG
   CC-3'

   according to the oligonucleotides in Fig. 6;
   b. the expression plasmid pEx2A/21 which, derived from pEx2A, contains the DNA sequence

   5'-ATACCCGGGCTCATCGCACTAATTTTAAAATTGAGGATAGGA
   GTATTCAGACAGCAATTGTGACGCGTCCAATTATCACTACGGCCG
   GCCCGAGTGACATGTATGTTCATGTAG-3'

   according to Fig. 4;
   c. the expression plasmid pEx2A($P_{1-4}$/$P_{1'-4'}$/89, which, derived from pEx2A/21 in the region of the amino acid positions $P_{1-4}$/$P_{1'-4'}$ contains a DNA sequence

   5'-CGCGTCCAATTGTTACAAACGTCGGACCATCTAGTATGTATG
   TTCATGTAG-3'

   corresponding to the serotype HRV89 according to Fig. 1;
   d. the expression plasmid pEx2A/S89''/C89 which, derived from pEx2A/$P_{1-4}$/$P_{1'-4'}$/89, instead of the C-terminal 2A protease region, contains the PvuII/HindIII-DNA fragment coding for the serotype HRV89, with the sequence

   5'-CTGGAGGTGAAGGTCACGTTGCTTTTATTGACCTGAGAAAAT
   TCCAGTGTGCTGAGGAGCAATAAA-3'

   according to Fig. 29;
   e. the expression plasmid pEx2A/S89/89 which, derived from pEx2A/S89''/C89, contains an Nsp (7524)I/PvuII-DNA fragment according to Fig. 30 coding for the amino acid position 9 to 121 of the 2A protease of the serotype HRV89, and a DNA fragment having the sequence

5'-CGCGTCCTGATGTTTTTACCGTTACAAACGTCGGACCATCAT

CGATGTTTGTACATG-3'

coding for the cleavage site of the 2A protease of serotype HRV89, according to Fig. 29;

f. the expression plasmid pEx2A/S2/89 which, derived from pEx2A/S89/89 contains a DNA fragment having the sequence 5'-CGCGTCCTATTATCACTACAGCTGGACCATCAT-3' coding for the cleavage site of the 2A protease of the serotype HRV2, according to Fig. 29, and

g. the expression plasmid pEx2A/S89'/89, which, derived from pEx2A/S89/89 contains a DNA fragment coding for the cleavage site of the 2A protease with the sequence

5'-CGCGTCCTGATGTTTTTACCGTTACAAACGCTGGACCATC

AT-3'

according to Fig. 29.

5. Oligonucleotides, characterised in that they code for a modified VP1/P2A region of the rhinoviral system, the modifications being at the cleavage site or in the cleavage region of the protease 2A.

6. Oligonucleotides according to claim 5, characterised in that they influence the "cis" activity.

7. Oligonucleotides according to claim 5, characterised in that the modifications lead to a disruption of the active centre of the rhinoviral protease 2A.

8. Oligonucleotides according to claim 5, characterised in that
   - the modifications code for the amino acids valine or isoleucine at the $P_1$-site of the protease HRV2-2A,
   - or the modifications code for Trp, Lys, Thr, Glu or $\Delta$Gly at the $P_1'$-site of the protease HRV2-2A,
   - or the modifications code for $\Delta$Pro at the $P_2'$-site or for aspartic acid at the $P_9'$-site of the protease HRV2-2A,
   - or the modifications code for the amino acids V-T-N-V-G-P-S-S at the $P_{1-4}/P_{1'-4}$-site of the protease HRV2-2A,
   - or the modifications code for the amino acids G-F-G-H-Q-N-K-A at the $P_{1'-8'}$ site of the protease HRV2-2A,
   - or that the modifications code for the amino acids G-F-G-M-Q-Y-K-A at the $P_{1'-8'}$ site of the protease HRV2-2A,
   - or the modifications code for the amino acids G-P-G-P-R-Y-G-G at the $P_{1'-8'}$ site of the protease HRV2-2A,
   - or the modifications code for the amino acids R-A-S-M-K-T-V-G-P-S-D-L-Y-V-H at the $P_{7-8'}$ site of the protease HRV2-2A,
   - or the modification codes for the amino acid asparagine at the $P_2$ site of the protease HRV2-2A,
   - or the modifications code for the amino acids T-V-T-N-A at the $P_{1-5}$ site of the protease HRV2-2A,
   - or the modifications code for the amino acids T-V-T-N-V at the $P_{1-5}$ site of the protease HRV2-2A,
   - or the modifications code for the amino acids D-V-F-T-V-T-N-V at the $P_{1-8}$ site of the protease HRV2-2A.

9. Oligonucleotides according to claim 5, characterised in that the modifications code for the amino acid alanine at the $P_1$ site of the protease HRV89-2A or for the amino acids A-T-T-I-I at the $P_{1-5}$ site of the protease HRV89-2A.

**10.** Test system, characterised in that the DNA molecule according to one of claims 1 and 2 is inserted in a suitable expression system.

**11.** Test system according to claim 10, characterised in that it is the expression plasmid pEx2A/21, pEx2A/II, pEx2A/P$_{1-4}$/P$_{1'-4'}$/89, pEx2A/S89''/C89, pEx2A/S89/89, pEx2A/S2/89 or pEx2A/S89'/89 according to claim 4.

**12.** Test system according to one of claims 10 or 11, characterized in that an oligonucleotide according to one of claims 5 to 9 is inserted using the non-wild type single signal sequences.

**13.** Oligopeptide, characterized in that it is coded by one of the oligonucleotides according to one of claims 5 to 9.

**14.** Oligopeptide, characterized in that it is cleaved in trans by the protease 2A and does not correspond to the amino acid sequence of the native VP1/P2A cleavage region.

**15.** Oligopeptide according to claim 14, characterized in that it has at least five amino acids of the C-terminal region of VP1 and eight amino acids from the N-terminus of the protease HRV2-2A or six amino acids from each of these regions.

**16.** Oligopeptide according to claim 14, characterized in that it is a peptide with the amino acid sequence I-V-T-R-P-I-I-T-T-Y-G-P-S-D-M or a peptide with the amino acid sequence T-R-P-I-I-T-T-Y-G-P-S-D-M-Y-V-H.

**17.** Process for preparing a DNA molecule which codes for the VP1/P2A region of the rhinoviral system, characterized in that non-native single signal sequences for restriction enzymes are inserted in the area of the cleavage region of the protease 2A.

**18.** Process for preparing a DNA molecule according to claim 17, characterised in that the HRV2- or HRV89-serotype is used as the rhinoviral system.

**19.** Process for preparing a DNA molecule according to claim 17 or 18, characterized in that the signal sequences for the restriction enzymes XmaI, SmaI, XmaIII, AvaI, NaeI, MluI, EagI, BglII or SnaBI can be recognized.

**20.** Process for preparing an oligonucleotide which codes for a modified VP1/P2A region of the rhinoviral system, characterized in that modifications are introduced into the cleavage site or into the cleavage region of the protease 2A.

**21.** Process for preparing an oligonucleotide according to claim 20, characterized in that modifications affect the cis-activity of the protease 2A.

**22.** Process for preparing an oligonucleotide according to claim 20 or 21, characterized in that the modifications lead to a disruption of the active centre of the rhinoviral protease 2A.

**23.** Process according to one of claims 20 to 22, characterized in that
   - the modifications code for the amino acids valine or izoleucine at the $P_1$-site of the protease HRV2-2A,
   - the modifications code for Trp, Lys, Thr, Glu or $\Delta$Gly at the $P_{1'}$-site of the protease HRV2-2A,
   - the modifications code for the deletion of proline at position $P_{2'}$ or for aspartic acid at the $P_{9'}$-site of the protease HRV2-2A,
   - the modifications code for the amino acids V-T-N-V-G-P-S-S at the $P_{1-4}$/$P_{1'-4'}$-site of the protease HRV2-2A,
   - the modifications code for the amino acids G-F-G-H-Q-N-K-A at the $P_{1'-8'}$ site of the protease HRV2-2A,
   - the modifications code for the amino acids G-F-G-M-Q-Y-K-A at the $P_{1'-8'}$ site of the protease HRV2-2A,

EP 0 410 147 B1

- the modifications code for the amino acids G-P-G-P-R-Y-G-G at the $P_{1'-8'}$ site of the protease HRV2-2A,
- the modifications code for the amino acids R-A-S-M-K-T-V-G-P-S-D-L-Y-V-H at the $P_{7-8'}$ site of the protease HRV2-2A,
- the modification codes for the amino acid asparagine at the $P_2$ site of the protease HRV2-2A,
- the modifications code for the amino acids T-V-T-N-A at the $P_{1-5}$ site of the protease HRV2-2A,
- the modifications code for the amino acids T-V-T-N-V at the $P_{1-5}$ site of the protease HRV2-2A,
- the modifications code for the amino acids D-V-F-T-V-T-N-V at the $P_{1-8}$ site of the protease HRV2-2A,
- the modifications code for the amino acid alanine at the $P_1$ site of the protease HRV89-2A or
- code for the amino acids A-T-T-I-I at the $P_{1-5}$ site of the protease HRV89-2A.

24. Process for producing a test system, characterized in that a DNA molecule obtainable according to claim 17 is inserted in a suitable expression system.

25. Process for preparing a test system according to claim 24, characterized in that an oligonucleotide which is obtainable according to one of claims 20 to 23 is inserted in one of the expression plasmids according to claim 4, using the non-wild type single signal. sequences.

**Revendications**

1. Molécule d'ADN caractérisée en ce qu'elle code la région VP1/P2A du système rhinoviral et présente dans le domaine de la région de clivage de la protéase 2A des séquences signal non singulières de type sauvage pour des enzymes de restriction.

2. Molécule d'ADN selon la revendication 1, caractérisée en ce que l'on utilise comme système rhinoviral le sérotype HRV2 ou HRV89.

3. Molécule d'ADN selon la revendication 1, caractérisée en ce que les séquences signal sont reconnaissables pour les enzymes de restriction XmaI, SmaI, XmaIII, AvaI, NaeI, MluI, EagI, BglII ou SnaBI.

4. Molécule d'ADN selon la revendication 1, caractérisée en ce qu'elle contient
   a. le plasmide d'expression pEx2A/II qui, dérivé de pEx2A, contient la séquence d'ADN

```
5'-GTAACCTTATTTATAGAAATCTTCATCTTTTCAACTCTGAGA

TGCATGAATCTATTTTGGTATCTTATTCATCAGATCTAATCATTT

ACCGAACAAACACTGTAGGTGATGATTACATTCCCTCTTGTGATT

GTACCCAAGCTACGTATTATTGCAAACATAAAAATAGATACTTCC

CAATTACAGTTACAAGCCATGACTGGATGAAATACAGGAAAGTGA

GTACTATCCCAAACACATACAGTACAATTTGTTGATTGGTGAGGG

CC-3'
```

selon les oligonucléotides de la figure 6;
b. le plasmide d'expression pEx2A/21 qui, dérivé de pEx2A, contient la séquence d'ADN

```
5'-ATACCCGGGCTCATCGCACTAATTTTAAAATTGAGGATAGGA

GTATTCAGACAGCAATTGTGACGCGTCCAATTATCACTACGGCCG

GCCCGAGTGACATGTATGTTCATGTAG-3'
```

selon la figure 4;
c. le plasmide d'expression pEx2A/$P_{1-4}$/$P_{1'-4'}$/89 qui, dérivé de pEx2A/21, contient dans le domaine des positions d'acides aminés $P_{1-4}$/$P_{1'-4'}$ une séquence d'ADN correspondant au sérotype HRV89

36

5'-CGCGTCCAATTGTTACAAACGTCGGACCATCTAGTATGTATG
TTCATGTAG-3'

selon la figure 1;

d. le plasmide d'expression pEx2A/S89''/C89 qui, dérivé de pEx2A/P$_{1-4}$/P$_{1'-4'}$/89, contient à la place du domaine C-terminal de la protéase 2A le fragment d'ADN PvuII/HindIII codant le sérotype HRV89, de séquence

5'-CTGGAGGTGAAGGTCACGTTGCTTTTATTGACCTGAGAAAAT
TCCAGTGTGCTGAGGAGCAATAAA-3'

selon la figure 29;

e. le plasmide d'expression pEx2A/S89/89 qui, dérivé de pEx2A/S89''/C89, contient un fragment d'ADN Nsp(7524)I/PvuII selon la figure 30 qui code les positions d'acides aminés 9 à 121 de la protéase 2A du sérotype HRV89 et un fragment d'ADN codant le site de clivage de la protéase 2A du sérotype HRV89, de séquence

5'-CGCGTCCTGATGTTTTTACCGTTACAAACGTCGGACCATCAT
CGATGTTTGTACATG-3'

selon la figure 29;

f. le plasmide d'expression pEx2A/S2/89 qui, dérivé de pEx2A/S89/89, contient un fragment d'ADN codant le site de clivage de la protéase 2A du sérotype HRV2, de séquence
5'-CGCGTCCTATTATCACTACAGCTGGACCATCAT-3'
selon la figure 29 et

g. le plasmide d'expression pEx2A/S89'/89 qui, dérivé de pEx2A/S89/89, contient un fragment d'ADN codant le site de clivage de la protéase 2A, de séquence
5'-CGCGTCCTGATGTTTTTACCGTTACAAACGCTGGACCATCAT-3'
selon la figure 29.

5. Oligonucléotides caractérisés en ce qu'ils codent une région VP1/P2A modifiée du système rhinoviral, les modifications étant situées au site de clivage ou dans la région de clivage de la protéase 2A.

6. Oligonucléotides selon la revendication 5, caractérisés en ce qu'ils influent sur l'activité "cis".

7. Oligonucléotides selon la revendication 5, caractérisés en ce que les modifications conduisent à une perturbation du centre actif de la protéase 2A rhinovirale.

8. Oligonucléotides selon la revendication 5, caractérisés
   - en ce que les modifications codent les acides aminés valine ou isoleucine au site P$_1$ de la protéase HRV2-2A,
   - ou en ce que les modifications codent Trp, Lys, Thr, Glu ou ΔGly au site P$_1'$ de la protéase HRV2-2A,
   - ou en ce que les modifications codent ΔPro au site P$_2'$ ou l'acide aspartique au site P$_9'$ de la protéase HRV2-2A,
   - ou en ce que les modifications codent les acides aminés V-T-N-V-G-P-S-S au site P$_{1-4}$/P$_{1'-4'}$ de la protéase HRV2-2A,
   - ou en ce que les modifications codent les acides aminés G-F-G-H-Q-N-K-A au site P$_{1'-8'}$ de la protéase HRV2-2A,
   - ou en ce que les modifications codent les acides aminés G-F-G-M-Q-Y-K-A au site P$_{1'-8'}$ de la protéase HRV2-2A,
   - ou en ce que les modifications codent les acides aminés G-P-G-P-R-Y-G-G au site P$_{1'-8'}$ de la protéase HRV2-2A,

EP 0 410 147 B1

- ou en ce que les modifications codent les acides aminés R-A-S-M-K-T-V-G-P-S-D-L-Y-V-H au site $P_{7-8'}$ de la protéase HRV2-2A,
- ou en ce que la modification code l'acide aminé asparagine au site $P_2$ de la protéase HRV2-2A,
- ou en ce que les modifications codent les acides aminés T-V-T-N-A au site $P_{1-5}$ de la protéase HRV2-2A,
- ou en ce que les modifications codent les acides aminés T-V-T-N-V au site $P_{1-5}$ de la protéase HRV2-2A,
- ou en ce que les modifications codent les acides aminés D-V-F-T-V-T-N-V au site $P_{1-8}$ de la protéase HRV2-2A.

9. Oligonucléotides selon la revendication 5, caractérisés en ce que les modifications codent l'acide aminé alanine au site $P_1$ de la protéase HRV89-2A ou les acides aminés A-T-T-I-I au site $P_{1-5}$ de la protéase HRV89-2A.

10. Système de test caractérisé en ce que la molécule d'ADN selon l'une des revendications 1 ou 2 est insérée dans un système d'expression approprié.

11. Système de test selon la revendication 10, caractérisé en ce qu'il s'agit du plasmide d'expression pEx2A/21, pEx2A/II, pEx2A/$P_{1-4}$/$P_{1'-4'}$/89, pEx2A/S89''/C89, pEx2A/S89/89, pEx2A/S2/89 ou pEx2A/S89'/89 selon la revendication 4.

12. Système de test selon l'une des revendications 10 ou 11, caractérisé en ce qu'un oligonucléotide selon l'une des revendications 5 à 9 est inséré au moyen des séquences signal non singulières de type sauvage.

13. Oligopeptide caractérisé en ce qu'il est codé par l'un des oligonucléotides selon l'une des revendications 5 à 9.

14. Oligopeptide caractérisé en ce qu'il est clivé en trans par la protéase 2A et ne correspond pas à la séquence d'acides aminés de la région de clivage VP1/P2A native.

15. Oligopeptide selon la revendication 14, caractérisé en ce qu'il présente au moins cinq acides aminés de la région C-terminale de VP1 et huit acides aminés de l'extrémité N-terminale de la protéase HRV2-2A ou six acides aminés de chacune de ces régions.

16. Oligopeptide selon la revendication 14, caractérisé en ce que c'est un peptide présentant la séquence d'acides aminés L-V-T-R-P-I-I-T-T-Y-G-P-S-D-M ou un peptide présentant la séquence d'acides aminés T-R-P-I-I-T-T-Y-G-P-S-D-M-Y-V-H.

17. Procédé de préparation d'une molécule d'ADN qui code la région VP1/P2A du système rhinoviral, caractérisé en ce que des séquences signal non singulières de type sauvage pour des enzymes de restriction sont insérées dans le domaine de la région de clivage de la protéase 2A.

18. Procédé de préparation d'une molécule d'ADN selon la revendication 17, caractérisé en ce que l'on utilise comme système rhinoviral le sérotype HRV2 ou HRV89.

19. Procédé de préparation d'une molécule d'ADN selon la revendication 17 ou 18, caractérisé en ce que les séquences signal sont reconnaissables pour les enzymes de restriction XmaI, SmaI, XmaIII, AvaI, NaeI, MluI, EagI, BgIII ou SnaBI.

20. Procédé de préparation d'un oligonucléotide qui code une région VP1/P2A modifiée du système rhinoviral, caractérisé en ce que des modifications sont insérées dans le site de clivage ou dans la région de clivage de la protéase 2A.

21. Procédé de préparation d'un oligonucléotide selon la revendication 20, caractérisé en ce que des modifications influent sur l'activité "cis" de la protéase 2A.

38

**22.** Procédé de préparation d'un oligonucléotide selon la revendication 20 ou 21, caractérisé en ce que les modifications conduisent à une perturbation du centre actif de la protéase 2A rhinovirale.

**23.** Procédé selon l'une des revendications 20 à 22, caractérisé
- en ce que les modifications codent les acides aminés valine ou isoleucine au site $P_1$ de la protéase HRV2-2A,
- en ce que les modifications codent Trp, Lys, Thr, Glu ou $\Delta$Gly au site $P_1'$ de la protéase HRV2-2A,
- en ce que les modifications codent la délétion de la proline au site $P_2'$ ou l'acide aspartique au site $P_9'$ de la protéase HRV2-2A,
- en ce que les modifications codent les acides aminés V-T-N-V-G-P-S-S- au site $P_{1-4}/P_{1'-4'}$ de la protéase HRV2-2A,
- en ce que les modifications codent les acides aminés G-F-G-H-Q-N-K-A au site $P_{1'-8'}$ de la protéase HRV2-2A,
- en ce que les modifications codent les acides aminés G-F-G-M-Q-Y-K-A au site $P_{1'-8'}$ de la protéase HRV2-2A,
- en ce que les modifications codent les acides aminés G-P-G-P-R-Y-G-G au site $P_{1'-8'}$ de la protéase HRV2-2A,
- en ce que les modifications codent les acides aminés R-A-S-M-K-T-V-G-P-S-D-L-Y-V-H au site $P_{7-8'}$ de la protéase HRV2-2A,
- en ce que la modification code l'acide aminé asparagine au site $P_2$ de la protéase HRV2-2A,
- en ce que les modifications codent les acides aminés T-V-T-N-A au site $P_{1-5}$ de la protéase HRV2-2A,
- en ce que les modifications codent les acides aminés T-V-T-N-V au site $P_{1-5}$ de la protéase HRV2-2A,
- en ce que les modifications codent les acides aminés D-V-F-T-V-T-N-V au site $P_{1-8}$ de la protéase HRV2-2A.
- en ce que les modifications codent l'acide aminé alanine au site $P_1$ de la protéase HRV89-2A ou
- codent les acides aminés A-T-T-I-I au site $P_{1-5}$ de la protéase HRV89-2A.

**24.** Procédé de préparation d'un système de test, caractérisé en ce qu'une molécule d'ADN qui peut être obtenue selon la revendication 17 est insérée dans un système d'expression approprié.

**25.** Procédé de préparation d'un système de test selon la revendication 24, caractérisé en ce qu'un oligonucléotide qui peut être obtenu selon l'une des revendications 20 à 23 est inséré au moyen des séquences signal non singulières de type sauvage dans l'un des plasmides d'expression selon la revendication 4.

```
wt:                     I  I  T  T  A  G  P  S  D
                     attatcactacagctggccccagtgaca
                     taatagtgatgtcgaccggggtcactgt

Mutationsoligo EBI 936:                          FIG.1.

           3´   gtgatgtATAccggggtcactgt     5´

                              Y
Ala-->Tyr:           attatcactacaTATggccccagtgaca
                     taatagtgatgtATAccggggtcactgt

wt:
                R  P  I  I  T  T  A  G  P  S  D  M  Y  V  H  V
           cgcgtccaattatcactacaGCTggccccagtgacatgtatgttcatgtag
              aggttaatagtgatgtCGAccggggtcactgtacatacaagtacatccattg

Ala-->Gln:
                              Q
           cgcgtccaattatcactacaCAGggccccagtgacatgtatgttcatgtag
              aggttaatagtgatgtGTCccggggtcactgtacatacaagtacatccattg

Ala-->Ile:
                              I
           cgcgtccaattatcactacaATCggccccagtgacatgtatgttcatgtag
              aggttaatagtgatgtTAGccggggtcactgtacatacaagtacatccattg

Ala-->Leu:
                              L
           cgcgtccaattatcactacaCTGggccccagtgacatgtatgttcatgtag
              aggttaatagtgatgtGACccggggtcactgtacatacaagtacatccattg

Ala-->Met:
                              M
           cgcgtccaattatcactacaATGggccccagtgacatgtatgttcatgtag
              aggttaatagtgatgtTACccggggtcactgtacatacaagtacatccattg

Ala-->Phe:
                              F
           cgcgtccaattatcactacaTTCggccccagtgacatgtatgttcatgtag
              aggttaatagtgatgtAAGccggggtcactgtacatacaagtacatccattg

Ala-->Val:
                              V
           cgcgtccaattatcactacaGTGggccccagtgacatgtatgttcatgtag
              aggttaatagtgatgtCACccggggtcactgtacatacaagtacatccattg

HRV89 P1-4/P1´-4´:
                        V  T  N  V  G  P  S  S
           cgcgtccaattGTTACAAACGTCGGACCATCTAGTatgtatgttcatgtag
              aggttaaCAATGTTTGCAGCCTGGTAGATCAtacatacaagtacatccattg
```

40

# FIG. 2A.

# FIG. 2B.

FIG. 3.

λ P_L Promoter

MS2 Polymerase
(98 AS Fragment)

C-terminaler Teil von VP3

VP1

Protease 2A + 2 Stop Codons

amp r

pEx2A

ori

Acc I  Pvu II    BstE II                    Apa I    Pvu II              Hind III
                                                                          Acc I
pEx 2A

                                                          aktives Zentrum

C-terminaler Teil von VP1  Protease 2A                    Vector-DNA

Acc I  Pvu II    BstE II    Bgl II      SnaB I    Apa I    Pvu II         Hind III
                                                                          Acc I
pEx 2A/II

Xma I
Acc          Sma I    BstE II                     Apa I    Pvu II         Hind III
             Mlu I                                                        Acc I
pEx 2A/21

Eag I

43

# FIG. 4.

Sma I/Xma I
atacCcgGgctcatcgcactaattttaaaattgaggataggagtattcagacagcaa
 tgGgcCcgagtagcgtgattaaaattttaactcctatcctcataagtctgtcgtt
Acc I


    Mlu I           Xma III Ava I                      BstE II
ttgtgacGCgTccaattatcactacGgcCggcccGagtgacatgtatgttcatgtag
aacactgCGcAggttaatagtgatgCcgGccgggCtcactgtacatacaagtacatccattg
                 Nae I


# FIG. 6.

2A-1512AB:

BstE II
gtaaccttatttatagaaatcttcatcttttcaactctgagatgcatgaatctattttggtatc
    gaataaatatctttagaagtagaaaagttgagactctacgtacttagataaaaccatag
        Bgl II
ttattcatcagatCtaatcatttaccgaaca******
aataagtagtctaGattagtaaatggcttgtttgtga


2A-1600AB:
                                            SnaB I
aacactgtaggtgatgattacattccctcttgtgattgtacccaagctacGtattattgcaaac
******catccactactaatgtaagggagaacactaacatgggttcgatgCataataacgtttg

ataaaaatagatacttcccaattacagttacaagccatgactgg
tattttttatctatgaagggttaatgtcaatgttcggta######


2A-1728AB:

######tatgaaatacaggaaagtgagtactatcccaaacacatacagtacaatttgttgattg
ctgaccatactttatgtcctttcactcatgatagggtttgtgtatgtcatgttaaacaactaac

    Apa I
gtgagggcc
cactc

FIG. 5.

# FIG. 7.

a.) native HRV2-Peptidsubstrate mit unterschiedlicher Länge:

```
T-R-P-I-I-T-T-A-G-P-S-D-M-Y-V-H    +      Peptid  H'
  R-P-I-I-T-T-A-G-P-S-D-M-Y-V-H    +      Peptid  A
    P-I-I-T-T-A-G-P-S-D-M-Y-V-H    +      Peptid  B
      I-I-T-T-A-G-P-S-D-M-Y-V-H   +/-     Peptid  C
        I-T-T-A-G-P-S-D-M-Y-V-H    -      Peptid  D
          T-T-A-G-P-S-D-M-Y-V-H    -      Peptid  E
    P-I-I-T-T-A-G-P-S-D-M-Y       +/-     Peptid  G
      I-I-T-T-A-G-P-S-D-M          -      Peptid  I
        I-T-T-A-G-P-S              -      Peptid  J
```

b.) HRV2-Peptidsubstrate mit Poliospaltsignal:

```
I-V-T-R-P-I-I-T-T-Y-G-P-S-D-M          +      Peptid  F
    T-R-P-I-I-T-T-Y-G-P-S-D-M-Y-V-H    +      Peptid  K
```

c.) native Polio-Peptidsubstrate:

```
S-T-K-D-L-T-T-Y-G-F-G-M-Q-N-K-A    -    Polio 2A/I
M-Q-K-L-L-D-T-Y-G-I-N-L-P-L-V-T    -    Polio 2A/II
```

FIG. 8.

GPSDMYVH-NH$_2$

Ac-TRPIITTY

Ac-TRPIITTYGPSDMYVH-NH$_2$

# FIG. 9.

FIG.10.

GPSDMYVH-NH₂

Ac-TRPIITTY

Ac-TRPIITTYGPSDMYVH-NH₂

# FIG. 11.

FIG. 12.

λ-P_L

Teil der M52-Polymerase

Teil von VP3

VP1

P2A

Teil von P2B

ori

Amp$^r$

pEx34c x 18521

FIG. 13.

HRV2  1  2

18731
18521

VP1 →

VP2 →
VP3 →

# FIG. 14.

# FIG.15.

### HRV2 Protease 2A

# FIG. 16.

# FIG. 17A.

# FIG. 17 B.

# FIG. 18.

Coomassie Blue

marker
pEx34b
pEx18521
pEx18731
pEx2A
pEx2A Cys 106 --> Ser
pEx2A Cys 112 --> Ser
pEx2A His 114 --> Gly
pEx2A Δ Gly 107/108
pEx2A Pro 103 --> Gly
pEx2A ΔGly 104

start
180
116
84
58
48,5
36,5
26,6

A
B
C

D

wt:

            R   P   I   I   T   T   A   G

      cgcgtccaattatcactacagctggc
         aggttaatagtgatgtcgaccgggct

$P_1$ Ala-->Thr:

                        T

      cgcgtccaattatcactacaACGggc
         aggttaatagtgatgtTGCccgggct

# FIG. 19.

```
wt:
            R   P   I   I   T   T   A   G   P   S   D   M   Y   V   H   V
          cgcgtccaattatcactacggccggcccgagtgacatgtatgttcatgtag
             aggttaatagtgatgccggccgggctcactgtacatacaagtacatccattg


Gly-->Glu:
                                        E
          cgcgtccaattatcactacggccGAAccgagtgacatgtatgttcatgtag
             aggttaatagtgatgccggCTTggctcactgtacatacaagtacatccattg


Gly-->Lys:
                                        K
          cgcgtccaattatcactacggccAAAccgagtgacatgtatgttcatgtag
             aggttaatagtgatgccggTTTggctcactgtacatacaagtacatccattg


Gly-->Thr:
                                        T
          cgcgtccaattatcactacggccACCccgagtgacatgtatgttcatgtag
             aggttaatagtgatgccggTGGggctcactgtacatacaagtacatccattg


Gly-->Trp:
                                        W
          cgcgtccaattatcactacggccTGGccgagtgacatgtatgttcatgtag
             aggttaatagtgatgccggACCggctcactgtacatacaagtacatccattg


Gly--> ΔGly:

          cgcgtccaattatcactacggcc...ccgagtgacatgtatgttcatgtag
             aggttaatagtgatgccgg...ggctcactgtacatacaagtacatccattg
```

# FIG. 20.

59

wt:

```
              R  P  I  I  T  T  A  G  P  S  D  M  Y  V  H  V
         cgcgtccaattatcactacggccggcccgagtgacatgtatgttcatgtag
            aggttaatagtgatgccggccgggctcactgtacatacaagtacatccattg
```

P$_2$':

Pro--> ΔPro:

```
         cgcgtccaattatcactacggccggc...agtgacatgtatgttcatgtag
            aggttaatagtgatgccggccg...tcactgtacatacaagtacatccattg
```

P$_4$':

Asp-->Thr:

```
                                              T
         cgcgtccaattatcactacggccggcccgagtACCatgtatgttcatgtag
            aggttaatagtgatgccggccgggctcaTGGtacatacaagtacatccattg
```

P$_9$':

Val-->Asp:

```
                                                           D
         cgcgtccaattatcactacggccggcccgagtgacatgtatgttcatGACg
            aggttaatagtgatgccggccgggctcactgtacatacaagtaCTGccattg
```

Val-->Thr:

```
                                                           T
         cgcgtccaattatcactacggccggcccgagtgacatgtatgttcatACCg
            aggttaatagtgatgccggccgggctcactgtacatacaagtaTGGccattg
```

# FIG. 21.

```
wt:
              R   P   I   I   T   T   A   G   P   S   D   M   Y   V   H   V
          cgcgtccaattatcactacggccggcccgagtgacatgtatgttcatgtag
            aggttaatagtgatgccggccgggctcactgtacatacaagtacatccattg


Poliovirus P1'-8':

              R   P   I   I   T   T   A   G   F   G   H   Q   N   K   A   V

          cgcgtccaattatcactacggccggcTTCGGCCATCAGAACAAAGCCgtag
            aggttaatagtgatgccggccgAAGCCGGTAGTCTAGTTTCGGcatccattg



Poliovirus P1'-8',P4'His-->Met, P6 Asn-->Tyr:

              R   P   I   I   T   T   A   G   F   G   M   Q   Y   K   A   V
          cgcgtccaattatcactacggccggcTTCGGTATGGAGTATAAAGCCgtag
            aggttaatagtgatgccggccgAAGCCATACCTCATATTTCGGcatccattg


HRV14 P1'-8',P2' Leu-->Pro:

              R   P   I   I   T   T   A   G   P   G   P   R   Y   G   G   V
          cgcgtccaattatcactacggccggcccgGGCCCGCGTTATGGCGGCgtag
            aggttaatagtgatgccggccgggcCCGGGCGCAATACCGCCGcatccattg


HRV1B P7-8':

              R   A   S   M   K   T   V   G   P   S   D   L   Y   V   H   V
          cgcgtGCCAGTATGAAAACGGTTGGTCCGAGTGACCTGtatgttcatgtag
            aCGGTCATACTTTTGCCAACCAGGCTCACTGGACatacaagtacatccattg
```

# FIG. 22.

61

wt:

```
                            R   P   I   I   T   T   A   G
                       cgcgtccaattatcactacggctggc
                          aggttaatagtgatgccgaccgggct
```

P$_2$ Thr -->Asn:

```
                                        N
                       cgcgtccaattatcactAACgctggc
                          aggttaatagtgaTTGcgaccgggct
```

HRV89 P$_{2-5}$:

```
                          T   V       N
                       cgcgtccaACCGTTactAACgctggc
                          aggtTGGCAAtgaTTGcgaccgggct
```

HRV89 P$_{1-5}$:

```
                          T   V       N   V
                       cgcgtccaACCGTTactAACGTCggc
                          aggtTGGCAAtgaTTGCAGccgggct
```

HRV89 P$_{1-8}$:

```
                    D   V   F   T   V       N   V
                 cgcgtccaGATGTTTTTACCGTTactAACGTCggc
                    aggtCTACAAAAATGGCAAtgaTTGCAGccgggct
```

# FIG. 23.

FIG. 24.

FIG. 25.

A

| VARIANTE | SEQUENZ | | % UNGESPALTEN |
|---|---|---|---|
| | R P I I T T A     G P S D M Y V H V | | |
| 1 | F G H Q N K A | | 100 |
| 2 | F G M Q Y K A | | 100 |
| 3 | P G P R Y G G | | 100 |
| 4 | A S M K    V     P S D L Y V H V | | 30 |

B

C

FIG. 26.

65

A

| VARIANTE | SEQUENZ | % UNGESPALTEN |
|---|---|---|
| | I V T R P . . . I I T T A  G P S D M Y V | |
| 1 | N | 14 |
| 2 | T V  N | 33 |
| 3 | T V  N V | 66 |
| 4 | D V F T V  N V | 78 |
| 5 | V  N V  S | 84 |

B

C

# FIG. 27.

```
                    VP1                            v
           2                 t r p - - - i i t t a g p s d m y v h v g n l i
           89                        D V F T V   N V       S   F


y r n i h l f n s e m h e s i l v s y s s d l i i y r t n t v g
                D L D D                                               E


d d y i p s c d c t q a t y y c k h k n r y f p i t v t s h d w
N       V       N         E C       H       D           T       A


y e i q e s e y y p k h i q y n l l i g e g p c e p g d c g g k


l i c k h g v i g i v t a g g d n h v a f i d l r h f h c a e e q
                M I             E G                   K   Q
```

# FIG. 28.

A.

C-Terminus von HRV89-2A (Oligonukleotide UK 956/957):

```
Pvu II                                                         Hind III
   G  G  E  G  H  V  A  F  I  D  L  R  K  F  Q  C  A  E  E  Q  *
ctggaggtgaaggtcacgttgcttttattgacctgagaaaattccagtgtgctgaggagcaataaa
gacctccacttccagtgcaacgaaaataactggactcttttaaggtcacacgactcctcgttattttcga
```

B.

Spaltstelle von HRV89-2A (Oligonukleotide EBI 2506/2498)

```
Mlu I                                     Cla I          Nsp (7524) I
   R  P  D  V  F  T  V  T  N  V  G  P  S  S  M  F  V  H
cgcgtcctgatgttttaccgttacaaacgtcggaccatcatcgatgtttgtacatg
   aggactacaaaaatggcaatgtttgcagcctggtagtagctacaaacat
```

# FIG. 29a.

C.

Mutationen in der Spaltstelle von pEx2A/S89/89.

```
wt:
                    R    P    D    V    F    T    V    T    N    V    G    P    S
                cgcgtcctgatgtttttaccgttacaaacgtcggaccatcat
                    aggactacaaaaatggcaatgtttgcagcctggtagtagc


P₁ Val--›Ala:

                                                       A
                cgcgtcctgatgtttttaccgttacaaacGCTggaccatcat
                    aggactacaaaaatggcaatgtttgCGAcctggtagtagc


HRV2 P₁₋₅:

                                    I    I    T    T    A
                cgcgtcct.........ATTATCACTACAGCTggaccatcat
                    agga.........TAATAGTGATGTCGAcctggtagtagc
```

# FIG. 29b.

A

λP_L

MS2   VP3        VP1           2A        HRV2 VP1/3
                                         HRV2 2A
              P                    Pv H

Ac   M   A B

B

λP_L

MS2   VP3        VP1           2A        HRV2 VP1/3
                                         HRV89 2A
              P                    Pv H

Ac   M   C N

FIG. 30.

A

| VARIANTE | | | SEQUENZ | | | | | | | | | | | | | | | | | | | |
|---|---|---|---|---|---|---|---|---|---|---|---|---|---|---|---|---|---|---|---|---|---|---|
| | SITE | ENZYM | | | | | | | | | | | | | | | | | | | | |
| 1 | 2 | 2 | I | V | T | R | P | . . . | I | I | T | T | A | | G | P | S | D | M | Y | V |
| 2 | 2 | 89 | | | | | | | | | | | | | | | | S | | F | |
| 3 | 89' | 89 | | | | D | V | F T V | | N | A | | | | | | | S | | F | |
| 4 | 89 | 89 | | | | D | V | F T V | | N | V | | | | | | | S | | F | |
| 5 | 89'' | 2/89C | | | | | | V | | N | V | | | | | | | S | | | |

FIG. 31.